# EUROPÄISCHE PATENTSCHRIFT

(11) **EP 1 434 562 B1**
(45) Veröffentlichungstag und Bekanntmachung des Hinweises auf die Patenterteilung: **16.08.2017**
(21) Anmeldenummer: 02777248.2
(22) Anmeldetag: 27.09.2002
(51) Int. Cl.: A61K 8/06

(54) **KOSMETISCHE UND DERMATOLOGISCHE STIFTE**
COSMETIC AND DERMATOLOGICAL STICK
BATONS COSMETIQUES ET DERMATOLOGIQUES

(30) Priorität: 29.09.2001 DE 10148314; 29.09.2001 DE 10148301; 29.09.2001 DE 10148302; 29.09.2001 DE 10148313; 12.10.2001 DE 10150619; 09.11.2001 DE 10155960
(43) Veröffentlichungstag der Anmeldung: 07.07.2004
(73) Patentinhaber: Beiersdorf AG, 20253 Hamburg (DE)
(72) Erfinder: BAUER, Anja, 22549 Hamburg (DE); DÖRSCHNER, Albrecht, 201 46 Hamburg (DE); FILBRY, Alexander, 22459 Hamburg (DE); GÖPPEL, Anja, 22527 Hamburg (DE); LANZENDÖRFER, Ghita, 22087 Hamburg (DE); SCHNEIDER, Kirsten, 22049 Hamburg (DE); SCHULZ, Jens, 22869 Schenefeld (DE); SCHREIBER, Jörg, 22087 Hamburg (DE); STELLING, Jessica, 22143 Hamburg (DE); TESCH, Mirko, 22305 Hamburg (DE)
(86) Internationale Anmeldenummer: PCT/EP2002/010904
(87) Internationale Veröffentlichungsnummer: WO 2003/028690

(56) Entgegenhaltungen:
- EP-A- 0 474 270
- EP-A- 1 036 553
- EP-A- 1 064 908
- WO-A-93/04658
- WO-A-98/17232
- DE-A- 2 335 549

## Beschreibung

Die vorliegende Erfindung betrifft kosmetische Stifte, welche sich durch einen hohen Wassergehalt und durch höhere Mengen an Hautbefeuchtungsmitteln auszeichnen und W/O-Emulsionen darstellen.
Insbesondere betrifft die vorliegende Erfindung Stifte zur Prophylaxe von Falten und Antifaltenstifte, Anti-Akne-Stifte sowie Stifte gegen unreine Haut, Sonnenschutzstifte und Aftersun-Stifte, Lippenstifte, bevorzugt Lippenpflegestifte, aber auch dekorative Lippenstifte, Kajalstifte, Foundationstifte, Lidschattenstifte, Abdeckstifte, Augenbrauenstifte, Augenkonturstifte, Befeuchtungsstifte für das Gesicht/den Körper.
Diese können zusätzlich Lichtschutzfilter, Pigmente, Puderstoffe, weitere Wirkstoffe und/oder Repellentien enthalten.

Technisch betrachtet, sind die meisten Stiftformulierungen wasserfreie Fettmischungen aus festen oder halbfesten Wachsen und flüssigen Ölen, wobei die hochgereinigten Paraffinöle und -wachse die Lippenstiftgrundmasse darstellen. Auch wasserhaltige Zubereitungen sind bekannt, welche gelegentlich auch in Form von W/O-Emulsionen vorliegen.

Übliche Grundstoffe des Standes der Technik für stiftförmige Zubereitungen sind beispielsweise flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und mikrokristalline Wachse bzw. Ozokerit), hochschmelzende Wachse (z.B. Carnaubawachs, Candelillawachs).

Der Nachteil bisher bekannter Stifte für Gesicht und Wangen besteht zum einen in einer mangelhaften Stabilität der Stifte. Viele Stifte sind empfindlich gegen Scherkräfte, und zerbröseln leicht, so dass sich die dekorative Emulsion schlecht auf der Haut verteilen lässt. Ferner sind sie nicht besonders temperaturstabil, schmieren und verfließen bei der Anwendung in den heißen Sommermonaten, während sie im Winter bei kalten Temperaturen spröde und stumpf werden.

Auch die sensorischen Eigenschaften lassen bisher zu wünschen übrig. Die Stifte sollten eigentlich eine angenehm kühlende Wirkung auf die Haut haben und sich cremig anfühlen, was bei den Produkten nach dem Stand der Technik nur teilweise erreicht wird.

Marktübliche Antifalten-Produkte sind zumeist Lotion, Cremes, Gelcremes, die einen Gehalt an Wirkstoffen zur Verringerung der Faltigkeit der Haut aufweisen. Wünschenswert wäre es, diese Wirkstoffe gezielt auf die betroffenen Hautbereiche auftragen zu können. Dazu bieten sich Stiftformulierungen an, die aber derzeit am Markt nur in Form von Lippenpflegestiften, Foundationstiften und dergleichen bekannt sind und die nicht zur Verringerung oder Propylaxe von Falten konzipiert sind. Vorteilhaft wäre derartige Stifte, da sie gezielt auf Areale um die Mundwinkel oder im Augen- und Stirnbereich aufgetragen werden könnten. Ferner kann man erwarten, daß die applizierten Stifteinhaltsstoffe weniger stark auf der Haut spreiten, was insbesondere im Augenbereich von Vorteil sein könnte.

Ein kosmetischer Stift soll schon bei leichtem Andruck einen nicht schmierigen, stumpfen oder klebrigen, aber dennoch gut haftenden Fettfilm an die abgeben. Durch diesen Fettfilm sollen die Lippen bzw. die Haut dann glatt und geschmeidig gemacht werden.

Die Haut der Lippen besitzt nur eine äußerst dünne Hornschicht. Schweißdrüsen sind auf den Lippen gar nicht, Talgdrüsen nur vereinzelt zu finden. Daher ist die Lippenhaut praktisch frei von Fett und neigt, besonders bei kaltem und trockenem Wetter, zum Austrocknen. Dabei können sich kleine Risse in der Haut bilden, und die Empfindlichkeit der Lippen gegenüber chemischen, physikalischen und mikrobiellen Einwirkungen (z.B. Nahrungsmittel, Sonnenlicht, Herpes-Simplex-Viren) steigt.

Dies zu verhindern ist die Aufgabe von Lippenpflegestiften. Diese Produkte enthalten meist zu einem hohen Anteil Wachse und Fettkomponenten, die nach dem Auftragen eine abdeckende Schicht über den Lippen ausbilden.

In die Zubereitungen für Lippenpflegestifte können zusätzlich Wirkstoffe eingearbeitet werden, die der Lippenpflege oder dem Lippenschutz förderlich sind, z.B. Vitamine, Feuchtigkeit spendende Mittel, Lichtschutzmittel, abdeckende Pigmente usw.

Die Lederhaut der Lippen weist gut durchblutete Papillen auf, die bis dicht unter die Lippenoberfläche reichen. Daher sind die Lippen rötlich gefärbt und, je nach Teintfarbe der betreffenden Person, von der übrigen Gesichtshaut mehr oder weniger stark farblich abgesetzt. Ein Stilmittel der dekorativen Kosmetik ist dann auch, die Lippenfarbe durch entsprechende Kosmetika auf den Typ der Person abzustimmen.

Produkte dieser Art sind dekorative Lippenstifte, in welche verschiedenste Farbpigmente eingearbeitet werden können. Auch diese Stifte enthalten zu hohen Anteilen Wachse und Fettkomponenten, die nach dem Auftragen eine abdeckende Lipidschicht über den Lippen ausbilden.

Die Aufgabe dieser Schicht ist jedoch nicht vorderhand, die Lippenhaut vor dem Austrocknen zu schützen. Die Lipidschicht dient hier als auf den Lippen haftende Grundlage für die eingearbeiteten Pigmentstoffe; die Pigmente selbst können aus mancherlei Gründen nicht ohne eine solche Grundlage auf die Lippen aufgetragen werden.

Es ist auch möglich, die Eigenschaften der pflegenden und dekorativen Lippenstifte miteinander zu kombinieren, d.h., in dekorative Lippenstifte pflegende oder schützende Substanzen einzuarbeiten.

Akne ist eine Hauterkrankung mit verschiedenen Formen und Ursachen, gekennzeichnet durch nicht entzündliche und entzündliche Knötchen, ausgehend von verstopften Haarfollikeln (Komedonen), die zur Pustel-, Abszeß- und Narbenbildung führen kann. Am häufigsten ist die Acne vulgaris, die vorwiegend in der Pubertät auftritt. Ursächliche Bedingungen für die Acne vulgaris sind die Verhornung und Verstopfung der Haarfollikel-Mündung, die vom Blutspiegel der männlichen Sexualhormone abhängige Talgproduktion und die Produktion freier Fettsäuren und gewebeschädigender Enzyme durch Bakterien (*Propionibacterium acnes*).

Anti-Akne-Wirkstoffe wie Octoxyglycerin sind beispielsweise in der US 6040347 und der DE 4240674 beschrieben. Zwar werden dort auch Stifte als denkbare Applikationsform genannt, allerdings sind darunter wasserfreie Fettstifte zu verstehen. Die Vorteile den Wirkstoff in einem wasserhaltigen W/O-Stift zu formulieren, wurde nicht erkannt.

Lippenpflegestifte enthalten meist zu einem hohen Anteil Wachse und Fettkomponenten, die nach dem Auftragen eine abdeckende Schicht über den Lippen ausbilden. In die Zubereitungen für Lippenpflegestifte können zusätzlich Wirkstoffe eingearbeitet werden, die der Lippenpflege oder dem Lippenschutz förderlich sind, z.B. Vitamine, Feuchtigkeit spendende Mittel, Lichtschutzmittel, abdeckende Pigmente usw.

Lippenstifte des Standes der Technik mit einem Gehalt an Paraffinen und Bienenwachs sind in "Kosmetik, Entwicklung Herstellung und Anwendung kosmetischer Mittel", S. 105, Herausgeber: W. Umbach, Georg Thieme Verlag, Stuttgart - New York, 1988, beschrieben.

Da sowohl pflegende als auch vorwiegend dekorative Lippenstifte des Standes der Technik teilweise gravierende Mängel aufweisen, war eine weitere Aufgabe der vorliegenden Erfindung, diesen Mängeln Abhilfe zu schaffen.

Wegen der hohen Empfindlichkeit des Lippenbereiches, insbesondere gegenüber ultravioletter Strahlung infolge des praktisch völligen Mangels an Pigmenten, empfiehlt sich, zumal bei erhöhter UV-Exposition wie im Hochgebirge, dem Lippenbereich einen Schutz gegen UV-Strahlung in Form von entsprechenden stiftförmigen Lichtschutzzubereitungen zukommen zulassen. Gerade in stiftförmigen Zubereitungen des Standes der Technik werden oft anorganischen Pigmente als UV-Absorber bzw. UV-Reflektoren zum Schutze des Lippenbereiches vor UV-Strahlen verwendet. Dabei handelt es sich insbesondere um Oxide des Titans, aber auch gelegentlich des Zinks, Eisens, Zirkoniums, Siliciums, Mangans, Aluminiums, Cers und Mischungen davon, sowie Abwandlungen.

Ein erheblicher Mangel der Formulierungen des Standes der Technik besteht unter anderem darin, daß es wegen der niedrigen Wassergehalte an sich akzeptabler Emulsionsstifte praktisch unmöglich war, wasserlösliche UV-Filtersubstanzen in solche Formulierungen einzuarbeiten. Eine weitere Aufgabe der vorliegenden Erfindung war also, Stifte mit ausschließlich wasserlöslichen UV-Filtern oder wasserdispergier-baren Pigmenten (zum Beispiel Titandioxid) zugänglich zu machen beziehungsweise Kombinationen aus wasserlöslichen und fettlöslichen UV-Filtern und Pigmenten.

Der Stand der Technik hat weitere Nachteile. Dazu zählt die Tatsache, daß wasserlösliche Wirkstoffe häufig nicht gut genug fettlöslich sind, als daß sie in nennenswertem Maße in die kosmetischen Grundlagen einzubauen wären. Andererseits wäre ein gewisser Wassergehalt durchaus erwünscht, um die Kompatibilität des kosmetischen Stiftes mit der menschlichen Haut zu erhöhen. Ferner sind Stifte mit sehr hohen Wasseranteilen nach dem Stand der Technik deshalb nicht machbar, weil das Wasser mit der hydrophoben Öl/Wachs/Emulgator-Matrix nicht kompatibel ist.

Stifte, die zusätzlich neben höheren Wassermengen größere Konzentrationen an wasserlöslichen Wirkstoffen, hohen Konzentrationen an Hautbefeuchtungsmitteln (3-50% Glycerin z.B.) und an fettlöslichen Wirkstoffen enthalten, sind nicht beschrieben. Es sind zwar Stifte mit größeren Wassermengen bekannt. Eine aktive Befeuchtung der Haut, die zudem länger anhalten soll und biophysikalisch messbare Befeuchtungswerte wie eine klassische O/W oder W/O-Emulsion aufweist, ist allerdings unbekannt. Dies liegt wohl daran, daß durch Wasser nur eine extrem kurzzeitige Befeuchtung hervorgerufen gerufen wird. Ferner sind wasserarme oder wasserfreie Stiftrezepturen nur deshalb passiv befeuchtend, weil okklusive Wachse verwendet werden, die einen Wasserstau in der Haut hervorrufen. Eine aktive Befeuchtung durch einen Hydrolipidfilm aus Wasser, der zusätzlich größere Mengen an Hautbefeuchtungsmitteln statt eines okklusiven Lipidfilms enthält oder eines nur wasserhaltigen Stiftes ist bisher nicht als vorteilhaft beschrieben worden. Solche Sonnenschutz oder Aftersunstifte wären aber vorteilhaft, weil sich dann wirkstoffhaltige Hydrolipidfilme statt Lipidfilme bilden könnten. Ferner könnten zusätzlich durch die verwendeten Wachse okklusive Effekte wie bei wasserfreien Stiften hervorgerufen werden, sodass ein Synergismus aus Wasser, Befeuchtungsmittel und Wachs für derart konzipierte Emulsionsstifte resultiert.

Ferner war bisher nicht bekannt, dass wasserhaltige Stifte zusätzlich Abdeckpigmente enthalten können oder auch Kombinationen aus Abdeckpigmenten und Perlglanzpigmenten oder ausschließlich Perlglanzpigmente. Perlglanzpigmente sind beispielsweise deshalb schwer in wasserhaltige Rezepturen zu integrieren, weil sie scherempfindlich sind, so daß der Perlglanzeffekt ausbleibt oder nur instabile Rezepturen entstehen. Ferner müssen generell die verwendeten Pigmente mit der Wasser/Befeuchtungsmittel/Lipid/Wachs-Matrix kompatibel gemacht werden.

Nach dem idealen Anforderungsprofil sollen sich kosmetische oder dermatologische Stifte glatt und ohne großen Reibungswiderstand auftragen lassen. Darüber hinaus muss eine solche Formulierung auch noch die Anforderungen erfüllen, daß der betreffende Stift bruchfest und temperaturbeständig sein muss und die Formulierung nicht ausölen darf.

Sollen kosmetische oder pharmazeutische Stifte bestimmte Wirkstoffe enthalten, ist denkbar, daß die übrigen Bestandteile mit den Wirkstoffen nicht kompatibel sind. Dies ist besonders häufig der Fall, wenn die Verwendung der kosmetischen Stifte als Sonnenschutzstifte oder Aftersun-Stifte vorgesehen ist, wenn wasserlösliche Lichtschutzfilter in größeren Mengen im Stift enthalten sein sollen, wenn zur Herstellung eines prophylaktisch wirkenden Antifaltenstifts oder eines Antifaltenstifts wasserlösliche Wirkstoffe in dem Fachmann bekannten Mengen eingearbeitet werden sollen, wenn zur Herstellung eines Antiakne-Stifts wasserlösliche Antiakne-Wirkstoffe in dem Fachmann bekannten Mengen eingearbeitet werden sollen, wenn wasserlösliche Hautbefeuchtungsmittel in größeren Mengen im Stift enthalten sein sollen oder wenn zur Herstellung eines Stifts zusätzlich weitere fett- oder wasserlösliche Wirkstoffe wie Pigmente, Perlglanzpigmente, Vitamine und/oder Antioxidantien eingearbeitet werden sollen.
Für einen Antiakne-Stift beispielsweise wäre es aber gerade besonders vorteilhaft, wenn der Anteil an fettlöslichen Bestandteilen möglichst niedrig läge.

Perlglanzpigmente sind schwer in wasserhaltige Rezepturen zu integrieren, weil sie scherempfindlich sind, so daß der Perlglanzeffekt ausbleibt oder nur instabile Rezepturen entstehen. Ferner müssen generell die verwendeten Pigmente mit der Wasser/Befeuchtungsmittel/Lipid/Wachs-Matrix kompatibel gemacht werden.

Zwar beschreiben sowohl die WO 98/17232 als auch die EP 1 064 908 kosmetische und dermatologische Stifte mit hohem Wassergehalt, wobei im ersteren auch das erfindungsgemäße Emulgatorsystem beschrieben ist und im zweiten der Einsatz von Pigmenten.

Als wichtigstes Ingredienz eines Stiftes sind die Pigmente zu nennen, die in das System stabil eingearbeitet werden müssen und die farbgebende Komponente darstellen. Bei der Einarbeitung von Pigmenten in Emulsionsstifte kann es zu unterschiedlichen Instabilitäten kommen. Dies sind in leichten Fällen Farbinhomogenitäten, dabei sind die unterschiedlichen Pigmente nicht gleichmäßig im Stift verteilt. In schwereren Fällen kann es zu Instabilitäten des Stift-Systems kommen, wobei Wärmestabilität und Stabilität gegen Abbrechen beeinträchtigt werden.

Deswegen ist es für einen ansprechenden Stift (sensorisch attraktiv, optisch einwandfrei und stabil) wichtig, daß alle Komponenten des Systems aufeinander abgestimmt sind.

Dazu gehören sowohl die Kombination von Pigmenten und Füllstoffen sowie die geeignete Auswahl von Emulgatoren, Wachsen und Ölen.

Die oben zitierten Schriften konnten nicht den Weg zur vorliegenden Erfindung weisen, da es sich bei den hier erwähnten Stiften nicht um Stifte (Foundation-Sticks) handelt deren Inhalt großflächig auf das Gesicht aufgetragen wird. Auch die Stabilität der erfindungsgemäßen Stifte ist jener der bisher beschriebenen weit überlegen. Sie sind deutlich unempfindlicher gegen Scherkräfte und bleiben im gesamten Temperaturintervall von -10 °C bis 53 °C stabil und streichfähig. Vor allen Dingen unterscheiden sich die sensorischen Eigenschaften der erfindungsgemäßen Stifte von denen, die in den oben zitierten Schriften offenbart sind.

Aus DE 23 35 549 ist ein Verfahren zur Herstellung eines kosmetischen Stiftes auf der Basis einer W/O-Emulsion bekannt. Nach dieser Lehre wird aus einer Polyhydroxyverbindung und einer nichtionogenen, oberflächenaktiven Verbindung ein Gel hergestellt, dieses mit einer kosmetischen Grundlage vermischt und Wasser in die Mischung emulgiert.

Die DE 41 28 748 beschreibt kosmetische Stifte, welche dadurch gekennzeichnet sind, daß sie Emulsionen darstellen und als wesentliche Bestandteile Bienenwachs, einen oder mehrere Ester aus einer gesättigten Carbonsäure mit 20 - 40 Kohlenstoffatomen und einem gesättigten Alkohol mit 14 - 34 Kohlenstoffatomen, Wasser, sowie gegebenenfalls weitere Lipide und/oder übliche Hilfs- und Zusatzstoffe enthalten.

Die US 4,719,103 beschreibt einen Antitranspirantstift auf der Basis einer W/O-Emulsion, welcher einen hohen Wasseranteil enthalte, welcher sich auszeichnet durch einen Gehalt an flüchtigen Silikonkomponenten, ein festes Alkanol sowie Polyglycerinfettsäureester, beispielsweise Polyglycerylisostearat, als Emulgator. Die US 4,704,271 und die US 4,725,431 beschreiben ähnliche Zubereitungen.

EP 0748622 beschreibt Stifte mit flüchtigen Ölen, wasserabweisenden Polymeren, die im flüchtigen Öl löslich sind und nichtflüchtigen Ölen sowie Puderinhaltstoffen.

Die GB 2162439 beschreibt paraffinhaltige Stifte, welche einen hohen Wasseranteil enthalten sollen, wobei die Emulgatoren aus der Gruppe der Metallsalze gewählt werden.

DE 19643237 beschreibt kosmetische Stifte, die sich durch einen höheren Wasseranteil auszeichnen. Diese enthalten unter anderem bestimmte Wachs- und Ölkomponenten, bestimmte W/O-Emulgatoren neben 30 bis 85 Gew.% Wasser. Der Einsatz von größeren Mengen an Hautbefeuchtungsmitteln ist nicht beschrieben, in den Beispielen ist lediglich der Einsatz von 2 Gew.% Glycerin offenbart.

In DE 29919474 werden W/O-Emulsionsstifte beschrieben. Durch die Verwendung von Polysacchariden wird eine dreidimensionale Struktur erzeugt, die den Stiften mehr Stabilität verleihen soll. Die Verwendung hoher Mengen an Hautbefeuchtungsmittel auch in Gegenwart von Pigmenten wird nicht beschrieben. Auch der Verzicht der Polysaccharide unter Erhalt der Stiftstruktur wird nicht als vorteilhaft angesehen.

In DE 20009445 werden Stifte beansprucht, die nur geringe Wassermengen enthalten (25%). Über Hautbefeuchtungsmittel wird nichts ausgesagt.

In EP 1064908 werden Emulsionsstifte beschrieben, die nur sehr geringe Anteile an Wasser enthalten (14%, S. 4, Bsp. 3). Der Gehalt an Hautbefeuchtungsmittel beträgt 9% (Glycerin, Butylenglycol, Sorbitol). Vorteilhaft insbesondere für den Einsatz in Anti-Akne Produkten wäre dagegen ein besonders geringer Anteil an Ölen oder Lipiden, da diese der Heilung der Akne entgegenwirken.

EP 0194887 beschreibt den Einsatz ethoxylierter Wachse oder auch Triglyceridwachse zur Herstellung von wasserfreien Stiften.

In WO 9817232 und in werden Lippenstifte beschrieben, die sich durch einen höheren Wasseranteil auszeichnen. Da neben dem beschriebenen Kühleffekt auch die Substantivität (beispielsweise bei Verwendung farbiger Pigmente oder Perlglanzpigmente) bei derartigen Stiften, die zusätzlich höhere Mengen an Hautbefeuchtungsmitteln enthalten sollen, wichtig ist, wurde in der Schrift nicht näher ausgeführt.

Dieses wurde auch nicht in den vorab beschriebenen Erfindungen dargelegt. Ferner wurde bisher nicht erwähnt, daß sich auch in Gegenwart von Triglyceridwachsen oder ethoxlierten Wachsen kosmetische Stifte herstellen lassen.

Ein erheblicher Mangel der Formulierungen des Standes der Technik besteht unter anderem darin, dass es wegen der niedrigen Wassergehalte an sich akzeptabler Emulsionsstifte praktisch unmöglich war, wasserlösliche Anti-Falten-Wirkstoffe in solche Formulierungen einzuarbeiten. Ferner besteht ein Mangel darin, wasser-und fettlösliche Wirkstoffe zu kombinieren. Beispielsweise können wasserlösliche oder dispergierbare Wirkstoffe wie Vitamin C, Carnitin, Liponsäure, Alphahydroxysäuren nur schwer nach dem Stand der Technik als Anti-Falten-Wirkstoffe eingearbeitet werden.

Perlglanzpigmente sind schwer in wasserhaltige Rezepturen zu integrieren, weil sie scherempfindlich sind, so dass der Perlglanzeffekt ausbleibt oder nur instabile Rezepturen entstehen. Ferner müssen generell die verwendeten Pigmente mit der Wasser/Befeuchtungsmittel/Lipid/Wachs-Matrix kompatibel gemacht werden.

Ausgehend hiervon liegt der Erfindung die Aufgabe zugrunde, feste W/O-Emulsionen so zu formulieren, dass neben hohen Wasseranteilen und einem hohen Gehalt an Hautbefeuchtungsmittelnstabil eingearbeitet werden können. Zusätzlich sollen in die festen W/O-Emulsionen auch Anti-Akne Wirkstoffen, wasserlösliche Anti-Falten-Wirkstoffen beziehungsweise Kombinationen aus wasserlöslichen und fettlöslichen Anti-Falten-Wirkstoffen auch Feststoffe wie Pigmente, Puderstoffe und UV-Filter sowie sowohl wasserlösliche bzw. in Wasser dispergierbare Wirkstoffe neben lipidlöslichen bzw. in Lipiden dispergierbare Wirkstoffe, insbesondere Antioxidantien, UV-Filter, Repellentien stabil eingearbeitet werden können.

Es war nach all diesem überraschend und nicht vorhersehbar, dass bei Raumtemperatur feste W/O-Emulsionen, enthaltend bezogen auf die Gesamtzubereitung
(a) eine Fettphase, welche
   (a1) mindestens eine ölkomponente, wobei die Ölkomponenten in einem Gehalt von 1 bis 20 Gew.% vorliegen,
   (a2) mindestens eine Wachskomponente umfasst, wobei die Wachskomponenten in einem Gehalt von 5 bis 20 Gew.% vorliegen und das Verhältnis von Öl- zu Wachskomponenten zwischen 3:1 und 1:3 liegt,
(b) eine Wasserphase, welche
   (b1) 35 bis 65 Gew.% Wasser sowie
   (b2) 4 bis 40 Gew.% eines Hautbefeuchtungsmittels gewählt aus der Gruppe Glycerin, Chitosan, Fucogel, Propylenglycol, Polyethylenglycol, Dipropylenglycol, Butylenglycol, Mannitol, Milchsäure, Polyethylenglycol, Glycin, Natriumpyrolidoncarbonsäure, Hyaluronsäure, Salze der angegebenen Säuren sowie Harnstoff und Salze von Metallen der ersten und zweiten Hauptgruppe umfasst,
(c)
   (c) einen W/O-Emulgator oder ein Gemisch aus mehreren W/O-Emulgatoren, gewählt aus der Gruppe der grenzflächenaktiven Substanzen der allgemeinen Struktur A-B-A', wobei A und A' gleiche oder verschiedene hydrophobe organische Reste darstellen und B eine hydrophile Gruppe bedeutet, wobei der oder die Emulgator(en) in Konzentrationen bis 5 Gew.% vorliegen. ,den Nachteilen des Standes der Technik abhelfen. Dabei ist es bevorzugt, wenn der W/O-Emulgator oder die W/O-Emulgatoren gewählt werden aus der Gruppe der Substanzen der allgemeinen Formel 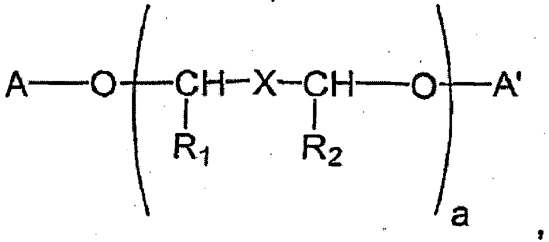 wobei A und A'gleiche oder verschiedene hydrophobe organische Reste darstellen, a eine Zahl von 1 bis 100, vorzugsweise 2 bis 60, insbesondere 5 bis 40 darstellt, X eine Einfachbindung oder die Gruppe 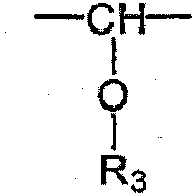 -darstellt,
      R1 und R2 unabhängig voneinander so gewählt werden H, Methyl, dass aber nicht beide Reste gleichzeitig Methyl darstellen,
      R3 gewählt wird aus der Gruppe H, sowie der verzweigten und unverzweigten, gesättigten und ungesättigten Alkyl-und Acylreste mit 1-20 Kohlenstoffatomen,
      oder daß der oder die W/O-Emulgatoren gewählt werden aus der Gruppe der Fettalkohole mit 8 - 30 Kohlenstoffatomen, Monoglycerinester gesättigter und/oder ungesättigter, verzweigter und/oder unverzweigter Alkancarbonsäuren oder Hydroxyalkansäuren einer Kettenlänge von 8 - 24, insbesondere 12 - 18 C-Atomen, Diglycerinester gesättigter und/oder ungesättigter, verzweigter und/oder unverzweigter Alkancarbonsäuren oder Hydroxyalkansäuren einer Kettenlänge von 8 - 24, insbesondere 12 - 18 C-Atomen, Triglycerinester gesättigter und/oder ungesättigter, verzweigter und/oder unverzweigter Alkancarbonsäuren oder Hydroxyalkansäuren einer Kettenlänge von 8 - 24, insbesondere 12 - 18 C-Atomen, Polyglycerinester gesättigter und/oder ungesättigter, verzweigter und/oder unverzweigter Alkancarbonsäuren oder Hydroxyalkansäuren einer Kettenlänge von 8 - 24, insbesondere 12 - 18 C-Atomen mit bis zu 10 Glycerineinheiten, Monoglycerinether gesättigter und/oder ungesättigter, verzweigter und/oder unverzweigter Alkohole einer Kettenlänge von 8 - 24, insbesondere 12 - 18 C-Atomen, Diglycerinether gesättigter und/oder ungesättigter, verzweigter und/oder unverzweigter Alkohole einer Kettenlänge von 8 - 24, insbesondere 12 - 18 C-Atomen, Triglycerinether gesättigter und/oder ungesättigter, verzweigter und/oder unverzweigter Alkohole einer Kettenlänge von 8 - 24, insbesondere 12 - 18 C-Atomen, Polyglycerinether gesättigter und/oder ungesättigter, verzweigter und/oder unverzweigter Alkohole einer Kettenlänge von 8 - 24, insbesondere 12 - 18 C-Atomen mit bis zu 10 Glycerineinheiten, Propylenglycolester gesättigter und/oder ungesättigter, verzweigter und/oder unverzweigter Alkancarbonsäuren oder Hydroxyalkansäuren einer Kettenlänge von 8 - 24, insbesondere 12 - 18 C-Atomen, Sorbitanester gesättigter und/oder ungesättigter, verzweigter und/oder unverzweigter Alkancarbonsäuren oder Hydroxyalkansäuren einer Kettenlänge von 8 - 24, insbesondere 12 - 18 C-Atomen, Sorbitanester von Polyolen, insbesondere des Glycerins, Pentaerythritylester gesättigter und/oder ungesättigter, verzweigter und/oder unverzweigter Alkancarbonsäuren oder Hydroxyalkansäuren einer Kettenlänge von 8 - 24, insbesondere 12 - 18 C-Atomen, Methylglucose Ester gesättigter und/oder ungesättigter, verzweigter und/oder unverzweigter Alkancarbonsäuren oder Hydroxyalkansäuren einer Kettenlänge von 8 - 24, insbesondere 12 - 18 C-Atomen, Polyglycerin Methylglucose Ester gesättigter und/oder ungesättigter, verzweigter und/oder unverzweigter Alkancarbonsäuren oder Hydroxyalkansäuren einer Kettenlänge von 8 - 24, insbesondere 12 - 18 C-Atomen, der Glyceryfettsäure Citrate, Cetyl Dimethicon Copolyole, der Alkyl Methicon Copolyole, der Alkyl Dimthicon Ethoxy Glucoside, oder daß die vorstehend genannten Typen von W/O-Emulgatoren zusätzlich in der Weise polyethoxyliert und/oder polypropoxyliert sind, daß sie ethoxylierte und/oder propopoxylierte W/O-Emulgatoren darstellen.

Besonders bevorzugt ist es wenn der W/O-Emulgator oder die W/O-Emulgatoren so gewählt werden, daß die Reste A und A' werden vorteilhaft gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema 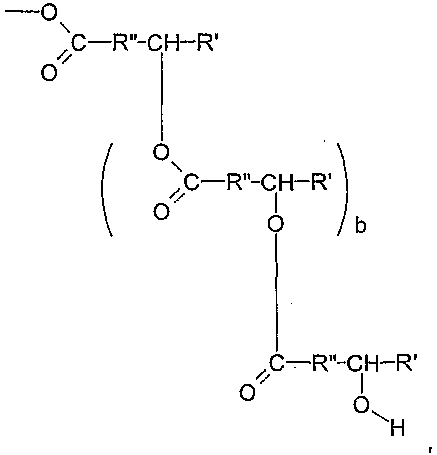 wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann.

Ganz besonders bevorzugt ist es, wenn der oder die W/O-Emulgatoren gewählt werden aus der Gruppe PEG-30 Dipolyhydroxystearat, Decaglycerylheptaoleat, Polyglyceryl-3-Diisostearat, PEG-8 Distearat, Diglycerin Dipolyhydroxystearat, Glycerinisostearat, Sorbitanisostearat, Polyglyceryl-3 methylglucose distearat, Steareth-2.

Bevorzugt ist es, wenn der oder die Stabilisatoren gewählt wird aus der Gruppe der Substanzen der allgemeinen Formel 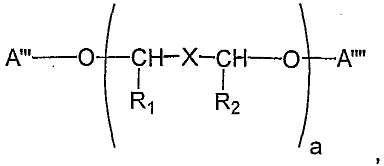 wobei
- A"' und A"" gleiche oder verschiedene hydrophobe organische Reste darstellen,
- a eine Zahl von 1 bis 100, vorzugsweise 2 bis 60, darstellt,
- X eine Einfachbindung oder die Gruppe 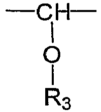
- darstellt,
- R₁ und R₂ unabhängig voneinander aus der Gruppe H, Methyl gewählt werden, daß aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1 - 20 Kohlenstoffatomen,
- wobei die Reste A"' und A"" können gleich oder verschieden sein und gewählt werden aus der Gruppe 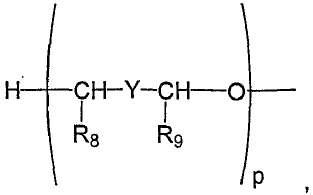
- wobei R₈ und R₉ gleich oder verschieden sein können und gewählt werden aus der Gruppe der gesättigten und ungesättigten Alkyl- und Acylreste mit 1 - 30 Kohlenstoffatomen, p eine Zahl von 1 - 20 darstellt und Y eine Einfachbindung oder die Gruppe 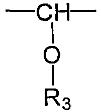 darstellt,
- wobei R₃ gewählt wird aus der Gruppe H, sowie der verzweigten und unverzweigten, gesättigten und ungesättigten Alkyl- und Acylreste mit 1 - 30 Kohlenstoffatomen,
   ferner können die Gruppe A'" und A"" unabhängig voneinander auch Alkylreste oder Acylreste darstellen.

Besonders bevorzugt ist es, wenn als Stabilisator das PEG-45 /Dodecylglycolcopolymer und/oder das PEG-22 / Dodecylglycolcopolymer und/oder das Methoxy PEG-22/Dodecyl Glycol Copolymer verwendet werden.

Besonders bevorzugt ist es, wenn die erfindungsgemäße W/O-Emulsion bei Raumtemperatur fest ist.

Bevorzugt ist ein kosmetischer und/oder dermatologischer Stift enthaltend erfindungsgemäße Emulsionen, der in einem Temperaturbereich von -10 °C bis 50 °C streich- und lagerfähig ist.

Besonders bevorzugt wird der erfindungsgemäße Stift in einem hülsenförmigen Packmittel angeboten. Dabei ist die Stifthülse besonders bevorzugt beidseitig von oben und unten befüllbar. Ganz besonders bevorzugt ist die Stifthülse bei einer Temperatur von 90 °C befüllbar.

Weiterhin bevorzugt ist es, wenn die Ölkomponente oder die Gesamtheit der Ölkomponenten gewählt wird aus der Gruppe der Ester aus gesättigten und/oder ungesättigten, verzweigten und/oder unverzweigten Alkancarbonsäuren einer Kettenlänge von 1 bis 44 C-Atomen und gesättigten und/oder ungesättigten, verzweigten und/oder unverzweigten Alkoholen einer Kettenlänge von 1 bis 44 C-Atomen, aus der Gruppe der Ester aus aromatischen Carbonsäuren und gesättigten und/oder ungesättigten, verzweigten und/oder unverzweigten Alkoholen einer Kettenlänge von 1 bis 30 C-Atomen sofern die Ölkomponente oder die Gesamtheit der Ölkomponenten bei Raumtemperatur eine Flüssigkeit darstellen.

Besonders bevorzugt ist es, wenn die Ölkomponente oder die Gesamtheit der Ölkomponenten gewählt wird aus der Gruppe der verzweigten und unverzweigten Kohlenwasserstoffe, der Silikonöle, Lanoline, der Adipinsäureester, der Butylenglycoldiester, der Dialkylether oder -carbonate, der Gruppe der gesättigten oder ungesättigten, verzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen, wobei die Triglycerinester bevorzugt aus der Gruppe der synthetischen, halbsynthetischen und natürlichen Öle wie Olivenöl, Sonnenblumenöl, Sojaöl, Erdnußöl, Rapsöl, Mandelöl, Palmöl, Kokosöl, Palmkernöl gewählt werden.

Weiterhin bevorzugt ist es, wenn die Wachskomponente oder die Gesamtheit der Wachskomponenten gewählt wird aus der Gruppe
- der Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, aus der Gruppe der Ester aus aromatischen Carbonsäuren und gesättigten und/oder ungesättigten, verzweigten und/oder unverzweigten Alkoholen einer Kettenlänge von 1 bis 80 C-Atomen sofern die Wachskomponente oder die Gesamtheit der Wachskomponenten bei Raumtemperatur einen Festkörper darstellen,
- der natürlichen Wachse,
- der Diester von Polyolen und C10-C80 Fettsäuren,
- der ethoxylierten Wachse,
- der Triglyceridwachse,
- der C16-C60 Fettsäuren (bzw. deren Salze) und/oder C16-C80 Fettalkohole.

Besonders bevorzugt ist es, wenn die Wachskomponente oder die Gesamtheit der Wachskomponenten gewählt wird aus der Gruppe
- der Ester aus gesättigten verzweigten Alkancarbonsäuren einer Kettenlänge von 1 bis 44 C-Atomen und gesättigten verzweigten Alkoholen einer Kettenlänge von 1 bis 44 C-Atomen, sofern die Wachskomponente oder die Gesamtheit der Wachskomponenten bei Raumtemperatur einen Festkörper darstellen,
- der natürlichen Wachse,
- der Diester von Polyolen und C10-C80 Fettsäuren,
- der ethoxylierten Wachse,
- der Triglyceridwachse,
- der C16-C60 Fettsäuren (bzw. deren Salze) und/oder C16-C80 Fettalkohole.

Weiterhin bevorzugt ist es, wenn zusätzliche ein Gehalt an einem oder mehreren wasserlöslichen und/oder mit Wasser quellbaren Polymeren vorhanden ist, insbesondere mit Alkylgruppen veretherte Cellulose- und/oder Stärkederivate, bevorzugt β-Glucane, Xanthangummi, Dextrane, Hydroxymethylcellulose, Hydroxyethylcellulose und/oder Hydroxypropylcellulose, Methoxy-PEG-22/ Dodecyl-Glycol-Copolymere, Poloxamere, mit einem oder mehreren n-Octenylsuccinatresten veresterter hydrophiler Stärke.

Die beschriebenen W/O-Emulsionen eignen sich besonders als kosmetische und/oder dermatologischen Zubereitungen zur Behandlung von Akneerkrankungen der Haut, zur Vorbeugung oder Behandlung von UV-Licht bedingten Schäden der Haut, zur Dekoration der Haut.

Dabei ist es bevorzugt, die Emulsionen als Stifte herzustellen.

Weiterhin bevorzugt ist es, wenn erfindungsgemäße Emulsionen oder Stifte zusätzlich
(a) mindestens ein Pigment und/oder mindestens einen Farbstoff und/oder mindestens einen Puderstoff oder
(b) mindestens einen gegen Falten wirksamen Stoff oder
(c) mindestens eine UVA-Filtersubstanz und/oder mindestens eine UVB-Filtersubstanz und/oder mindestens ein anorganisches Pigment oder
(d) mindestens einen gegen Akne wirksamen Stoff enthalten.

Die Erfindung umfasst auch die Verwendung erfindungsgemäßer Emulsionen oder Stifte zur Hautbefeuchtung.

Im Falle eines Gehaltes an mindestens einem Pigment und/oder mindestens einem Farbstoff und/oder mindestens einem Puderstoff ist es bevorzugt, wenn als Pigmente beschichtete Pigmente eingesetzt werden.

Im Falle eines Gehaltes an mindestens einem Pigment und/oder mindestens einem Farbstoff und/oder mindestens einem Puderstoff ist es bevorzugt, wenn erfindungsgemäße Emulsionen oder Stifte zusätzlich Füllstoffe enthalten und die Gesamtmenge an Pigmenten und Füllstoffen 10 bis 20 Gew.-% der Zubereitung beträgt, wobei besonders bevorzugt die wässrige Phase in einer Konzentration von 35 bis 75 Gewichts-% vorliegt.

Im Falle eines Gehaltes an mindestens einem Pigment und/oder mindestens einem Farbstoff und/oder mindestens einem Puderstoff ist es bevorzugt, wenn die Ölkomponente oder die Gesamtheit der Ölkomponenten gewählt wird aus der Gruppe der verzweigten und unverzweigten Kohlenwasserstoffe, Lanoline, der Adipinsäureester, der Butylenglycoldiester, der cyclischen oder linearen Silikonöle, der verzweigten und unverzweigten Salicylate und Benzoate, der Dialkylether, der Dialkylcarbonate, der Gruppe der gesättigten oder ungesättigten, verzweigten Alkohole, sowie der Fettsäuretriglyceride, namentlich der synthetischen oder natürlichen Triglycerinester gesättigter und/oder ungesättigter, verzweigter und/oder unverzweigter Alkancarbonsäuren einer Kettenlänge von 8 bis 24, insbesondere 12 - 18 C-Atomen.

Im Falle eines Gehaltes an mindestens einem Pigment und/oder mindestens einem Farbstoff und/oder mindestens einem Puderstoff ist es bevorzugt, wenn als Wachse Esterwachse eingesetzt werden.

Solche Emulsionen oder Stiften mit einem Gehalte an mindestens einem Pigment und/oder mindestens einem Farbstoff und/oder mindestens einem Puderstoff werden bevorzugt verwendet als kosmetische Zubereitungen bzw. zur Dekoration der Haut und/oder als Make-up für Gesicht und Wangen (Foundation oder Grundierung) welcher sich gleichmäßig auftragen lässt und eine kühlende und pflegende Wirkung besitzt..

Im Falle eines Gehaltes an mindestens einer UVA-Filtersubstanz und/oder mindestens einer UVB-Filtersubstanz und/oder mindestens einem anorganisches Pigment ist es bevorzugt, wenn die erfindungsgemäßen Stifte oder Emulsionen weitere UV-Filter aus der Gruppe der Benzotriazole, Triazine, Hydroxybenzophenonderivate, bei Raumtemperatur flüssigen, wasserlöslichen, sulfonierten und organische und/oder anorganische Pigmente enthalten.

Im Falle eines Gehaltes an mindestens einer UVA-Filtersubstanz und/oder mindestens einer UVB-Filtersubstanz und/oder mindestens einem anorganisches Pigment ist es besonders bevorzugt, wenn als Hydroxybenzophenon ein Hydroxybenzophenon, welches durch die chemische Strukturformel 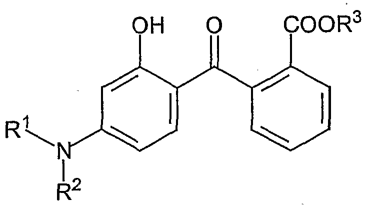 worin
R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und R³ einen C₁-C₂₀-Alkyl Rest bedeutet, verwendet wird.

Im Falle eines Gehaltes an mindestens einer UVA-Filtersubstanz und/oder mindestens einer UVB-Filtersubstanz und/oder mindestens einem anorganisches Pigment ist es ganz besonders bevorzugt, wenn erfindungsgemäße Emulsionen oder Stifte als Hydroxybenzophenon das Aminobenzophenon, welches durch die chemische Strukturformel 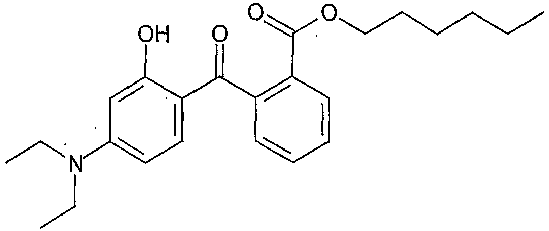 gekennzeichnet ist, gewählt wird.

Im Falle eines Gehaltes an mindestens einer UVA-Filtersubstanz und/oder mindestens einer UVB-Filtersubstanz und/oder mindestens einem anorganisches Pigment ist es ganz außergewöhnlich bevorzugt, wenn erfindungsgemäße Emulsionen oder Stifte weitere UV-Filter aus der Gruppe der Benzotriazole, Triazine, bei Raumtemperatur flüssigen, wasserlöslichen, sulfonierten und organische und/oder anorganische Pigmente enthalten.

Im Falle eines Gehaltes an mindestens einer UVA-Filtersubstanz und/oder mindestens einer UVB-Filtersubstanz und/oder mindestens einem anorganisches Pigment ist es besonders bevorzugt, wenn die Ölkomponente oder die Gesamtheit der Ölkomponenten gewählt wird aus der Gruppe der verzweigten und unverzweigten Kohlenwasserstoffe, Lanoline, der Adipinsäureester, der Butylenglycoldiester der cyclischen oder linearen Silikonöle, der Dialkylether, der Dialkylcarbonate, der Gruppe der gesättigten oder ungesättigten, verzweigten Alkohole, sowie der Fettsäuretriglyceride, besonders bevorzugt der synthetischen oder natürlichen Triglycerinester gesättigter und/oder ungesättigter, verzweigter und/oder unverzweigter Alkancarbonsäuren einer Kettenlänge von 8 bis 24, besonders bevorzugt 12 - 18 C-Atomen, der verzweigten und unverzweigten Salicylate und Benzoate.

Im Falle eines Gehaltes an mindestens einem gegen Akne wirksamen Stoff können als W/O-Emulgatoren vorteilhaft zusätzlich zu den oben genannten bevorzugten Emulgatoren Glycerylfettsäure Citrate verwendet werden.

Es war erstaunlich, daß die erfindungsgemäßen Zubereitungen die Einarbeitung hoher Wassermengen, selbst bei Gegenwart nur geringer Mengen an erfindungsgemäß verwendeten Emulgatoren erlaubt. Die Freisetzung insbesondere wasserlöslicher Wirkstoffe ist gegenüber den herkömmlicher Zubereitungen deutlich erhöht. Ein Beispiel ist die Steigerung des Lichtschutzfaktors: erfindungsgemäß in die Formulierung eingearbeitete Lichtschutzmittel sind in geringerer Konzentration besser wirksam, als die Zubereitungen des Standes der Technik, also zum Beispiel im Vergleich zu W/O-Stiften mit niedrigem Wassergehalt oder im Vergleich zu wasserfreien Suspensionstiften. Die Freisetzung insbesondere wasserlöslicher Wirkstoffe ist gegenüber den herkömmlicher Zubereitungen deutlich erhöht. Erfindungsgemäß in die Formulierung eingearbeitete Akne-Wirkstoffe sind in geringerer Konzentration besser wirksam, als die Zubereitungen des Standes der Technik, also zum Beispiel im Vergleich zu W/O-Stiften mit niedrigem Wassergehalt oder im Vergleich zu wasserfreien Suspensionstiften. Es kann gezeigt werden, daß Hautbefeuchtungswerte resultieren, die man üblicherweise nur von fließfähigen O/W oder W/O Emulsionen her kennt (Nivea). Ferner lassen sich in Gegenwart von höheren Konzentrationen an Wasser und Befeuchtungsmittel Pigmente, Puderrohstoffe oder sogar Perlglanzpigmente einarbeiten. Erstaunlich war, daß die Adhäsion derartiger Stiftformulierungen ausgezeichnet ist, ein Kühleffekt resultiert, die Produkte nicht klebrig sind. Dies war insofern bemerkenswert, da durch die Verwendung größerer Anteile an Wasser und größerer Konzentrationen an Befeuchtungsmitteln (3-60%) wesentlich weniger haftende Inhaltsstoffe wie Ölkomponenten und Wachse pro abgeriebene Stiftmenge im Vergleich zu wasserfreien Stiften oder nur wasserhaltigen Stiften zu Verfügung steht. Ferner weisen derartige farbige Stifte ebenfalls sehr gute befeuchtende Eigenschaften aus und unterscheiden sich daher von üblichen Marktprodukten, deren Befeuchtungseffekte nur durch Okklusion hervorgerufen wird. Die Wasserphase in derartigen Stiften hat mehrere Vorteile: Sie erzeugt einen Kühleffekt, ist ein Medium zum Lösen der Hautbefeuchtungsmittel, erlaubt die Auflösung wasserlöslicher Wirkstoffe, verleiht dem Stift eine angenehme Sensorik und erlaubt es, kostengünstigere Rezepturen im Vergleich zu reinen Fettstiften anzubieten. Ferner führten auch zusätlich eingearbeitete Pigmente zu einer faltenabdeckenden Wirkung.

Überraschend war, daß sich in Gegenwart größerer Konzentrationen an Hautbefeuchtungsmitteln erfindungsgemäße Emulsionsstifte herstellen lassen, die SPF-Werte von 25 oder höher erhalten lassen. Stifte mit hohen SPF-Werten, die gleichzeitig die Haut kühlen und zusätzlich Hautbefeuchtungsmittel und/oder Wirkstoffe an die Haut abgeben, sind besonders elegant. Es konnten hohe SPF-Werte auch durch den kombinierten Einsatz wasser- und fettlöslicher UV-Filter realisiert werden, wobei zusätzlich auch Titandioxid als Pigment vorteilhaft ist. Dieser kombinierte Einsatz von wasser- und fettlöslichen UV-Filtern ist im Unterschied zu reinen Fettstiften erleichtert, weil in den festen W/O-Stiften zusätzlich eine Wasserphase anwesend ist, die ein Lösen des wasserlöslichen Filters erst ermöglicht und so einen Synergismus erlaubt. Dies gilt analog für in Wasser dispergierbare Pigmente. Die Wasserphase in derartigen Stiften hat daher mehrere Vorteile: Sie erzeugt einen Kühleffekt, ist ein Medium zum Lösen der Hautbefeuchtungsmittel, erlaubt die Auflösung wasserlöslicher Filter und Pigmente sowie weiterer wasserlöslicher Wirkstoffe, verleiht dem Stift eine angenehme Sensorik und erlaubt es, kostengünstigere Rezepturen im Vergleich zu reinen Fettstiften anzubieten.

Aber auch die kosmetischen Eigenschaften der erfindungsgemäßen wasserreichen Stifte erweisen sich gegenüber denen des Standes der Technik deutlich verbessert. Beispielsweise lässt sich selbst ohne weitere Zusätze eine angenehme Kühlwirkung auf der Haut durch bloßes Auftragen erzielen, was sich insbesondere bei der Verwendung als Lippenpflegestift, dekorativer Lippenstift, Kajalstift, Foundationstift, Lidschattenstift, Abdeckstift, Lippenstift mit Sonnenschutzfiltern, Augenbrauenstift, Augenkonturstift, Befeuchtungsstift für das Gesicht/den Körper, prophylaktisch wirkender Antifaltenstift oder als Antifaltenstift, Sonnenschutzstift, Repellentstift, Aftersun Stift angenehm bemerkbar macht. Aftersun Stifte, die mit oder ohne UV-Filter fomuliert werden können in Gegenwart von den Sonnenbrand nachträglich oder propylaktisch milderndernden Wirkstoffen, sind wegen des Kühleffekts bei der Applikation auf sonnenbrandgeschädigte Haut vorteilhaft.

Bei der Verwendung als dekorative Lippenstifte, Kajalstifte, Foundationstifte, Lidschattenstifte, Abdeckstifte, Augenbrauenstifte, Augenkonturstifte Befeuchtungsstift für das Gesicht/den Körper , Stifte mit einem Gehalt an gegen Falten wirksamen Stoffen, Sonnenschutzstift, Repellentstift, Aftersun Stift machen sich deutliche Verbesserungen gegenüber dem Stande der Technik dadurch bemerkbar, daß beispielsweise zur Herstellung dieser Stifte es möglich ist, wasserdispergierbares Titandioxid einzusetzen beziehungsweise auch Kombinationen aus lipiddispergierbaren und wasserdispergiebaren Metalloxiden.

Die Erfindung umfasst auch Das Verfahren zur Herstellung erfindungsgemäßer Emulsionen. Die Herstellung erfindungsgemäßer Stifte ist dabei sehr einfach, da es sich um ein Ein-Schritt-Verfahren handelt, bei der beispielsweise die Wasserphase zur heißen Fettphase gegeben und anschließend auf Raumtemperatur abgekühlt wird.

Ferner zeichnet sich das erfindungsgemäße Verfahren dadurch aus, daß zur Herstellung der erfindungsgemäßen Stifte eine Vielzahl von Emulgatoren beziehungsweise Ölkomponenten eingesetzt werden können.

Die Herstellung erfindungsgemäßer Stifte ist dabei sehr einfach, da es sich um ein Ein-Schritt-Verfahren handelt, bei der beispielsweise die Wasserphase zur heißen Fettphase gegeben und anschließend auf Raumtemperatur abgekühlt wird.

Der erfindungsgemäße kosmetische und/oder dermatologische Stift wird erfindungsgemäß vorteilhaft in einer Stifthülse verpackt, die beidseitig von oben und unten befüllbar ist. Diese Stifthülse ist bei einer Gießtemperatur von 90 °C befüllbar. Derartige Stifthülsen werden beispielsweise von der Firma Laffon angeboten.

Ferner ist erfindungsgemäß eine Stifthülse für kosmetische und/oder dermatologische Zubereitungen enthaltend eine kosmetische Schminkzubereitung wie sie in dieser Schrift beschrieben ist.

Nicht zuletzt ist die Verwendung erfindungsgemäßen Stiftes als Make-up-Stift für Gesicht und Wangen (Foundation) welcher sich gleichmäßig auftragen lässt und eine kühlende und pflegende Wirkung besitzt erfindungsgemäß.

Es hat sich in erstaunlicher Weise herausgestellt, daß die erfindungsgemäß verwendeten wasserlöslichen und/oder mit Wasser quellbaren Polymere darüber hinaus die Hautfreundlichkeit der erfindungsgemäßen kosmetischen Zubereitungen erhöhen. Es wird ein angenehmeres Gefühl beim Auftragen der Stiftmasse auf die Haut erzielt.

Die Ölkomponente oder die Gesamtheit der Ölkomponenten der erfindungsgemäßen wasserhaltigen, kosmetischen Stifte sollen bei Raumtemperatur eine Flüssigkeit darstellen, die Wachskomponente oder die Gesamtheit der Wachskomponente sollen bei Raumtemperatur einen Festkörper bilden. Es ist von Vorteil, die Ölkomponenten und die Wachskomponenten so aufeinander abzustimmen, daß das Gemisch aus Ölkomponenten und Wachskomponenten ohne restliche Komponenten, also etwa ohne Wasserphase und ohne Emulgator, bei Raumtemperatur einen Festkörper bildet.

Die Ölkomponente oder die Gesamtheit der Ölkomponenten der erfindungsgemäßen wasserhaltigen, kosmetischen Stifte wird bevorzugt gewählt aus der Gruppe der Ester aus gesättigten und/oder ungesättigten, verzweigten und/oder unverzweigten Alkancarbonsäuren einer Kettenlänge von 1 bis 44 C-Atomen und gesättigten und/oder ungesättigten, verzweigten und/oder unverzweigten Alkoholen einer Kettenlänge von 1 bis 44 C-Atömen, aus der Gruppe der Ester aus aromatischen Carbonsäuren und gesättigten und/oder ungesättigten, verzweigten und/oder unverzweigten Alkoholen einer Kettenlänge von 1 bis 30 C-Atomen sofern die Ölkomponente oder die Gesamtheit der Ölkomponenten bei Raumtemperatur eine Flüssigkeit darstellen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe, der Silikonöle, Lanoline, der Adipinsäureester, der Butylenglycoldiester, der Dialkylether oder -carbonate, der Gruppe der gesättigten oder ungesättigten, verzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl, Rizinusöl, Weizenkeimöl, Traubenkernöl, Distelöl, Nachtkerzenöl, Macadamianußöl und dergleichen mehr. Erfindungsgemäß von Bedeutung sind beispielsweise Kokosglyceride (Myritol 331).

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.
Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB)* und/oder Diethylhexylnaphthalat (*Hallbrite TQ*).

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Ganz besonders vorteilhafte Ölkomponenten können aus der Gruppe Ethylhexyl Cocoate, Myristyl Myristate, Dicaprylylcarbonat, Cetearyl Isononanoat, Octyldodecanol, Polydencene, Squalan, Dicaprylylether, Triisostearin, Butylenglycol Dicaprylat/Dicaprat, Ricinusöl, Capryl-Caprinsäure-triglycerid, Di-(2-Ethylhexyl)adipat), Lanolinöl, Isopropylpalmitat, Cocoglycerid gewählt werden. Ferner sind auch natürliche Öle wie Avocadoöl und Macadamiaöl besonders vorteilhaft. Von diesen sind von besonders ausgezeichnetem Vorteil Dicaprylylcarbonat, Cetearyl Isononanoat, Octyldodecanol, Capryl-Caprinsäuretriglycerid, Di-(2-Ethylhexyl)adipat), Avocadoöl und Macadamiaöl.

Besonders vorteilhaft wird die Ölphase gewählt aus der Gruppe Dicaprylylcarbonat, Octyldodecanol, Dicaprylyether, Ethylhexylcocoat, Capryl-Caprinsäure-triglycerid, Di-(2-Ethylhexyl)adipat, Cocoglycerid, Butyloctylsalicylat, Hexyldecylbenzoat, Butyloctylbenzoat, C12-15 Alkyl Benzoat, Lanolinöl, Butylenglycol Dicaprylat/dicaprat, Cetearylisononanoat.

Im Falle eines Gehaltes an mindestens einem Pigment und/oder mindestens einem Farbstoff und/oder mindestens einem Puderstoff sind bevorzugt weiterhin Lipide gewählt aus der Gruppe der synthetische und natürlichen Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, wie z.B. Jojobaöl.

Im Falle eines Gehaltes an mindestens einem Pigment und/oder mindestens einem Farbstoff und/oder mindestens einem Puderstoff können ferner die Bestandteile der Fettphase vorteilhaft gewählt werden aus der Gruppe der Guerbetalkohole. Guerbetalkohole sind benannt nach Marcel Guerbet, der ihre Herstellung erstmalig beschrieb. Sie entstehen nach der Reaktionsgleichung 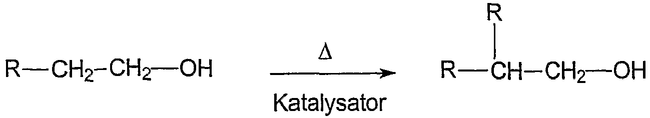 durch Oxidation eines Alkohols zu einem Aldehyd, durch Aldol-Kondensation des Aldehyds, Abspaltung von Wasser aus dem Aldol- und Hydrierung des Allylaldehyds. Guerbetalkohole sind selbst bei niederen Temperaturen flüssig und bewirken praktisch keine Hautreizungen. Vorteilhaft können sie als fettende, überfettende und auch rückfettend wirkende Bestandteile in Haut- und Haarpflegemitteln eingesetzt werden.

Die Verwendung von Guerbet-Alkoholen in Kosmetika ist an sich bekannt. Solche Species zeichnen sich dann meistens durch die Struktur 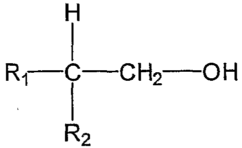 aus. Dabei bedeuten R₁ und R₂ in der Regel unverzweigte Alkylreste.

Erfindungsgemäß vorteilhaft werden der oder die Guerbet-Alkohole gewählt aus der Gruppe, bei denen
R₁ = Propyl, Butyl, Pentyl, Hexyl, Heptyl oder Octyl und
R₂ = Hexyl, Heptyl, Octyl, Nonyl, Decyl, Undecyl, Dodecyl, Tridecyl oder Tetradecyl.

Erfindungsgemäß bevorzugte Guerbet-Alkohole sind das 2-Butyloctanol - es hat die chemische Struktur 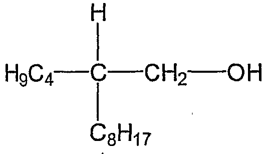 und ist beispielsweise unter der Handelsbezeichnung Isofol® 12 von der Gesellschaft Condea Chemie GmbH erhältlich - und das 2-Hexyldecanol - es hat die chemische Struktur 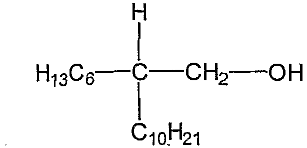 und ist beispielsweise unter der Handelsbezeichnung Isofol® 16 von der Gesellschaft Condea Chemie GmbH erhältlich.

Auch Mischungen von erfindungsgemäßen Guerbet-Alkoholen sind erfindungsgemäß vorteilhaft zu verwenden. Mischungen aus 2-Butyloctanol und 2-Hexyldecanol sind beispielsweise unter der Handelsbezeichnung Isofol® 14 von der Gesellschaft Condea Chemie GmbH erhältlich.

Die Gesamtmenge an Guerbet-Alkoholen in der fertigen Stiftformulierung wird vorteilhaft aus dem Bereich bis 25,0 Gew.-%, bevorzugt 0,5 - 15,0 Gew.-% gewählt, bezogen auf das Gesamtgewicht des Stiftes.

Unpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen. Die nachfolgende **Tabelle 1** führt Lipide auf, die als Einzelsubstanzen oder auch im Gemisch untereinander erfindungsgemäß vorteilhaft sind. Die betreffenden Grenzflächenspannungen gegen Wasser sind in der letzten Spalte angegeben. Es ist jedoch auch vorteilhaft, Gemische aus höher- und niederpolaren und dergleichen zu verwenden.

| ***1.1 Tabelle 1: erfindungsgemäß vorteilhafte Lipide*** | | |
|---|---|---|
| **Handelsname** | INCI-Bezeichnung | **(mN/m)** |
| Isofol® 14 T | Butyl Decanol + Hexyl Decanol + Hexyl Octanol + Butyl Octanol | 27,6 |
| Isofol® 16 | Hexyl Decanol | 24,3 |
| Eutanol® G | Octyldodecanol | 24,8 |
| Cetiol® OE | Dicaprylyl Ether | 22,1 |
| Miglyol® 812 | Caprylic/Capric Triglyceride | 21,3 |
| Cegesoft® C24 | Octyl Palmitate | 23,1 |
| Isopropylstearat | Isopropyl Stearate | 21,9 |
| Estol® 1540 EHC | Octyl Octanoate | 30,0 |
| Finsolv® TN | C₁₂₋₁₅ Alkyl Benzoate | 21,8 |
| Cetiol® SN | Cetearyl Isonoanoate | 28,6 |
| Dermofeel® BGC | Butylene Glycol Caprylate/Caprate | 21,5 |
| Trivent® OCG | Tricaprylin | 20,2 |
| MOD | Octyldodeceyl Myristate | 22,1 |
| Cosmacol® ETI | Di-C₁₂₋₁₃Alkyl Tartrate | 29,4 |
| Miglyol® 829 | Caprylic/Capric Diglyceryl Succinate | 29,5 |
| Prisorine® 2036 | Octyl Isostearate | 29,7 |
| Tegosoft® SH | Stearyl Heptanoate | 28,7 |
| Abil® Wax 9840 | Cetyl Dimethicone | 25,1 |
| Cetiol® LC | Coco-Caprylate/Caprate | 24,8 |
| IPP | Isopropyl Palmitate | 22,5 |
| Luvitol® EHO | Cetearyl Octanoate | 28,6 |
| Cetiol® 868 | Octyl Stearate | 28,4 |

Im Falle eines Gehaltes an mindestens einem Pigment und/oder mindestens einem Farbstoff und/oder mindestens einem Puderstoff wird die Fettphase vorteilhaft gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Im Falle eines Gehaltes an mindestens einem Pigment und/oder mindestens einem Farbstoff und/oder mindestens einem Puderstoff sind besonders vorteilhaft Mischungen aus Octyldodecanol, Capryl-Caprinsäure-triglycerid, Dicaprylylether oder Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Im Falle eines Gehaltes an mindestens einem Pigment und/oder mindestens einem Farbstoff und/oder mindestens einem Puderstoff sind von den Kohlenwässerstoffen Paraffinöl, Cycloparaffin, Squalan, Squalen, hydriertes Polyisobuten bzw. Polydecen vorteilhaft im Sinne der vorliegenden Erfindung zu verwenden.

Als Wachse können unverzweigte, gesättigte und/oder ungesättigte, aliphatische Fettalkohole oder Fettsäuren eingesetzt werden, die eine Kettenlänge von C22 bis C60 haben, wobei die genannten Alkohole bzw Fettsäuren sowohl einzeln als auch im Gemisch vorliegen können. Behensäuren sowie noch längerkettige Fettsäuren (C-24-60 Fettsäuren) sind besonders vorteilhaft zu verwenden. Ferner können diese auch verzeigt sein. Im Falle eines Gehaltes an mindestens einem Pigment und/oder mindestens einem Farbstoff und/oder mindestens einem Puderstoff können Fett- und/oder Wachskomponenten auch aus der Gruppe der pflanzlichen Wachse, tierischen Wachse, Mineralwachse und petrochemischen Wachse gewählt werden, wobei es bevorzugt ist, wenn die Wachskomponente oder die Gesamtheit der Wachskomponenten gewählt wird aus der Gruppe der Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, aus der Gruppe der Ester aus aromatischen Carbonsäuren und gesättigten und/oder ungesättigten, verzweigten und/oder unverzweigten Alkoholen einer Kettenlänge von 1 bis 80 C-Atomen sofern die Wachskomponente oder die Gesamtheit der Wachskomponenten bei Raumtemperatur einen Festkörper darstellen
und es besonders bevorzugt ist, wenn die Wachskomponente oder die Gesamtheit der Wachskomponenten gewählt wird aus der Gruppe
- der Ester aus gesättigten verzweigten Alkancarbonsäuren einer Kettenlänge von 14 bis 44 C-Atomen und gesättigten verzweigten Alkoholen einer Kettenlänge von 14 bis 44 C-Atomen, sofern die Wachskomponente oder die Gesamtheit der Wachskomponenten bei Raumtemperatur einen Festkörper darstellen,
- der natürlichen Wachse,
- der Diester von Polyolen und/oder C10-C80 Fettsäuren,
- der ethoxylierten Wachse,
- der Triglyceridwachse,
der C16-C60 Fettsäuren (bzw. deren Salze) und/oder C16-C80 Fettalkohole, wobei erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse, sofern die im Hauptanspruch geforderten Bedingungen eingehalten werden;
weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), Syncrowax HGLC (C₁₆₋₃₆ -Fettsäuretriglycerid) und Syncrowax AW 1C (C₁₈₋₃₆-Fettsäure) bei der CRODA GmbH erhältlichen sowie Montanesterwachse, Sasolwachse, hydrierte Jojobawachse, Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C₂₀₋₄₀-Alkylstearat, C₂₀₋₄₀-Alkylhydroxystearoylstearat und/oder Glykolmontanat.;
die Wachskomponente oder die Gesamtheit der Wachskomponenten der erfindungsgemäßen W/O-Emulsionsstifte wird bevorzugt gewählt aus der Gruppe der Ester aus gesättigten und/oder ungesättigten, verzweigten und/oder unverzweigten Alkancarbonsäuren einer Kettenlänge von 14 bis 44 C-Atomen und gesättigten und/oder ungesättigten, verzweigten und/oder unverzweigten Alkoholen einer Kettenlänge von 14 bis 44 C-Atomen, aus der Gruppe der Ester aus aromatischen Carbonsäuren bzw. Hydroxycarbonsäuren (z.B. 12-Hydroxystearinsäure) und gesättigten und/oder ungesättigten, verzweigten und/oder unverzweigten Alkoholen einer Kettenlänge von 3 bis 30 C-Atomen sofern die Wachskomponente oder die Gesamtheit der Wachskomponenten bei Raumtemperatur einen Festkörper darstellen; bevorzugt ist, die Wachskomponenten zu wählen aus der Gruppe der
- Ester aus gesättigten verzweigten Alkancarbonsäuren einer Kettenlänge von 14 bis 44 C-Atomen und gesättigten unverzweigten Alkoholen einer Kettenlänge von 14 bis 44 C-Atomen, sofern die Wachskomponente oder die Gesamtheit der Wachskomponenten bei Raumtemperatur einen Festkörper darstellen,
und/oder der
- Ester aus gesättigten unverzweigten Alkancarbonsäuren einer Kettenlänge von 14 bis 44 C-Atomen und gesättigten verzweigten Alkoholen einer Kettenlänge von 14 bis 44 C-Atomen, sofern die Wachskomponente oder die Gesamtheit der Wachskomponenten bei Raumtemperatur einen Festkörper darstellen;
- Polyethylenwache (Performalene 400),
besonders bevorzugt ist, die Wachskomponenten zu wählen aus der Gruppe der Ester aus gesättigten verzweigten Alkancarbonsäuren einer Kettenlänge von 14 bis 44 C-Atomen und gesättigten verzweigten Alkoholen einer Kettenlänge von 14 bis 44 C-Atomen, sofern die Wachskomponente oder die Gesamtheit der Wachskomponenten bei Raumtemperatur einen Festkörper darstellen; Insbesondere vorteilhaft können die Wachskomponenten aus der Gruppe der C₁₆₋₃₆-Alkylstearate, der C₁₀₋₄₀-Alkylstearate, der C₂₀₋₄₀Alkylisostearate, der C₂₀₋₄₀-Dialkyldimerate, der C₁₈₋₃₈ Alkylhydroxystearoylstearate, der C₂₀₋₄₀-Alkylerucate gewählt werden, ferner C₃₀₋₅₀-Alkylbienenwachs, Cetearylbehenat, Polyethylenwachs (Performalene 400); dabei können die Wachskomponenten weiterhin sowohl einzeln als auch im Gemisch vorliegen. Dabei ist ein Wachsanteil von 5 bis 20 Gew. % zu wählen. Bevorzugt sind die Wachse aus der Gruppe der Esterwachse zu wählen, besonders bevorzugt sind Esterwachse von langkettigen unverzweigten Alkylketten. Weiterhin von Vorteil ist es , wenn die Alkylketten unterschiedlicher Kettenlänge sind;.es können beliebige Abmischungen von Öl- und Wachskomponenten vorteilhaft im Sinne der vorliegenden Erfindung eingesetzt werden.

Die langkettigen Fettsäuren können auch in Form Ihrer Salze eingesetzt werden (Seifen von einwerigen, zwei oder dreiwertigen Kationen wie beispielsweise Kalziumbehenat)

Vorteilhaft im Sinne der vorliegenden Erfindung sind insbesondere Fettalkohole bzw. Fettalkoholgemische, welche durch Verseifung von Wachsen oder Wachsgemischen erhältlich sind. Die als Ausgangsprodukt verwendeten Wachse bzw. Wachsgemische können als natürliche Produkte unterschiedlich zusammengesetzt sein.

Vorteilhafte Fettalkohole bzw. Fettalkoholgemische sind beispielsweise aus Bienenwachs, Chinawachs, Hummelwachs und anderen Insektenwachsen erhältlich.

Auch Fettalkohole bzw. Fettalkoholgemische, welche aus Pflanzenwachsen erhältlich sind, sind vorteilhaft im Sinne der vorliegenden Erfindung. Vorzugsweise verwendbar sind Cuticularwachse niederer und höherer Pflanzen, Algen, Flechten, Moose und Pilze, wie beispielsweise Candelillawachs, Carnaubawachs, Japanwachs, Espartograswachs, Korkwachs, Reiswachs, Zuckerrohrwachs, Fruchtwachse, z. B. Apfelwachs, Blütenwachse, Blattwachse von Nadelhölzern, Kaffeewachs, Flachswachs, Sesamwachs, Jojobaöl und dergleichen mehr.

Ferner können auch Reiswachse, Fruchtwachse wie Apfelwachs, Orangenwachs, Zitronenwachs, Grapefruitwachs, Lorbeerwachs (= Bayberrywax) und dergleichen, vorteilhaft verwendet werden. Außerdem können diese natürlichen Wachse auch ohne synthetische Wachse allein eingesetzt werden.

Ferner können auch Wachse von Diestern der C10-C80 Fettsäuren eingesetzt werden, wobei als Alkoholkomponente Propylenglycol, Ethylenglycol, Polyethylenglycol, Polypropylenglcol, Polyglycerin gewählt ist. Ferner können auch Pentaerythritol tri orthotetra -ester von C10 bis C80 Fettsäuren oder auch entspr. Fettsäuren der Sorbitantriester sowie Sucrosepolyester mit 3-8 mol Substitutionsgrad eingesetzt werden.

Beispielsweise ist der Ethylenglycolester von C18-36-Fettsäuren (Syncrowax ERL-C) oder auch Ethylenglycoldistearate und Glycol Distearat geeignet.

Ferner können Wachse aus der Gruppe der Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, aus der Gruppe der Ester aus aromatischen Carbonsäuren bzw. Hydroxycarbonsäuren (z.B. 12-Hydroxystearinsäure) und gesättigten und/oder ungesättigten, verzweigten und/oder unverzweigten Alkoholen einer Kettenlänge von 1 bis 80 C-Atomen sofern die Wachskomponente oder die Gesamtheit der Wachskomponenten bei Raumtemperatur einen Festkörper darstellen, gewählt werden.

Bevorzugt ist, die Wachskomponenten zu wählen aus der Gruppe der
- Ester aus gesättigten verzweigten Alkancarbonsäuren einer Kettenlänge von 1 bis 60 C-Atomen und gesättigten unverzweigten Alkoholen einer Kettenlänge von 1 bis 60 C-Atomen, sofern die Wachskomponente oder die Gesamtheit der Wachskomponenten bei Raumtemperatur einen Festkörper darstellen,
und/oder der
- Ester aus gesättigten unverzweigten Alkancarbonsäuren einer Kettenlänge von 1 bis 60 C-Atomen und gesättigten verzweigten Alkoholen einer Kettenlänge von 1 bis 60 C-Atomen, sofern die Wachskomponente oder die Gesamtheit der Wachskomponenten bei Raumtemperatur einen Festkörper darstellen.

Insbesondere vorteilhaft können die Wachskomponenten aus der Gruppe der C₁₆₋₃₆-Al-kylstearate, der C₁₀₋₄₀-Alkylstearate, der C₂₀₋₄₀Alkylisostearate, der C₂₀₋₄₀-Dialkyldimerate, der C₁₈₋₃₈-Alkylhydroxystearoyistearate, der C20-40 Dialkyldimerate, der C₂₀₋₄₀-Alkylerucate gewählt werden, ferner C₃₀₋₅₀-Alkylbienenwachs, Cetylpalmitat, Methylpalmitat, Cetearylbehenat, Octacosanyl Stearate. Auch Siliconwachse wie beispielsweise Stearyltrimethylsilan/Stearylalkohol sind gegebenenfalls vorteilhaft.

Vorteilhaft im Sinne der vorliegenden Erfindung sind außerdem Esterwachse, die Ester aus
1. gesättigten und/oder ungesättigten, verzweigten und/oder unverzweigten Mono- und/oder Dicarbonsäure mit 10 bis 50 Kohlenstoffatomen, bevorzugt 15 - 45 Kohlenstoffatomen und
2. Glycerin
darstellen. Dabei können Mono-, Di- und Triglyceride vorteilhaft sein.

Besonders vorteilhaft sind die im folgenden aufgelisteten Glyceride:

| Glycerid | Handelsname | erhältlich bei |
|---|---|---|
| C₁₆₋₁₈ -Triglycerid | Cremeol HF-52-SPC | Aarhus Oliefabrik |
| Glycerylhydroxystearat | Naturchem GMHS | Rahn |
| Hydrierte Coco-Glyceride | Softisan 100 | Hüls AG |
| Caprylisäure/Caprinsäure/Isostearinsäure/ Adipinsäure Triglycerid | Softisan 649 | Dynamit Nobel |
| C₁₈₋₃₆ Triglycerid | Syncrowax HGLC | Croda GmbH |
| Glyceryltribehenat | Syncrowax HRC | Croda GmbH |
| Glyceryl-tri-(12-hydroxystearat) | Thixcin R | Rheox/NRC |
| Hydriertes Ricinusöl | Cutina HR | Henkel KGaA |
| C₁₆₋₂₄-Triglycerid | Cremeol HF-62-SPC | Aarhus Oliefabrik |

Besonders bevorzugt ist, die Wachskomponenten zu wählen aus der Gruppe der Triglyceridwachse wie C18-38 Triglycerid oder Tribehenin zu wählen.

Ferner hat sich herausgestellt, daß ethoxylierte Wachse wie beispielsweise PEG-8 Bienenwachs, PEG 6 Sorbitanbienenwachs, PEG-2 hydrogeniertes Castoroil, PEG-12 Carnaubawachs vorteilhaft sind, da sie die Stiftmatrix weicher machen und ferner eine bessere Solublisierung wasserlöslicher Inhaltsstoffe ermöglichen.

Ferner hat sich herausgestellt, daß neben des Einsatzes einer der vorab beschriebenen Wachse bestimmte Wachskombinationen vorteilhaft sind.

Es ist von Vorteil, das Verhältnis von Öl- und Wachskomponenten zueinander aus dem Bereich der Gewichtsverhältnisse zwischen 3 : 1 bis 1 : 3, ganz besonders bevorzugt 2 : 1 bis 1 : 2, einzustellen.

Es ist von größerem Vorteil, Emulsionsstifte mit Gehalt von über 10 Gew.-% Wasser auszustatten insbesondere dann, wenn wasserlösliche oder wasserdispergierbare Wirkstoffe wie Hautbefeuchtungsmittel, Anti-Akne-Mittel, UV-Filter, Abdeckpigmente, und wasserdispergierbare Pigmente in dem Fachmann bekannten Konzentrationen eingesetzt werden sollen.

Als Hautbefeuchtungsmittel lassen sich vorteilhaft Glycerin, Chitosan, Fucogel, Propylenglycol, Dipropylenglycol, Butylenglycol, Mannitol, Milchsäure, Natriumpyrolidoncarbonsäure, Hyaluronsäure, Salze der angegebenen Säuren sowie Glycin, Harnstoff und Salze von Metallen der ersten und zweiten Hauptgruppe verwenden.

Besonders geeignet sind Glycerin, Milchsäure, Butylenglycol, Harnstoff, Hyaluronsäure, ganz besonders bevorzugt ist dabei Glycerin.

Der Gehalt an Hautbefeuchtungsmitteln beträgt4 bis 40 Gew.-%, insbesondere 5 bis 40 Gew.-%, bezogen auf das Gesamtgewicht der Zubereitungen.

Die erfindungsgemäßen wasserreichen Stifte sind hervorragende Vehikel für dermatologische Wirkstoffe. Insbesondere eignen sie sich als Träger für gegen Akne wirksame Substanzen. So ist es vorteilhaft, den erfindungsgemäß verwendeten Zubereitungen gegen Akne wirksame Substanzen zuzugeben, beispielsweise gegen *Propionibacterium acnes* wirksame Stoffe (etwa solche, die in DE-OS 42 29 707, DE-OS 43 05 069, DE-OS 43 07 976, DE-OS 43 37 711, DE-OS 43 29 379 beschrieben werden) aber auch andere gegen Akne wirksame Substanzen, beispielsweise all-trans-Retinsäure, 13-cis-Retinsäure und verwandte Stoffe) oder antientzündliche Wirkstoffe, beispielsweise Batylalkohol (α-Octadecylglycerylether), Selachylalkohol (α-9-Octadecenylglycerylether), Chimylalkohol (α-Hexadecylglycerylether), Bisabolol, saure Aluminium- und/oder Zirkoniumsalze, Glycerylcaprinat, 2-Butyloktansäure, Milchsäure, Salicylsäure, Zinksalze, Citronensäure, Diglycerinmonocaprinat, Glycerylcaprylat, Polyglyceryl-3-caprylat und/oder Octoxyglycerin. Besonders bevorzugte Anti-Akne Wirkstoffe davon sind saure Aluminium- und/oder Zirkoniumsalze, Milchsäure, Salicylsäure, Zinksalze, Citronensäure, Diglycerinmonocaprinat, Glycerylcaprinat, 2-Butyloktansäure, Glycerylcaprylat, Polyglyceryl-3-caprylat und/oder Octoxyglycerin. Insbesondere sind auch Kombinationen der genannten Wirkstoffe vorteilhaft.

Die Menge der Antiaknemittel (eine oder mehrere Verbindungen) in den Zubereitungen beträgt vorzugsweise 0,01 bis 30 Gew.-%, besonders bevorzugt 0,1 - 20 Gew.-%, insbesondere 1-10 Gew.-%, bezogen auf das Gesamtgewicht der Zubereitung.

Ferner zeichnet sich das erfindungsgemäße Verfahren dadurch aus, daß zur Herstellung der erfindungsgemäßen Stifte eine Vielzahl von Emulgatoren beziehungsweise Ölkomponenten eingesetzt werden können.

Erfindungsgemäße W/O-Emulgatoren sind oben genannt.

Es kann erfindungsgemäß von Vorteil sein, dass auch andere polyethoxylierte und/oder polypropoxylierte Emulgatoren Verwendung finden, beispielsweise polyethoxyliertes hydrogeniertes oder nichthydrogeniertes Ricinusöl, ethoxyliertes Cholesterin, ethoxlierte Fettalkohole wie Steareth- 2, ethoxylierte Fettsäuren, ethoxylierte Dicarbonsäuren, ethoxylierte Wachse wie PEG (-6, -8, -12, -20) Bienenwachs, PEG (-6, -8, - 20 Sorbitanbienenwachs), ethoylierte Carnaubawachse (PEG-12 Carnaubawachs).

Insbesondere vorteilhafte W/O-Emulgatoren sind Glyceryllanolat, Glycerylmonostearat, Glycerylmonoisostearat, Glycerylmonomyristat, Glycerylmonooleat, Diglycerylmonostearat, Diglycerylmonoisostearat, Diglyceryldiisostearat, Propylenglycolmonostearat, Propylenglycolmonoisostearat, Propylenglycoldiisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, 2-Ethylhexylglycerinether, Selachylalkohol, Chimylalkohol, Polyethylenglycol(2)stearylether (Steareth-2), Glycerylmonolaurat, Glycerylmonocaprinat, Glycerylmonocaprylat, Glycerylsorbitanstearat Polyglyceryl-4 Isostearat, Polyglyceryl-2-sesquiisostearat, PEG-7 hydrogeniertes Castoröl, PEG-40-Sorbitanperisostearat, Isostearyldiglycerylsuccinat, PEG-5-Cholesterylether, Triglycerindiisostearat.

Der erfindungsgemäß verwendete W/O-Emulgator bzw. die erfindungsgemäß verwendeten W/O-Emulgatoren, welcher oder welche in das Schema A-B-A' passen, liegt bzw. liegen vorteilhaft in Konzentrationen von 0,1 - 5 Gew.-%, jeweils bezogen auf das Gesamtgewicht der Zusammensetzung. Es ist vorteilhaft, die Gesamtkonzentration der W/O-Emulgatoren, was auch diejenigen Emulgatoren einschließt, die nicht in das Schema A-B-A' passen, nicht größer als etwa 25 - 30 Gew.-% und nicht geringer als etwa 0,1 Gew.-% zu wählen, jeweils bezogen auf das Gesamtgewicht der Zubereitungen. Es kann erfindungsgemäß von Vorteil sein, daß die vorstehend genannten Typen von W/O-Emulgatoren zusätzlich polyethoxyliert und/oder polypropoxyliert sind, oder daß auch andere polyethoxylierte und/oder polypropoxylierte Produkte Verwendung finden, beispiels-weise polyethoxyliertes hydrogeniertes oder nichthydrogeniertes Ricinusöl, ethoxyliertes Cholesterin, ethoxlierte Fettalkohole wie Steareth- 2.

Die erfindungsgemäß verwendeten Stabilisatoren werden erfindungsgemäß vorteilhaft gewählt aus der Gruppe der Substanzen der allgemeinen Formel 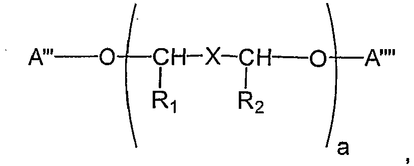 wobei
- A"' und A"" gleiche oder verschiedene hydrophobe organische Reste darstellen,
- a eine Zahl von 1 bis 100, vorzugsweise 2 bis 60, darstellt,
- X eine Einfachbindung oder die Gruppe 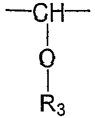
- darstellt,
- R₁ und R₂ unabhängig voneinander aus der Gruppe H, Methyl gewählt werden daß aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1 - 20 Kohlenstoffatomen.

Die Strukturformel ist nicht so zu interpretieren, daß durch den Index a alle in der Klammer repräsentierten Reste R₁, R₂ bzw R₃ im gesamten Molekül jeweils gleich sein müssen. Vielmehr können diese Reste in jedem der a Fragmente 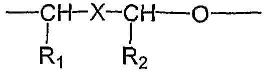 frei gewählt werden.

Die Reste A"' und A"" können gleich oder verschieden sein und werden bevorzugt gewählt aus der Gruppe 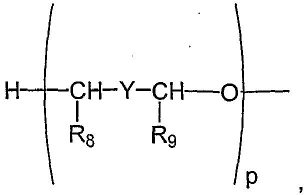 wobei R₈ und R₉ gleich oder verschieden sein können und gewählt werden aus der Gruppe der gesättigten und ungesättigten Alkyl- und Acylreste mit 1 - 30 Kohlenstoffatomen, p eine Zahl von 1 - 20 darstellt und Y eine Einfachbindung oder die Gruppe 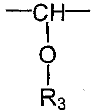 darstellt, wobei R₃ gewählt wird aus der Gruppe H, sowie der verzweigten und unverzweigten, gesättigten und ungesättigten Alkyl- und Acylreste mit 1 - 30 Kohlenstoffatomen.

Bevorzugter Stabilisator ist das PEG-45 /Dodecylglycolcopolymer, welches die Struktur 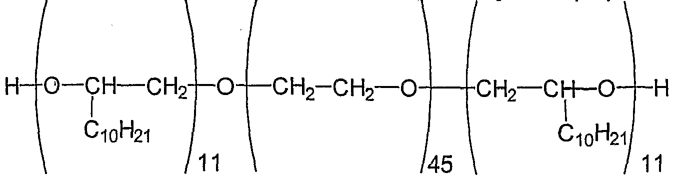 besitzt. Es wird von der. Gesellschaft Akzo Nobel Chemicals GmbH unter der Bezeichnung ELFACOS® ST 9 angeboten. Aber auch das entsprechende PEG-22 / Dodecylglycolcoplymer ist vorteilhaft zu verwenden.

Ferner können die Gruppe A"' und A""unabhängig voneinander auch Alkylreste oder Acylreste darstellen. Besonders vorteilhaft ist auch als Stabilisator das Methoxy-PEG-22-Dodecyl Glycol Copolymer zu verwenden. Es wird von der Gesellschaft Akzo Nobel Chemicals GmbH unter der Bezeichnung ELFACOS® E 200 angeboten.

Der Stabilisator bzw. die Stabilisatoren liegen in Konzentrationen von 0,01 - 5 Gew.-%, jeweils bezogen auf das Gesamtgewicht der Zusammensetzung.

Es ist insbesondere dann vorteilhaft, Stabilisatoren zu wählen, wenn erfindungsgemäße Zubereitungen einen hohen Gehalt an destabilisierenden Substanzen, beispiels-weise Lichtschutzfilter enthalten sollen. Ist der Gehalt an destabilisierenden Substanzen gering, kann man auf den Stabilisator verzichten.

Im Falle eines Gehaltes an mindestens einem Pigment und/oder mindestens einem Farbstoff und/oder mindestens einem Puderstoff können die erfindungsgemäßen Emulsionen oder Stifte vorteilhaft einen oder mehrere Emulgatoren enthalten, welche vorteilhaft gewählt werden aus der Gruppe der folgenden oberflächenaktiven Substanzen des Typs A-B-A'

Dabei ist es bevorzugt, wenn der W/O-Emulgator oder die W/O-Emulgatoren gewählt werden aus der Gruppe der Substanzen der allgemeinen Formel 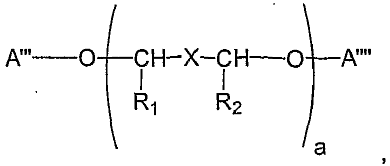 wobei
- A"' und A"" gleiche oder verschiedene hydrophobe organische Reste darstellen,
- a eine Zahl von 1 bis 100, vorzugsweise 2 bis 60, darstellt,
- X eine Einfachbindung oder die Gruppe 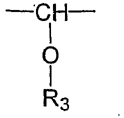
- darstellt,
- R₁ und R₂ unabhängig voneinander aus der Gruppe H, Methyl gewählt werden, dass aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1 - 20 Kohlenstoffatomen,
- wobei die Reste A"' und A"" können gleich oder verschieden sein und gewählt werden aus der Gruppe 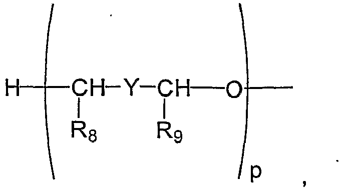
- wobei R₈ und R₉ gleich oder verschieden sein können und gewählt werden aus der Gruppe der gesättigten und ungesättigten Alkyl- und Acylreste mit 1 - 30 Kohlenstoffatomen, p eine Zahl von 1 - 20 darstellt und Y eine Einfachbindung oder die Gruppe 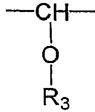 darstellt,
- wobei R₃ gewählt wird aus der Gruppe H, sowie der verzweigten und unverzweigten, gesättigten und ungesättigten Alkyl- und Acylreste mit 1 - 30 Kohlenstoffatomen.

Ferner können im Falle eines Gehaltes an mindestens einem Pigment und/oder mindestens einem Farbstoff und/oder mindestens einem Puderstoff die Gruppe A"' und A"" unabhängig voneinander auch Alkylreste oder Acylreste darstellen.

Bevorzugt wird dabei als Stabilisator das PEG-45 /Dodecylglycolcopolymer und/oder das PEG-22 / Dodecylglycolcopolymer, PEG-30 Dipolyhydroxystearate und/oder das Methoxy PEG-22/Dodecyl Glycol Copolymer verwendet.

Vorteilhaft ist es dabeiweiterhin, wenn zusätzlich Co-Emulgatoren enthalten sind, die gewählt werden aus der Gruppe der Substanzen der allgemeinen Formel 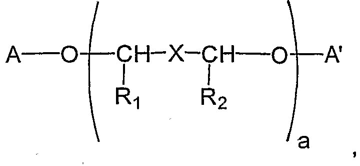 wobei
- A und A' gleiche oder verschiedene hydrophobe organische Reste darstellen,
- a eine Zahl von 1 bis 100, vorzugsweise 2 bis 60, insbesondere 5 bis 40 darstellt,
- X eine Einfachbindung oder die Gruppe 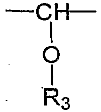
- darstellt,
- R₁ und R₂ unabhängig voneinander so gewählt werden H, Methyl, dass aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1 - 20 Kohlenstoffatomen,
oder dass der oder die W/O-Co-Emulgatoren gewählt werden aus der Gruppe der Fettalkohole mit 8 - 30 Kohlenstoffatomen, Monoglycerinester gesättigter und/oder ungesättigter, verzweigter und/oder unverzweigter Alkancarbonsäuren einer Kettenlänge von 8 - 24, insbesondere 12 - 18 C-Atomen, Diglycerinester gesättigter und/oder ungesättigter, verzweigter und/oder unverzweigter Alkancarbonsäuren einer Kettenlänge von 8 - 24, insbesondere 12 - 18 C-Atomen, Triglycerinester gesättigter und/oder ungesättigter, verzweigter und/oder unverzweigter Alkancarbonsäuren einer Kettenlänge von 8 - 24, insbesondere 12 - 18 C-Atomen, Polyglycerinester gesättigter und/oder ungesättigter, verzweigter und/oder unverzweigter Alkancarbonsäuren einer Kettenlänge von 8 - 24, insbesondere 12 - 18 C-Atomen mit bis zu 10 Glycerineinheiten, Monoglycerinether gesättigter und/oder ungesättigter, verzweigter und/oder unverzweigter Alkohole einer Kettenlänge von 8 - 24, insbesondere 12-18 C-Atomen, Diglycerinether gesättigter und/oder ungesättigter, verzweigter und/oder unverzweigter Alkohole einer Kettenlänge von 8 - 24, insbesondere 12 - 18 C-Atomen, Triglycerinether gesättigter und/oder ungesättigter, verzweigter und/oder unverzweigter Alkohole einer Kettenlänge von 8 - 24, insbesondere 12 - 18 C-Atomen, Polyglycerinether gesättigter und/oder ungesättigter, verzweigter und/oder unverzweigter Alkancarbonsäuren einer Kettenlänge von 8 - 24, insbesondere 12 - 18 C-Atomen mit bis zu 10 Glycerineinheiten, Propylenglycolester gesättigter und/oder ungesättigter, verzweigter und/oder unverzweigter Alkancarbonsäuren einer Kettenlänge von 8 - 24, insbesondere 12 - 18 C-Atomen, Sorbitanester gesättigter und/oder ungesättigter, verzweigter und/oder unverzweigter Alkancarbonsäuren einer Kettenlänge von 8 - 24, insbesondere 12 - 18 C-Atomen, Sorbitanester von Polyolen, insbesondere des Glycerins, Pentaerythritylester gesättigter und/oder ungesättigter, verzweigter und/oder unverzweigter Alkancarbonsäuren einer Kettenlänge von 8 - 24, insbesondere 12 - 18 C-Atomen, Methylglucose Ester gesättigter und/oder ungesättigter, verzweigter und/oder unverzweigter Alkancarbonsäuren einer Kettenlänge von 8 - 24, insbesondere 12 - 18 C-Atomen, Polyglycerin Methylglucose Ester gesättigter und/oder ungesättigter, verzweigter und/oder unverzweigter Alkancarbonsäuren einer Kettenlänge von 8 - 24, insbesondere 12 - 18 C-Atomen oder dass die vorstehend genannten Typen von W/O-Emulgatoren zusätzlich in der Weise polyethoxyliert und/oder polypropoxyliert sind, dass sie ethoxylierte und/oder propopoxylierte W/O-Emulgatoren darstellen.

Besonders bevorzugt ist es im Falle eines Gehaltes an mindestens einem Pigment und/oder mindestens einem Farbstoff und/oder mindestens einem Puderstoff , wenn der W/O-Co-Emulgator oder die W/O-Co-Emulgatoren so gewählt werden, dass die Reste A und A' werden vorteilhaft gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema. 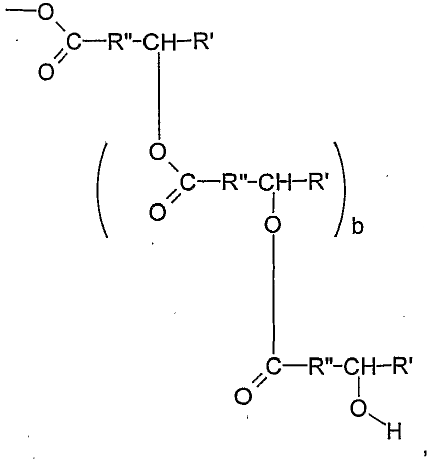 wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann.

Ganz besonders bevorzugt ist es im Falle eines Gehaltes an mindestens einem Pigment und/oder mindestens einem Farbstoff und/oder mindestens einem Puderstoff, wenn der oder die Co-Emulgatoren gewählt werden aus der Gruppe Decaglycerylheptaoleat, Polyglyceryl-3-Diisostearat, PEG-8 Distearat, Diglycerin Dipolyhydroxystearat; als besonders geeignet hat sich der Einsatz von einem Emulgator / Co-Emulgator - Gemisch im Bereich von 1 bis 8 Gew.% erwiesen, besonders bevorzugt ist die Einsatzkonzentration von 26%, Dabei werden die Verhältnisse von Emulgator zu Co-Emulgator vorzugsweise von 1:0 bis 1:1 gewählt.

Außerdem können die erfindungsgemäßen Emulsionen Farbstoffe und/oder Pigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Die einsetzbaren Pigmente können organischen und anorganischen Ursprungs sein, wie beispielsweise organische vom Azo-Typ, Indigoide, Triphenylmethan-artige, Anthrachinone, und Xanthin Farbstoffe, die als D&C and FD&C blues, browns, greens, oranges, reds, yellows bekannt sind. Anorganische bestehen aus unlöslichen Salzen von zertifizierten Farbstoffen, die als Lakes oder Eisenoxide bezeichnet werden.

Farbige Pigmente: Anorganische und Organische: Barium lakes, calcium lakes, aluminum lakes, titandioxide, mica and iron oxides. Als Al-Salze sind z.B Red 3 Aluminum Lake, Red 21 Aluminum Lake, Red 27 Aluminum Lake, Red 28 Aluminum Lake, Red 33 Aluminum Lake, Yellow 5 Aluminum Lake, Yellow 6 Aluminum Lake, Yellow 10 Aluminum Lake, Orange 5 Aluminum Lake, Blue 1 Aluminum Lake und Kombinationen einsatzbar.

Als Eisenoxide oder -oxidhydrate sind z.B. cosmetic yellow oxide C22-8073 (Sunchemical) cosmetic oxide MC 33-120 (Sunchemical), cosmetic brown oxide C33-115 (Nordmann& Rassmann), cosmetic russet oxide C33-8075 (Sunchemical) bekannt und gegebenfalls vorteilhaft. Als Alumosilicat ist ultramarinblau (Les colorants Wacker) einsetzbar.

Auch Perlglanzpigmente lassen sich in die erfindungsgemäßen Emulsionen einarbeiten. Dazu zählen natürliche Perlglanzpigmente, wie z. B.
▪ "Fischsilber" (Guanin/Hypoxanthin-Mischkristalle aus Fischschuppen) und
▪ "Perlmutt" (vermahlene Muschelschalen),
monokristalline Perlglanzpigmente wie z. B. Bismuthoxychlorid (BiOCl),
Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Diese sind beipsielsweise von den Firmen Costenoble (Cloisonne-Typ, Flamenco-Typ, Low Lustre-Typ), Merck (Colorona-Typen, Microna-Typ, Timiron-Typ, Colorona, Ronasphere), Les Colornats Wacker (Covapure, Vert oxyde de Chrome), Cadre (Colorona, Sicopearl), BASF (Sicopearl, Sicovit), Rona (Colorona) bekannt. Als besonders vorteilhaftes Perlglanzpigment haben sich beispielsweise Timiron Silk Gold und Colrona Red Gold bewährt.

Vorteilhaft sind ferner beispielsweise die folgenden Perlglanzpigmentarten auf Basis von Glimmer/Metalloxid:

| 1.1.1.1.1 Gruppe | **1.1.1.2 Belegung / Schichtdicke** | **1.1.1.3 Farbe** |
|---|---|---|
| **Silberweiße Perlglanzpigmente** | TiO₂: 40 - 60 nm | silber |
| **Interferenzpigmente** | TiO₂ 60 - 80 nm | gelb |
| | TiO₂ 80-100 nm | rot |
| | TiO₂: 100 -140 nm | blau |
| | TiO₂: 120 -160 nm | grün |
| **Farbglanzpigmente** | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| **Kombinationspigmente** | TiO₂ / Fe₂O₃ | Goldtöne |
| | TiO₂ / Cr₂O₃ | grün |
| | TiO₂ / Berliner Blau | tiefblau |
| | TiO₂ / Carmin | rot |

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von SiO₂ hergestellt werden. Solche Pigmente, die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihrer Partikelgröße von 40 - 180 µm zusätzlich zu der Farbe einen Glitzereffekt auf.

Die erfindungsgemäßen kosmetischen und dermatologischen Zubereitungen können Farbstoffe und/oder Farbpigmente enthalten, insbesondere wenn sie in Form von dekorativen Lippenstiften, Lippenkonturenstiften, Abdeckstiften, Kajalstiften, Augenkonturenstiften und/oder Augenbrauenstiften vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. (Die Stoffe sind nach ihrer Colour Index Number geordnet.)
Die Colour Index Nummern (CIN) sind dem Rowe Colour Index, 3. Auflage, Society of Dyers and Colourists, Bradford, England, 1971 entnommen.

| **Chemische oder sonstige Bezeichnung** | **CIN** | **Farbe** |
|---|---|---|
| Pigment Green | 10006 | grün |
| Acid Green 1 | 10020 | grün |
| 2,4-Dinitrohydroxynaphthalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxynaphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8-trisulfosäure | 16290 | rot |
| 8-Amino-2-phenylazo-1-naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2", 4"-dimethyl)-bis-phenylazo)1,3-dihydroxy-benzol | 20170 | orange |
| Acid Black 1 | 20470 | schwar Z |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1 "-phenylazo)-7'-sulfo-1'-naphthylazo]-1-hydroxy-7-aminonaphthalin-3,6-disulfosäure | 27755 | schwarz |
| 4'-[(4"-Sulfo-1 "-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-ß-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenylcarbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N-ethy)-N-p-sulfo-benzyl)Δ^{2,5}-cyclohexadienimin | 42090 | blau |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylamino-naphthofuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂ 7 H20 | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactofiavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |
| Bromkresofgrün | | grün |
| Acid Red 195 | | rot |

Besonders bevorzugt ist der Einsatz von anorganischen Farbpigmenten wie rotes und schwarzes Eisenoxid (CIN: 77 491 (rot) und 77 499 (schwarz)), Eisenoxidhydrat (CIN: 77 492), Manganammoniumdiphosphat, Ultramarin, Chromoxid und Chromhydroxid, Eisenhexacyanoferrat und Titandioxid.
Erfindungsgemäße Zubereitungen enthalten dabei ein Titandioxid, das sowohl in der Kristallmodifikation Rutil als auch Anatas vorliegen kann und im Sinne der vorliegenden Erfindung vorteilhaft oberflächlich behandelt ("gecoatet") ist, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆ Natriummetaphosphat (NaPO₃)ₙ, Siliciumdioxid (SiO₂) (auch: Silica, CAS-Nr.: 7631-86-9), Zirkoniumoxid (ZrO₂) oder Eisenoxid (Fe₂O₃). Diese anorganischen Oberflächenbeschichtungen können allein, in Kombination und/oder in Kombination mit organischen Beschichtungsmaterialien vorkommen.
Hierzu werden Oxide, Oxidhydrate oder Phosphate beispielsweise der Elemente Al, Si, Zr in dichten Schichten auf die Pigmentoberfläche aufgefällt.
Die anorganische Nachbehandlung geschieht im allgemeinen in einer wässrigen Suspension des Pigmentes durch Zugabe löslicher Nachbehandlungschemikalien, wie z.B. Aluminiumsulfat, und anschließende Ausfällung des im neutralen Bereich schwerlöslichen Hydroxides durch gezielte Einstellung des pH-Wertes mit Natronlauge. Nach der anorganischen Nachbehandlung werden die gecoateten Pigmente durch Filtration aus der Suspension abgetrennt und sorgfältig gewaschen, um die gelösten Salze zu entfernen, anschließend werden die isolierten Pigmente getrocknet.

Besonders bevorzugt im Sinne dieser Erfindung sind Titandioxide, auf die Aluminiumhydroxid auf die Oberfläche aufgebracht worden ist, wie z.B. die von Sun Chemical erhältlichen Titandioxid Typen C47-051 und C4- 5157. Weiterhin bevorzugte Pigmente sind Titandioxide, die mit Aluminium- und / oder Siliziumoxiden gecoated sind, wie z.B. von der Firma Krosnos Titan: Kronos 1071 und 1075 oder von der Firma Kingfisher: A310.03 Tudor Aspen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel). oder Alginsäure. Diese organischen Oberflächenbeschichtungen können allein, in Kombination und/oder in Kombination mit anorganischen Beschichtungsmaterialien vorkommen.

Die Farbstoffe und Pigmente können sowohl einzeln als auch im Gemisch vorliegen sowie gegenseitig miteinander beschichtet sein, wobei durch unterschiedliche Beschichtungsdikken im allgemeinen verschiedene Farbeffekte hervorgerufen werden.Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Die Liste der genannten Farbstoffe und Farbpigmente, die in den erfindungsgemäßen Emulsionsstiften verwendet werden können, soll selbstverständlich nicht limitierend sein.

Füllstoffe im Sinne der vorliegenden Erfindung sind partikuläre Substanzen, die in der Regel keinen Farbeffekt in der kosmetischen Formulierung erzeugen, in der sie eingesetzt werden. Ferner haben erfindungsgemäße Füllstoffe üblicherweise einen niedrigen Brechungsindex und daraus resultierend keine oder eine nur sehr geringe Deckkraft.

Der Stand der Technik kennt eine Reihe von Füllstoffen, welche z. B. als Trägermaterialien bei der Formulierung von Pudern oder als Viskositäts- und Sensorik-Modulatoren in Emulsionen oder wasserfreien Formulierungen dienen. Derartige Füllstoffe werden häufig auch eingesetzt, um mattierende Effekte auf der Haut zu erlangen oder um Sebum zu absorbieren.

Darüber hinaus beeinflusst der Einsatz von Füllstoffen im allgemeinen auch die Verteilbarkeit üblicher Formulierungen auf der Haut sowie die Gleichmäßigkeit eines möglichen Farbeffektes.

Die Füllstoffe im Sinne der vorliegenden Erfindung werden vorteilhaft aus der Gruppe der anorganische Füllstoffe gewählt, beispielsweise aus der Gruppe der Silikate.

Silikate sind Salze und Ester (Kieselsäureester) der Orthokieselsäure [Si(OH)₄] und deren Kondensationsprodukte. Die Silikate sind nicht nur die artenreichste Klasse der Mineralien, sondern auch geologisch und technisch außerordentlich wichtig. Über 80 % der Erdkruste bestehen aus Silikaten.

Vorteilhaft im Sinne der vorliegenden Erfindung sind beispielsweise Schichtsilikate. Schichtsilikate (Phyllosilikate, Blattsilikate) sind (idealerweise) Silikat-Strukturen mit zweidimensional unendlichen Schichten aus [SiO₄]⁴⁻-Tetraedern, wobei jedes Tetraeder über 3 Brücken-Sauerstoffe mit Nachbar-Tetraedern verbunden ist.

Chemische Formeln lassen sich für Schichtsilikate nur angenähert aufstellen, da sie ein großes lonenaustausch-Vermögen besitzen und Silicium gegen Aluminium und dieses wiederum gegen Magnesium, Fe²⁺, Fe³⁺, Zn und dergleichen ausgetauscht werden kann. Die daraus möglicherweise resultierende negative Ladung der Schichten wird in der Regel durch Kationen, insbesondere durch Na⁺ und Ca²⁺ in Zwischenschicht-Positionen ausgeglichen.

Schichtsilikate können durch reversible Einlagerung von Wasser (in der 2- bis 7-fachen Menge) und anderen Substanzen wie z. B. Alkoholen, Glykolen und dergleichen mehr aufquellen. Ihre Verwendung als Verdickungsmittel in kosmetischen Mitteln ist dementsprechend an sich bekannt. Allerdings konnte der Stand der Technik nicht den Weg zur vorliegenden Erfindung weisen.

Vorteilhafte Schichtsilikate, welche im Sinne der vorliegenden Erfindung eingesetzt werden können, sind beispielsweise solche, deren größte Ausdehnungsrichtung im unmodifizierten und ungequollenen Zustand im Mittel eine Länge von weniger als 10 µm hat. Beispielsweise können die mittleren Ausdehnungen der verwendeten modifizierten Schichtsilikatpartikel bei 1000 nm x 100 nm x 1 nm und darunter liegen. Die effektive Größe der modifizierten Schichtsilikatpartikel in einer kosmetischen oder dermatologischen Formulierung hängt selbstverständlich von der Menge an eingelagerten Substanzen ab.

Erfindungsgemäß vorteilhafte (Schicht-) Silikate sind insbesondere:
⇒ Talkum: Mg₃ [Si₄O₁₀] (OH)₂,
⇒ Kaolin: Al₂[Si₂O₅] (OH)₄
⇒ Montmorillonit: M⁺ Al [Si₂O₅](OH), auch Smektite genannt. Darunter fallen:
   - Bentonite = Montmorillonite mit Ca (Fuller Erden) oder Na (Wyoming Bentonite)
   - Hektorite: M⁺_{0,3}(Mg_{2,7}Li_{0,3})[Si₄O₁₀(OH)₂], worin M⁺ meist Na⁺ darstellt,
   - Glimmer (Mica), ein Alumosilikat, das leicht spaltbar ist und in tafeligen Kristallen vorliegt. Glimmer ist transparent bis durchscheinend und weist Perlglanz auf. Die wichtigste Form ist Muskovit: K Al₂ [AlSi₃O₁₀] (OH, F)₂. Sericite ist eine Sonderform des Glimmers, die kleinere Plättchen als Muskovit aufweist.

Auch Siliciumoxide (SiO₂) sind vorteilhaft im Sinne der vorliegenden Erfindung zu verwenden. Erfindungsgemäß bevorzugt sind beispielsweise Aerosile (fumed Silica), welche hochdisperse Kieselsäuren mit häufig irregulärer Form sind, deren spezifische Oberfläche in der Regel sehr groß ist (200 - 400 m² / g) und mit Hilfe des Herstellverfahrens gesteuert werden kann. Aerosile werden auch bezeichnet als: Amorphous Silica Amorphous Silicon Oxide Hydrate Silica, Amorphous Silicic Anhydride Silicon Dioxide Silicon Dioxide.

Erfindungsgemäß vorteilhafte Aerosile sind z. B. unter den folgenden Handlesnamen erhältlich:
Aerosil 130 (Degussa Hüls), Aerosil 200 (Degussa Hüls), Aerosil 255 (Degussa Hüls),
Aerosil 300 (Degussa Hüls), Aerosil 380 (Degussa Hüls), B-6C (Suzuki Yushi), CAB-O-SIL Fumed Silica (Cabot), CAB-O-SIL EH-5 (Cabot), CAB-O-SIL HS-5 (Cabot), CAB-O-SIL LM-130 (Cabot), CAB-O-SIL MS-55 (Cabot), CAB-O-SIL M-5 (Cabot), E-6C (Suzuki Yushi), Fossil Flour MBK (MBK), MSS-500 (Kobo), Neosil CT 11 (Crosfield Co.), Ronasphere (Rona/EM Industries), Silica, Anhydrous 31 (Whittaker, Clark & Daniels), Silica, Crystalline 216 (Whittaker, Clark & Daniels), Silotrat-1 (Vevy), Sorbosil AC33 (Crosfield Co.), Sorbosil AC 35 (Crosfield Co.), Sorbosil AC 37 (Crosfield Co.), Sorbosil AC 39 (Crosfield Co.), Sorbosil AC77 (Crosfield Co.), Sorbosil TC 15 (Crosfield Co.), Spherica (Ikeda), Spheriglass (Potters-Ballotini), Spheron L-1500 (Presperse), Spheron N-2000 (Presperse), Spheron P-1500 (Presperse), Wacker HDK H 30 (Wacker-Chemie), Wacker HDK N 20 (Wacker-Chemie), Wacker HDK P 100 H (Wacker Silicones), Wacker HDK N 20P (Wacker-Chemie), Wacker HDK N 25P (Wacker-Chemie), Wacker HDK S 13 (Wacker-Chemie), Wacker HDK T 30 (Wacker-Chemie), Wacker HDK V 15 (Wacker-Chemie), Wacker HDK V 15 P (Wacker-Chemie), Zelec Sil (DuPont).

Siliciumoxide lassen sich auch in sphärischer Form herstellen, wobei hier die spezifische Oberfläche kleiner ist als bei den Aerosilen, da die Teilchen größer und rund sind. Ein Beispiel hierfür sind die Ronaspheren (mittlerer Teilchendurchmesser < 3 µ) der Fa. Merck. Ihr Einsatz ist bevorzugt.

Weitere erfindungsgemäß bevorzugte Füllstoffe sind Siliciumdioxide, deren freien OH-Gruppen an der Teilchenoberfläche (ganz oder teilweise) organisch modifiziert sind.

Vorteilhaft sind z. B. die durch Addition von Dimethylsilyl-Gruppen erhältlichen Silica Dimethyl Silylate, wie beispielsweise Aerosil R972 (Degussa Hüls), Aerosil R974 (Degussa Hüls), CAB-O-SIL TS-610 (Cabot), CAB-O-SIL TS-720 (Cabot), Wacker HDK H15 (Wacker-Chemie), Wacker HDK H18 (Wacker-Chemie) und/oder Wacker HDK H20 (Wacker-Chemie).

Ferner vorteilhaft sind die durch Addition von Trimethylsilylgruppen erhältlichen Silica Silylate (z. B. Aerosil R 812 (Degussa Hüls), CAB-O-SIL TS-530 (Cabot), Sipernat D 17 (Degussa Hüls), Wacker HDK H2000 (Wacker-Chemie)).

Ferner vorteilhaft im Sinne der vorliegenden Erfindung sind die durch Hydrolyse- und Kondensationsreaktionen von Methyltrimethoxysilane erhältlichen Polymethylsilsesquioxane, die ebenfalls eine runde Form besitzen und deren Teilchengrößenverteilung durch die Herstellung gesteuert werden kann.

Bevorzugte Polymethylsilsesquioxane werden beispielsweise unter den Handelsnamen Tospearl 2000 B von GE Bayer Silikones, Tospearl 145A von Toshiba, AEC Silicone Resin Spheres von A & E Connock sowie Wacker - Belsil PMS MK von der Wacker-Chemie angeboten.

**Weiterer vorteilhafter Füllstoff im Sinne der vorliegenden Erfindung ist Bornitrid. Bornitrid ist isoelektronisch mit Kohlenstoff (d. h. es sind Graphit- und Diamantform möglich). Bornitrid zeichnet sich durch seine chemische Inertheit aus.**

Vorteilhaft im Sinne der vorliegenden Erfindung sind beispielsweise die im folgenden aufgelisteten Bornitride:

| **Handelsname** | **erhältlich bei:** |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Tres BN^{®} | Carborundum |
| Wacker-Bornitrid BNP | Wacker-Chemie |

Die Füllstoffe im Sinne der vorliegenden Erfindung werden darüber hinaus vorteilhaft aus der Gruppe der organische Füllstoffe gewählt.

Erfindungsgemäß vorteilhafte organische Füllstoffe sind z. B. natürliche Polymere, wie Seidenpuder, mikrokristalline Cellulose und/oder Zinkstearate.

Vorteilhafte organische Füllstoffe sind ferner Stärke und Stärkederivate, wie:
⇒ Maisstärke Zea Mays (Amidon De Mais MST (Wackherr), Argo Brand Corn Starch (Corn Products), Pure-Dent (Grain Processing), Purity 21C (National Starch)),
⇒ Reisstärke (D.S.A. 7 (Agrana Stärke), Oryzapearl (Ichimaru Pharcos)),
⇒ Distarch Phosphate (Corn PO4 (Agrana Stärke), Corn PO4 (Tri-K)),
⇒ Sodium Corn Starch Octenylsuccinate (C* EmCap - Instant 12639 (Cerestar USA)),
⇒ Aluminium Starch Octenylsuccinate (Covafluid AMD (Wackherr), Dry Flo-PC (National Starch), Dry Flo Pure (National Starch), Fluidamid DF 12 (Roquette)),

Erfindungsgemäß bevorzugte organische Füllstoffe sind auch synthetische Polymere, d. h. Polymerpartikel, welche in der Zubereitung in Form von Feststoffen vorliegen, wie beispielsweise Polycarbonate, Polyether, Polyethylen, Polypropylen, Polyvinylchlorid, Polystyrol, Polyamide, Polyurethane, Polyacrylate und dergleichen mehr. Besonders vorteilhaft ist z. B. die Substanz mit der INCI-Bezeichnung HDI / Trimethylol Hexyllactone Crosspolymer, welche unter der Bezeichnung BPD-500/Plastic Powder D von der Firma Kobo erhältlich ist.

Ferner vorteilhaft im Sinne der vorliegenden Erfindung ist Nylon (Polyamid 6 und Polyamid 12), wie beispielsweise mikrofeine Polyamid-Partikel, insbesondere die unter der Handelsbezeichnung SP-500 bei der Firma TORAY erhältlichen. Ferner vorteilhaft sind Polyamid 6- (auch: Nylon 6), bzw. Polyamid 12- (auch: Nylon 12), Partikel. Polyamid 6 ist das aus ε-Aminocapronsäure (6-Aminohexansäure), oder ε-Caprolactam aufgebaute Polyamid [Poly(ε-caprolactam)], und Polyamid 12 ist ein Poly(ε-laurinlactam), aus ε-Laurinlactam. Vorteilhaft im Sinne der vorliegenden Erfindung sind beispielsweise Orgasol^{®} 1002 (Polyamid 6), und Orgasol^{®} 2002 (Polyamid 12), von der Firma ELF ATOCHEM.

Weitere vorteilhafte organische Füllstoffe sind:
⇒ PMMA: Polymethylmethacrylate
⇒ Polyethylene Spheres
⇒ Polyurethane
⇒ Silikon Resins: Trimethylsiloxysilicate (z. B. SR 1000 GE Bayer Silicones),
⇒ Silikonelastomere
⇒ Polytetrafluorethyen (PTFE)

So kann es z. B. von erheblichem Vorteil sein, solche Silikonelastomeren in Zubereitungen gemäß der vorliegenden Erfindung zu verwenden, wie sie beispielsweise in den US-Patenten US 4980167 oder US 4742142 beschrieben werden. Vorteilhafte Silikonelastomere sind ferner z. B. solche, welche unter den Namen KSG6 von Shin Etsu, Tefil E-505C oder Trefil E-506C von Dow Corning, Gransil von Grant Industries (SR-CYC, SR-DMF10, SR-DC556), vertrieben werden sowie solche, die in Form von vorgefertigten Gelen verkauft werden (wie z. B. KSG15, KSG17, KSG16, KSG18 von Shin Etsu, Gransil SP 5CYC Gel, Gransil SR DMF 10 Gel, Gransil SR DC 556 Gel, Gransil GCM, Gransil PM Gel, Gransil DMG-5, SF 1204 und JK 113 von Gereral Electric). Weitere vorteilhafte Silikonelastomere können gewählt werden aus der Gruppe der Vinyl Dimethicone Crosspolymere, wie z. B. das Dow Corning 9506 Cosmetic Powder von Dow Corning (INCI: Dimethicone / Vinyl Dimethicone Crosspolymer).

Weiterhin vorteilhaft eingesetzt werden können sogenannte Silikonharze, wie z.B. KSP-100, KSP-200 oder KSP-300 von Shin Etsu, die ebenfalls unter der INCI bezeichnung Dimethicone / Vinyl Dimethicone Crosspolymer geführt werden oder SR 1000 von GE Bayer Silicones, das die INCI Bezeichnung Trimethylsiloxy Silicate trägt

Weiterhin bevorzugt ist auch Lauroyl Lysine , das unter der bezeichnung Amihope LL von Ajinomoto vertrieben wird.

Die Gesamtmenge an mindestens einem Füllstoff in den fertigen kosmetischen oder dermatologischen Zubereitungen wird vorteilhaft aus dem Bereich von 0,05 - 20,0 Gew.-%, bevorzugt 0,5 - 10,0 Gew.-% gewählt, bezogen auf das Gesamtgewicht der Zubereitungen.

Weiterhin gehören zu den bevorzugten anorganischen Siliziumverbindungen die and der Oberfläche organisch modifizierten sphärischen Partikel.
Von diesen sind besonders bevorzugt die Polymethylsilsesquioxane und hydrophob modifizierte Aerosile, wie z.B. Aerosil R 972.

Zu den bevorzugten organischen Siliziumverbindungen gehören die Siloxan Elastomere und Siloxan Harze. Von denen sind besonders bevorzugt die KSP-Typen von Shin Etsu, sowie das Trimethylsiloxysilicate.

Weitere erfindungsgemäß bevorzugte Füllstoffe kommen aus der Gruppe der sphärischen Partikel. Besonders bevorzugt ist der mittlere Teilchendurchmesser kleiner als 20 µm. Desweiteren sind bevorzugt sphärische Partikel mit einem mittlerern Teilchendurchmesser kleiner als 10 µm. Davon besonders bevorzugt sind Nylon-12, das z.B. als SP-501 oder SP-500 von der Firma Kobo vertrieben wird. Weiterhin bevorzugt sind Polymethylmethacrylate, die z.B. unter dem Handelsnamen Covabead LH 85 von LCW vertrieben wird.

Weiterhin bevorzugt eingesetzt werden können Lauroyl Lysine und Bismut Oxychlorid.

Ferner ist es erfindungsgemäß dem Stift Koservierungsmittel zuzusetzen.
In der Lebensmitteltechnologie zugelassene Konservierungsmittel, die mit ihrer E-Nummer nachfolgend aufgeführt sind, sind erfindungsgemäß vorteilhaft zu verwenden.

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylpheno lat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner sind erfindungsgemäß in der Kosmetik gebräuchliche Konervierungsmittel oder Konservierungshilfsstoffe Dibromdicyanobutan (2-Brom-2₋brommethylglutarodinitril), 3-lod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol geeignet. Formaldehydabspalter.

Dieses sind erfindungsgemäß in der Kosmetik gebräuchliche Konservierungsmittel oder Konservierungshilfsstoffe, wie sie auch in der Kosmetikverodrnung aufgelistet sind. Besonders bevorzugt werden eingesetzt 3-lod-2-propinylbutyl-carbamatlmidazolidinylharnstoff, Diazolinidylharnstoff (z.B. erhältlich von der Firma ISP Sutton Laboratories unter dem Handelsnamen Germall II), 5-Chlor-2-methyl-4-isothiazolin-3-on und 2-Methyl-4-Isothiazolon, die als Mischung unter den Handelsnamen Kathon CG und Rokonsal S1 vertrieben werden, 1,3-Dimethyloyl-5,5-dimethylhydantoin, welches alleine unter dem Namen Glydant von der Firma Lonza vertrieben wird oder in der Abmischung mit 3-lod-2-propinylbutylcarbamat unter dem Namen Glydant Plus, 2-Ferner sind Phenylhydroxyalkylether, insbesondere die unter der Bezeichnung Phenoxyethanol bekannte Verbindung aufgrund ihrer bakteriziden und fungiziden Wirkungen auf eine Anzahl von Mikroorganismen als Konservierungsmittel geeignet. Weiter bevorzugt ist auch Silberchlorid, welches z.B. von der Firma Johnson Matthey als Abmischung mit Titandioxid unter dem Namen JM Acticare vertrieben wird.

Auch andere keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Aufgrund des hohen Anteils der Wasserphase erfindungsgemäßer Emulsionen lassen sich sowohl große Mengen hydrophiler, als auch hydrophober Wirkstiffe in die Formulierungen einarbeiten. Derartige erfindungsgemäß vorteilhafte Wirkstoffe sind beispielsweise Acetylsalicylsäure, Azulen, Ascorbinsäure, Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die γ-Linolensäure, Ölsäure, Eicosapentaensäure, Docosahexaensäure, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Zusätzlich können Pflegewirkstoffe eingearbeitet werden, welche sich nicht wie bisher auf die fettlöslichen Wirkstoffe beschränken, sondern auch aus der Gruppe der wasserlöslichen Wirkstoffe gewählt werden können, beispielsweise Vitamine und dergleichen mehr.

Besonders bevorzugte Wirkstoffe im Sinne der vorliegenden Erfindung sind α-Glucosylrutin, Coenzym Q10, 3-Hydroxy-4-(trimethylammonio)-buttersäurebetain und Sericosid.

Vorteilhafte Wirkstoffe sind weiterhin Antioxidantien, insbesondere solche, welche nicht nur die Bestandteile der Formulierung, sondern auch die Haut vor oxidativer Beanspruchung schützen können.

Die Zubereitungen enthalten daher vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden. Es ist dabei vorteilhaft, Antioxidantien als einzige Wirkstoffklasse zu verwenden, etwa dann, wenn eine kosmetische oder dermatologische Anwendung im Vordergrunde steht wie die Bekämpfung der oxidativen Beanspruchung der Haut. Es ist aber auch günstig, die erfindungsgemäßen W/O-Emulsionsstifte mit einem Gehalt an einem oder mehreren Antioxidantien zu versehen, wenn die Zubereitungen einem anderen Zwecke dienen sollen, z.B. als Desodorantien oder Sonnenschutzmittel.

Besonders vorteilhaft werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Pentat-, Hexa-, Heptahioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol deren Derivate, Vitamin C und Derivate (z.B. Ascorbylpalmitate, Mg - Ascorbylphosphate, Ascorbylacetate), Isoascorbinsäure und ihre Derivate, Tocopherole und Derivate (z.B. Vitamin E - acetat), Vitamin A und Derivate (Vitamin A - palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können zwar öllösliche oder öldispergierbare Antioxidantien eingesetzt werden. Es hat sich jedoch herausgestellt, daß die Erfindung gerade dem Einsatz wasserlöslicher oder wasserdispergierbarer Antioxidantien in Stiftformulierungen die Pforten öffnet.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001-10 Gew.-%, bezogen auf das Gesamtgewicht der Formulierung, zu wählen.

Erfindungsgemäß können Wirkstoffe auch sehr vorteilhaft gewählt werden aus der Gruppe der lipophilen Wirkstoffe, insbesondere aus folgender Gruppe:
Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die γ-Linolensäure, Ölsäure, Eicosapentaensäure, Docosahexaensäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl®, Eucerit® und Neocerit®.

Die erfindungsgemäßen Stifte tragen ferner in vorzüglicher Weise zur Hautglättung bei, insbesondere, wenn sie mit einer oder mehreren Substanzen versehen sind, die die Hautglättung fördern.

Eine erstaunliche Eigenschaft der erfindungsgemäße Zubereitungen ist, daß diese sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Besonders vorteilhaft werden der oder die Wirkstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen.

Bevorzugter NO-Synthasehemmer ist das Nitroarginin.

Weiter vorteilhaft werden der oder die Wirkstoffe gewählt aus der Gruppe, welche Catechine und Gallensäureester von Catechinen und wäßrige bzw. organische Extrakte aus Pflanzen oder Pflanzenteilen umfaßt, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica.

Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Wirkstoffe im Sinne der vorliegenden Erfindung. Sie sind durch folgende Grundstruktur gekennzeichnet (Substitutionspostitionen angegeben):

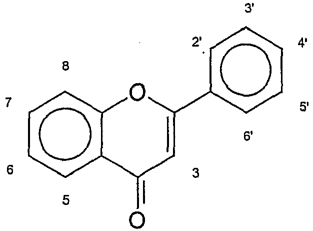

Einige der wichtigeren Flavone, welche auch bevorzugt in erfindungsgemäßen Zubereitungen eingesetz werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt gewählt aus der Gruppe der Substanzen der generischen Strukturformel 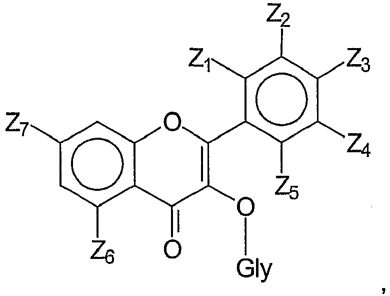 wobei Z₁ bis Z₇ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel 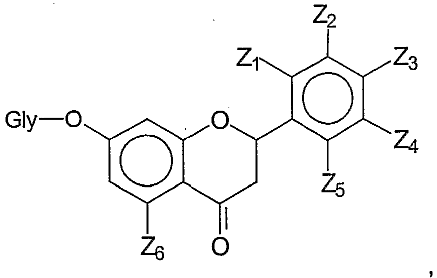 wobei Z₁ bis Z₆ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 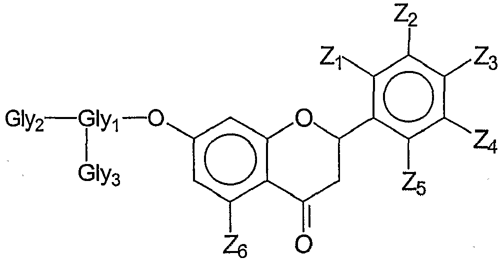 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struktur

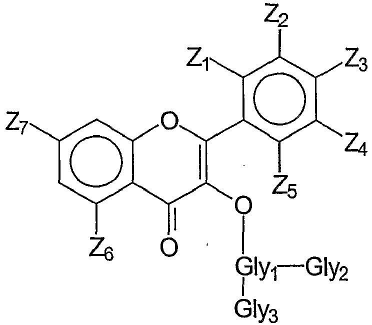

Besonders vorteilhaft werden die erfindungsgemäßen Flavonglycoside aus der Gruppe, welche durch die folgende Struktur wiedergegeben werden: 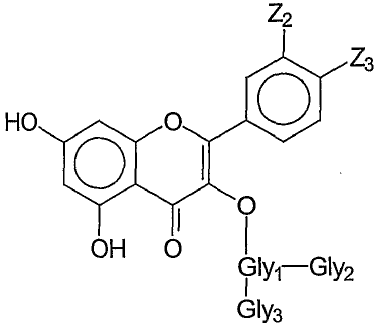 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnogluco-sid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-glucopy-ranosid)), Monoxerutin (3,3',4',5-Tetrahydroxy-7-(2-hydroxyethoxy)-flavon-3-(6-O-(6-de-oxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomareïn (3',4',7,8-Tetrahydroxyflavanon-7-glucosid) und Isoquercetin (3,3',4',5,7-Pentahydroxyflavanon-3-(β-D-Glucopyrano-sid).

Vorteilhaft ist es auch, den oder die Wirkstoffe aus der Gruppe der Ubichinone und Plastochinone zu wählen.

Ubichinone zeichnen sich durch die Strukturformel 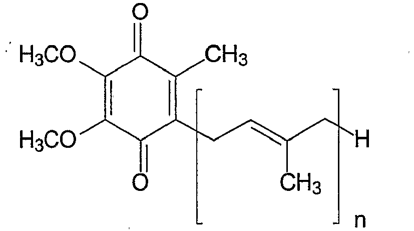 aus und stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen und Hefen mit n=6. Bei den meisten Säugetieren einschließlich des Menschen überwiegt Q10.

Besonders vorteilhaft ist Coenzym Q10, welches durch folgende Strukturformel gekennzeichnet ist:

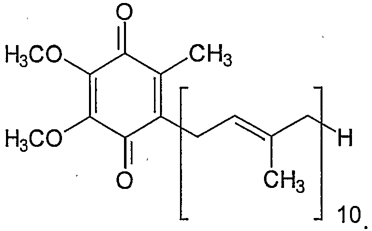

Plastochinone weisen die allgemeine Strukturformel 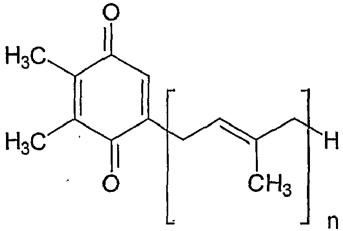 auf. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Auch Kreatin und/oder Kreatinderivate sind bevorzugte Wirkstoffe im Sinne der vorliegenden Erfindung. Kreatin zeichnet sich durch folgende Struktur aus:

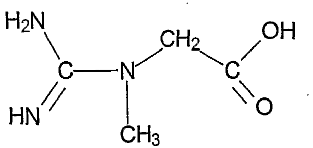

Bevorzugte Derivate sind Kreatinphosphat sowie Kreatinsulfat, Kreatinacetat, Kreatinascorbat und die an der Carboxylgruppe mit mono- oder polyfunktionalen Alkoholen veresterten Derivate.

Ein weiterer vorteilhafter Wirkstoff ist L-Carnitin [3-Hydroxy-4-(trimethylammonio)-buttersäurebetain]. Auch Acyl-Carnitine, welche gewählt aus der Gruppe der Substanzen der folgenden allgemeinen Strukturformel 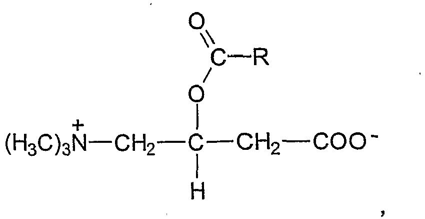 wobei R gewählt wird aus der Gruppe der verzweigten und unverzweigten Alkylreste mit bis zu 10 Kohlenstoffatomen sind vorteilhafte Wirkstoffe im Sinne der vorliegenden Erfindung. Bevorzugt sind Propionylcarnitin und insbesondere Acetylcarnitin. Beide Eritantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.

Weitere vorteilhafte Wirkstoffe sind Pyridoxol, Aminoguadin, Phytochelatin, Isoflavone (Genistein, Daidzein, Daidzin, Glycitin), Niacin, Tyrosinsulfat, Dioic Acid, Adenosin, Pyridoxin, Arginin, Vitamin K, Biotin, Aromastoffe, α-Glucosylrutin, Tocopherol, Liponsäure, Panthenol, Tocopherolacetat, Retinol, Biotin, Vitamin C, Kreatin, Coenzym Q10, 3-Hydroxy-4-(trimethylammonio)-buttersäurebetain und Sericosid.
Die Liste der genannten Wirkstoffe bzw. Wirkstoffkombinationen, die in den erfindungsgemäßen Zubereitungen verwendet werden können, soll selbstverständlich nicht limitierend sein. Die Wirkstoffe können einzelnen oder in beliebigen Kombinationen miteinander verwendet werden.

Vorteilhafte Wirkstoffe sind weiterhin Antioxidantien, insbesondere solche, welche nicht nur die Bestandteile der Formulierung, sondern auch die Haut vor oxidativer Beanspruchung schützen können. Besonders vorteilhafte Antioxidantien sind Urocaninsäure, Carnosin, Carotinoide, Carotine, Liponsäure, α-Hydroxyfettsäuren, α-Hydroxysäuren, Ubichinon. Die Menge der Antioxidantien (eine oder mehrere Verbindungen) in den Zubereitungen beträgt vorzugsweise 0,001 bis 30 Gew.-%, besonders bevorzugt 0,05 - 20 Gew.%, insbesondere 1 -10 Gew.-%, bezogen auf das Gesamtgewicht der Zubereitung.

Es ist gegebenenfalls möglich und vorteilhaft, die erfindungsgemäßen Zubereitungen als Grundlage für pharmazeutische Formulierungen zu verwenden. Mutatis mutandis gelten entsprechende Anforderungen an die Formulierung medizinischer Zubereitungen. Die Übergänge zwischen reinen Kosmetika und reinen Pharmaka sind dabei fließend. Als pharmazeutische Wirkstoffe sind erfindungsgemäß grundsätzlich alle Wirkstoffklassen geeginet, wobei lipophile Wirkstoffe bevorzugt sind. Beispiele sind: Antihistaminika, Antiphlogistika, Antibiotika, Antimykotika, die Durchblutung fördernde Wirkstoffe, Keratolytika, Antihistaminika, Antiphlogistika, Antibiotika, Antimykotika, die Durchblutung fördernde Wirkstoffe, Keratolytika, Hormone, Steroide, Vitamine, Hormone, Steroide, Vitamine usw.

Besonders vorteilhafte Repellent-Wirkstoffe im Sinne der vorliegenden Erfindung sind die obengenannten Wirkstoffe N,N-Diethyl-3-methylbenzamid, 3-(N-n-Butyl-N-acetyl-amino)-propionsäureethylester, 1-Piperidincarbonsäure-2-(2-hydroxyethyl)-1-methylpropylester und Dimethylphthalat.

Es ist auch möglich, zusätzliche Substanzen zu verwenden, welche die Konsistenz der erfindungsgemäßen Zubereitungen modifizieren, beispielsweise Verdicker, welche gewählt werden können aus der Gruppe der Substanzen welche mindestens zwei hydrophile Reste tragen, welche über eine hydrophobe Gruppierung miteinander verbunden sind, also den Molekülschemata 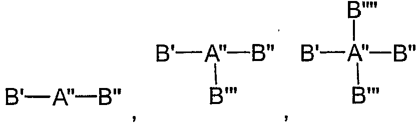 usw. folgen.

Dabei stellen die Reste B mit den verschiedenen Indizes hydrophile Gruppen dar, die Reste A mit den verschiedenen Indizes hydrophobe Gruppen.

Solche Verdicker werden bevorzugt gewählt aus der Gruppe der Triblockcopolymere des Typs 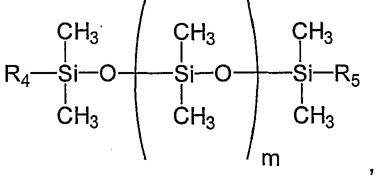 wobei m eine Zahl von 10 bis 10000 darstellen kann, R₄ und R₅ gleich oder verschieden sein können und gewählt werden aus der Gruppe, die durch die allgemeine Struktur 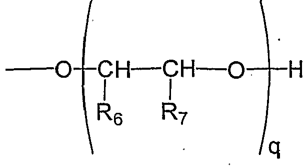 repräsentiert wird. Dabei können R₆ und R₇ unabhängig voneinander so gewählt werden daß sie H und Methyl, daß aber nicht beide Reste gleichzeitig Methyl darstellen können. q ist eine Zahl von 2 bis 1000, bevorzugt von 10 bis 200.

R₄ und R₅ können auch Polylolreste darstellen (z.B. Glyceryl-, Polyglyceryl-, Sorbityl-, Cellulosereste usw.).

Insbesondere dann, wenn die erfindungsgemäßen Zubereitungen sich durch leichte oder erleichterte Abwaschbarkeit von menschlicher Haut auszeichnen sollen, ist es von Vorteil, den Zubereitungen wasserlösliche und/oder mit Wasser quellbare Polymere einzuverleiben, insbesondere mit Alkylgruppen veretherte Cellulose- und/oder Stärkederivate. Besonders vorteilhaft sind β-Glucane, Xanthangummi, Dextrane, Hydroxymethylcellulose, Hydroxyethylcellulose und/oder Hydroxypropylcellulose, Methoxy-PEG-22/Dodecyl-Glycol-Copolymere, Poloxamere.

Vorteilhafte wasserlösliche und/oder mit Wasser quellbare Polymere können auch gewählt werden als mit einem oder mehreren n-Octenylsuccinatresten veresterter hydrophiler Stärke enthalten. Solche Stärkederivate zeichnen sich aus durch eine Struktur

### Stärke-Xₙ, wobei X den Rest

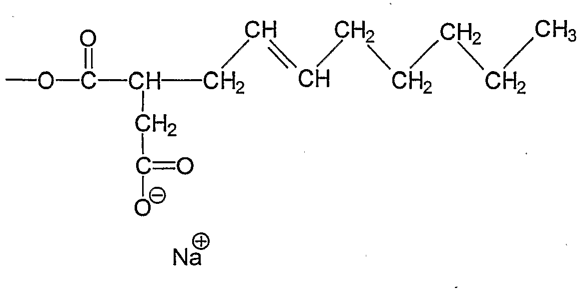

symbolisiert.

Erfindungsgemäß vorteilhaft zu verwendende Stärkederivate tragen offiziell noch keinen INCI-Namen (International Nomenclature Cosmetic Ingredient) dieser müßte die Bezeichnung "Starch Sodium Octenyl Succinate" tragen. Besonders vorteilhaft sind solche Produkte, welcher unter der Bezeichnung Amiogum®, insbesondere Amiogum®23 von der Gesellschaft Cerestar US verkauft werden.

Es wird bevorzugt, den Gehalt an wasserlöslichen und/oder mit Wasser quellbaren Polymeren im Konzentrationsbereich von 0,01 - 5,0 Gew.-%, besonders bevorzugt 0,1 - 1,0 Gew.-%, zu wählen.

Die Einarbeitung solcher wasserlöslichen und/oder mit Wasser quellbaren Polymere erfolgt bevorzugt dadurch, daß sie der Wasserphase einverleibt und mit der Wasserphase, besonders bevorzugt nach vollständiger Auflösung bzw. Quellung in die aufgeschmolzene Fettphase der Zubereitungen gegeben werden.

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UVA-Filtersubstanz und/oder mindestens eine UVB-Filtersubstanz und/oder mindestens ein anorganisches Pigment.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische und/oder dermatologische Zubereitungen zur Verfügung zu stellen, die die Haut vor dem gesamten Bereich der ultravioletten Strahlung schützen. Sie können auch als Sonnenschutzmittel dienen.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden.

Solche Pigmente können im Sinne der vorliegenden Erfindung vorteilhaft oberflächlich behandelt ("gecoatet") sein, wobei beispielsweise ein amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Erfindungsgemäß vorteilhaft sind z. B. Titandioxidpigmente, die mit Octylsilanol beschichtet sind. Geeignete Titandioxidpartikel sind unter der Handelsbezeichnung T805 bei der Firma Degussa erhältlich. Besonders vorteilhaft sind ferner mit Aluminiumstearat beschichtete TiO₂-Pigmente, z. B. die unter der Handelsbezeichnung MT 100 T bei der Firma TAYCA erhältlichen.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

### Auch andere UV-Filtersubstanzen, welche das Strukturmotiv

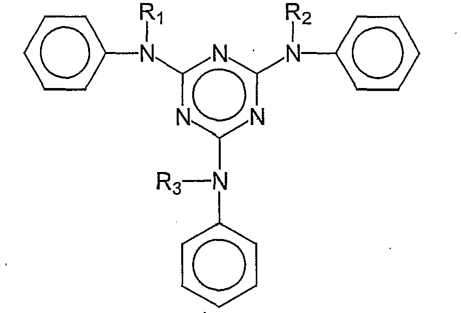

aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 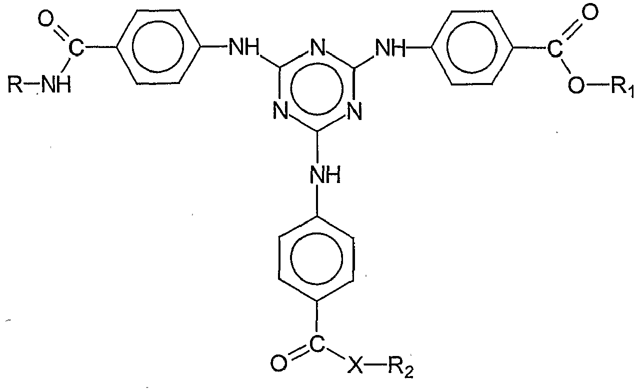 wiedergegeben wird, wobei
- R: einen verzweigten oder unverzweigten C₁-C₁₈-Alkylrest, einen C₅-C₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 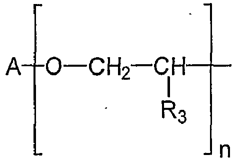 bedeutet, in welcher
A einen verzweigten oder unverzweigten C₁-C₈-Alkylrest, einen C₅-C₁₂-Cycloalkyl-oder Arylrest darstellt, gegebenenfalls substituiert mit einer oder mehreren C₁-C₄- Alkylgruppen,
R₃ ein Wasserstöffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 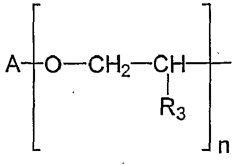 bedeutet, in welcher
A einen verzweigten oder unverzweigten C₁-C₁₈-Alkylrest, einen C₅₋C₁₂-Cycloalkyl-oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 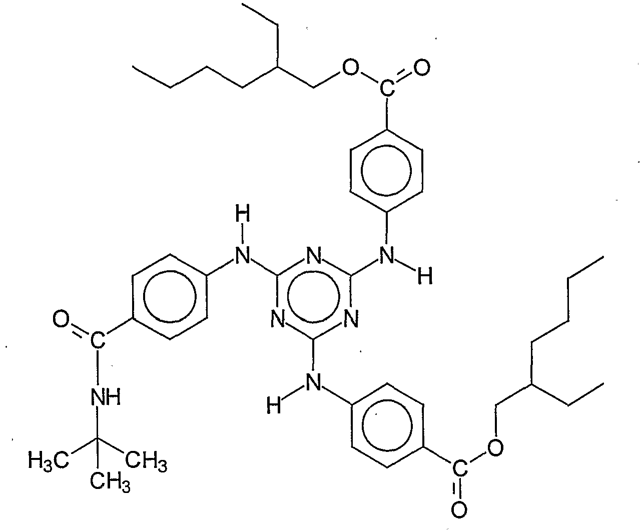 wiedergegeben wird, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 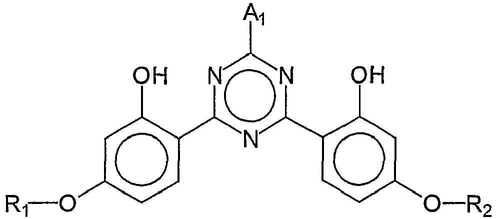 wiedergegeben wird, wobei R₁, R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxa-nyl]propyl]-phenol (CAS-Nr.: 155633-54-8) mit der INCI-Bezeichnung Drometrizole Trisiloxane.

Die UV-B- und/oder Breitband-Filter können öllöslich oder wasserlöslich sein. Vorteilhafte öllösliche UV-B- und/oder Breitband-Filtersubstanzen sind z. B.:
▪ 3-Benzylidencampher-Derivate, vorzugsweise 3-(4-Methylbenzyliden)campher, 3-Benzylidencampher;
▪ 4-Aminobenzoesäure-Derivate, vorzugsweise 4-(Dimethylamino)-benzoesäure(2-ethylhexyl)ester, 4-(Dimethylamino)benzoesäureamylester;
▪ 2,4,6-Trianilino-(p-carbo-2'-ethyl-1'-hexyloxy)-1,3,5-triazin;
▪ Ester der Benzalmalonsäure, vorzugsweise 4-Methoxybenzalmalonsäuredi(2-ethylhexyl)ester;
▪ Ester der Zimtsäure, vorzugsweise 4-Methoxyzimtsäure(2-ethylhexyl)ester, 4-Methoxyzimtsäureisopentylester;
▪ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
▪ 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.
▪ sowie an Polymere gebundene UV-Filter.

Vorteilhafte wasserlösliche UV-B- und/oder Breitband-Filtersübstanzen sind z. B.:
▪ Salze der 2-Phenylbenzimidazol-5-sulfonsäure, wie ihr Natrium-, Kalium- oder ihr Triethanolammonium-Salz, sowie die Sulfonsäure selbst;
▪ Sulfonsäure-Derivate des 3-Benzylidencamphers, wie z. B. 4-(2-Oxo-3-bornylidenme-thyl)benzolsulfonsäure, 2-Methyl-5-(2-oxo-3-bornylidenmethyl)sulfonsäure und deren Salze.
• Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
• Weitere vorteilhafte UV-A-Filtersubstanzen sind die Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate, welches beispielsweise unter der Handelsbezeichnung Neo Heliopan AP bei Haarmann & Reimer erhältlich ist.
• 1,4-di(2-oxo-10-Sulfo-3-bornylidenmethyl)-Benzol (auch: 3,3'-(1,4-Phenylendimethy-lene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxa-nyl]propyl]-phenol (CAS-Nr.: 155633-54-8) mit der INCI-Bezeichnung Drometrizole Trisiloxane, welches unter der Handelsbezeichnung Mexoryl® XL bei der Fa. Chimex erhältlich ist.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A- und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.
Ferner kann es gegebenenfalls von Vorteil sein, Filmbildner in die erfindungsgemäßen kosmetischen oder dermatologischen Zubereitungen einzuarbeiten, beispielsweise um die Wasserfestigkeit der Zubereitungen zu verbessern oder die UV-Schutzleistung zu erhöhen (UV-A- und/oder UV-B-Boosting). Geeignet sind sowohl wasserlösliche bzw. dispergierbare als auch fettlösliche Filmbildner, jeweils einzeln oder in Kombination miteinander.

Vorteilhafte wasserlöslich bzw. dispergierbare Filmbildner sind z. B. Polyurethane (z. B. die Avalure® -Typen von Goodrich), Dimethicone Copolyol Polyacrylate (Silsoft Surface® von der Witco Organo Silicones Group), PVP/VA (VA = Vinylacetat) Copolymer (Luviscol VA 64 Powder der BASF) etc.

Vorteilhafte fettlösliche Filmbildner sind z. B., die Filmbildner aus der Gruppe der Polymere auf Basis von Polyvinylpyrrolidon (PVP)

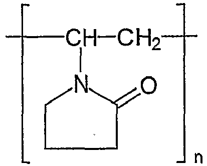

Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Erfindungsgemäße Emulsionen können auch Puderstoffe enthalten. Als Puderstoffe werden beispielsweise Wismuthoxichlorid, titanisierter Glimmer, Siliciumdioxid (fumed silica), spherische Siliciumdioxid-Perlen, Polymethylmethacyrlat-Perlen, micronisiertes Teflon, Bornitrid, Acrylatpolymere, Aluminumsilicat, Aluminum-Stärke-Octenylsuccinat, Bentonit, Calciumsilicat, Cellulose, Kreide, Maisstärke, Glycerylstärke, Hectorit, hydrisiertes Silica, Kaolin, Magnesiumhydroxide, Magnesiumoxid, Magnesiumsilicate, Magnesiumtrisilicat, Maltodextrin, Montmorillonit, microcristalline Cellulose, Reisstärke, Silica, Talk, Mica, Titaniumdioxid, Zinklaurate, Zinkmyristat, Zinkneodecanoat, Zinkrosinat, Zinkstearat, Polyehtylen, Aluminiumoxid, Attapulgit, Calciumcarbonat, Calciumsilicat, Dextran, Kaolin, Nylon, Silicasilylat, Seidenpuder, Serecit, Zinnoxid, Titaniumhydroxid, Trimagnesiumphosphat, Wallnußschalenpuder oder beliebige Mischungen eingesetzt werden.

In die erfindungsgemäßen Zubereitungen können vorteilhaft zusätzlich die üblichen Bestandteile kosmetischer Stifte eingearbeitet werden, z.B. die üblichen Hilfs- und Zusatzstoffe wie Parfümöle, Konservierungsmittel, Farbpigmente, Lichtschutzmittel, Stabilisatoren.

Es ist dem Fachmanne natürlich bekannt, daß anspruchsvolle kosmetische Zubereitungen zumeist nicht ohne die üblichen Hilfs- und Zusatzstoffe denkbar sind. Darunter zählen beispielsweise Konsistenzgeber, Füllstoffe, Parfum, zusätzliche Wirkstoffe wie Vitamine oder Proteine, Insektenrepellentien, Alkohol, Wasser, Salze, antimikrobiell, proteolytisch oder keratolytisch wirksame Substanzen usw.

### Beispiele

Alle Mengenangaben, Prozentangaben oder Teile beziehen sich, soweit nicht anders angegeben, auf das Gewicht, insbesondere auf das Gesamtgewicht der Zubereitungen oder der jeweiligen Mischungen.

Die nachfolgenden Beispiele sollen die Erfindung erläutern

**Beispiel 1**

| Prophylaktisch wirkender bzw Antifaltenstift mit hohem Wasser- und Moisturizeranteil | |
|---|---|
| | Gew.-% |
| PEG-45/ Dodecyl Glycol Copolymer | 1,60 |
| Polyglyceryl-3-diisostearat | 1,60 |
| Cetearyl Isononanoat | 14,70 |
| Ubiquinon (Cyclodextrin verkapselt) | 0.20 |
| Ubiquinon | 0.20 |
| Biotin | 0,20 |
| Abdeckende Pigmente | 1,50 |
| C₂₀₋₄₀-Alkylstearat | 9,00 |
| Glycerin | 10,00 |
| Parfüm | 0,30 |
| Konservierung | 0,58 |
| Wasser | ad 100,00 |

**Beispiel 2**

| Prophylaktisch wirkender bzw Antifaltenstift mit hohem Wasser- und Moisturizeranteil | |
|---|---|
| | Gew.-% |
| PEG-45/ Dodecyl Glycol Copolymer | 1,60 |
| Polyglyceryl-3-diisostearat | 1,60 |
| Caprylsäure/Caprinsäure Triglycerid | 14,70 |
| Ubiquinon (Cyclodextrin verkapselt) | 0.20 |
| Ubiquinon | 0.20 |
| Biotin | 0,20 |
| Abdeckende Pigmente | 1,50 |
| C₂₀₋₄₀-Alkylstearat | 9,00 |
| Glycerin | 10,00 |
| Parfüm | 0,30 |
| Konservierung | 0,58 |
| Wasser | ad 100,00 |

**Beispiel 3**

| Prophylaktisch gegen Falten wirkender bzw. Antifaltenstift mit hohem Wasser- und Moisturizeranteil | |
|---|---|
| | Gew.-% |
| PEG-45/ Dodecyl Glycol Copolymer | 1,60 |
| Polyglyceryl-3-diisostearat | 1,60 |
| Caprylsäure/Caprinsäure Triglycerid | 5,70 |
| Ubiquinon (Cyclodextrin verkapselt) | 0.20 |
| Ubiquinon | 0.20 |
| Biotin | 0,20 |
| Abdeckende Pigmente | 10,00 |
| C₂₀₋₄₀-Alkylstearat | 9,00 |
| Glycerin | 10,00 |
| Octyldodecanol | 5,70 |
| Dicaprylylether | 5,70 |
| Shea Butter | 1,00 |
| Jojobaöl | 1,00 |
| Parfüm | 0,30 |
| Konservierung | 0,58 |
| Wasser | ad 100,00 |

**Beispiel 4**

| Prophylaktisch wirkender bzw Antifaltenstift mit hohem Wasser- und Moisturizeranteil | |
|---|---|
| | Gew.-% |
| PEG-45/ Dodecyl Glycol Copolymer | 1,60 |
| Polyglyceryl-3-diisostearat | 1,60 |
| Caprylsäure/Caprinsäure Triglycerid | 5,70 |
| Ubiquinon (Cyclodextrin verkapselt) | 0.20 |
| Ubiquinon | 0.20 |
| Biotin | 0,20 |
| Abdeckende Pigmente | 10,00 |
| C₂₀₋₄₀-Alkylstearat | 9,00 |
| Glycerin | 10,00 |
| Octyldodecanol | 5,70 |
| Dicaprylylether | 5,70 |
| Shea Butter | 1,00 |
| Jojobaöl | 1,00 |
| Parfüm | 0,30 |
| Konservierung | 0,58 |
| Wasser | ad 100,00 |

**Beispiel 5**

| Prophylaktisch wirkender bzw Antifaltenstift mit hohem Wasser- und Moisturizeranteil | |
|---|---|
| | Gew.-% |
| PEG-45/ Dodecyl Glycol Copolymer | 2,00 |
| Polyglyceryl-3-diisostearat | 2,40 |
| PEG-8 Bienenwachs | 1,00 |
| Octyldodecanol | 10,75 |
| Squalen | 1,00 |
| Ubiquinon (Cyclodextrin verkapselt) | 0.20 |
| Ubiquinon | 0.20 |
| Biotin | 0,20 |
| Abdeckende Pigmente | 10,00 |
| C₂₀₋₄₀-Alkylstearat | 8,00 |
| Glycerin | 10,00 |
| Jojobaöl | 1,00 |
| Parfüm | 0,30 |
| Konservierung | 0,58 |
| Wasser | ad 100,00 |

**Beispiel 6**

| Prophylaktisch wirkender bzw Antifaltenstift mit hohem Wasser- und Moisturizeranteil | |
|---|---|
| | Gew.-% |
| PEG-45/ Dodecyl Glycol Copolymer | 1,60 |
| Polyglyceryl-3-diisostearat | 1,60 |
| Caprylsäure/Caprinsäure Triglycerid | 14,70 |
| C₂₀₋₄₀-Alkylstearat | 9,00 |
| Ascorbinsäure | 3,00 |
| Glycerin | 10,00 |
| Parfüm | 0,30 |
| Konservierung | 0,58 |
| Wasser | ad 100,00 |

**Beispiel 7**

| Prophylaktisch wirkender bzw Antifaltenstift mit hohem Wasser- und Moisturizeranteil | |
|---|---|
| | Gew.-% |
| PEG-45/ Dodecyl Glycol Copolymer | 1,80 |
| Polyglyceryl-3-diisostearat | 2,70 |
| PEG-8 Bienenwachs | 1,10 |
| Octyldodecanol | 5,90 |
| Squalan | 1,00 |
| Ricinusöl | 3,00 |
| Jojobaöl | 1,00 |
| Avocadoöl | 1,50 |
| Macadamiaöl | 1,50 |
| Hydrogeniertes Polydecen | 4,24 |
| Tocopherolacetat | 1,10 |
| Cetylpalmitat | 1,10 |
| Ubiquinon (Cyclodextrin verkapselt) | 0.20 |
| Liponsäure | 0.20 |
| Biotin | 0,20 |
| Abdeckende Pigmente | 11,50 |
| Bis- Ethylhexyloxyphenol Methoxyphenyl Triazin | 1,00 |
| Ethylhexyltriazon | 1,00 |
| Glycerin | 7,50 |
| Parfüm | 0,30 |
| Konservierung | 0,58 |
| Wasser | ad 100,00 |

**Beispiel 8**

| Prophylaktisch wirkender bzw Antifaltenstift mit hohem Wasser- und Moisturizeranteil | |
|---|---|
| | Gew.-% |
| PEG-45/Dodecyl Glycol Copolymer | 1,80 |
| Polyglyceryl-3-diisostearat | 2,70 |
| PEG-8 Bienenwachs | 1,10 |
| Octyldodecanol | 5,90 |
| Squalan | 1,00 |
| Ricinusöl | 3,00 |
| Jojobaöl | 1,00 |
| Avocadoöl | 1,50 |
| Macadamiaöl | 1,50 |
| Hydrogeniertes Polydecen | 4,24 |
| Tocopherolacetat | 1,10 |
| Cetylpalmitat | 1,10 |
| Ubiquinon (Cyclodextrin verkapselt) | 0.20 |
| Carnithin | 0.20 |
| Biotin | 0,20 |
| Abdeckende Pigmente | 11,50 |
| Bis- Ethylhexyloxyphenol Methoxyphenyl Triazin | 1,00 |
| Ethylhexyltriazon | 1,00 |
| Glycerin | 7,50 |
| Parfüm | 0,30 |
| Konservierung | 0,58 |
| Wasser | ad 100,00 |

**Beispiel 9**

| Prophylaktisch wirkender bzw Antifaltenstift mit hohem Wasser- und Moisturizeranteil | |
|---|---|
| | Gew.-% |
| PEG-45/ Dodecyl Glycol Copolymer | 1,60 |
| Polyglyceryl-3-diisostearat | 1,60 |
| Caprylsäure/Caprinsäure Triglycerid | 14,70 |
| C₂₀₋₄₀-Alkylstearat | 9,00 |
| Tocopherol | 3,00 |
| Glycerin | 10,00 |
| Parfüm | 0,30 |
| Konservierung | 0,58 |
| Wasser | ad 100,00 |

**Beispiel 10**

| Prophylaktisch wirkender bzw Antifaltenstift mit hohem Wasser- und Moisturizeranteil | |
|---|---|
| | Gew.-% |
| PEG-45/ Dodecyl Glycol Copolymer | 1,60 |
| Polyglyceryl-3-diisostearat | 1,60 |
| Caprylsäure/Caprinsäure Triglycerid | 14,70 |
| C₁₈₋₃₆-Säuretriglycerid | 1,00 |
| C₂₀₋₄₀-Alkylstearat | 9,00 |
| α-Glucosylrutin, Creatin (1:1) | 0,50 |
| Glycerin, Fucogel (9:1) | 10,00 |
| Konservierung | 0,58 |
| Wasser | ad 100,00 |

**Beispiel 11**

| Prophylaktisch wirkender bzw Antifaltenstift mit hohem Wasser- und Moisturizeranteil | |
|---|---|
| | Gew.-% |
| PEG-45/ Dodecyl Glycol Copolymer | 1,60 |
| Sorbitansiostearat | 1,60 |
| Caprylsäure/Caprinsäure Triglycerid | 14,70 |
| C₁₈₋₃₆-Säuretrigycerid | 1,00 |
| C₂₀₋₄₀-Alkylstearat | 9,00 |
| Glycerin, Glycin (9:1) | 10,00 |
| Konservierung | 0,58 |
| Wasser | ad 100,00 |

**Beispiel 12**

| Prophylaktisch gegen falten wirkender bzw. Antifaltenstift mit hohem Wasser- und Moisturizeranteil | |
|---|---|
| | Gew.-% |
| PEG-45/ Dodecyl Glycol Copolymer | 1,80 |
| PEG-30 Dipolyhydroxystearat | 2,70 |
| PEG-8 Bienenwachs | 1,10 |
| Octyldodecanol | 5,90 |
| Squalan | 1,00 |
| Ricinusöl | 3,00 |
| Jojobaöl | 1,00 |
| Avocadoöl | 1,50 |
| Macadamiaöl | 1,50 |
| Hydrogeniertes Polydecen | 4,24 |
| Tocopherolacetat | 1,10 |
| Cetylpalmitat | 1,10 |
| Ubiquinon (Cyclodextrin verkapselt) | 0.20 |
| Methylpalmitat | 1,00 |
| Biotin | 0,20 |
| Abdeckende Pigmente | 11,50 |
| Bis- Ethylhexyloxyphenol Methoxyphenyl Triazin | 1,00 |
| Ethylhexyltriazon | 1,00 |
| Glycerin, Milchsäure (9:1) | 7,50 |
| Parfüm | 0,30 |
| Konservierung | 0,58 |
| Wasser | ad 100,00 |

**Beispiel 13**

| Prophylaktisch wirrkender bzw Antifaltenstift mit hohem Wasser- und Moisturizeranteil | |
|---|---|
| | Gew.-% |
| PEG-45/ Dodecyl Glycol Copolymer | 1,60 |
| Polyglyceryl-3 methylglucose distearat | 1,60 |
| Cetearyl Isononanoat | 14,70 |
| Ubiquinon (Cyclodextrin verkapselt) | 0.20 |
| Ubiquinon | 0.20 |
| Tocopherol | 0,20 |
| Abdeckende Pigmente | 1,50 |
| C₂₀₋₄₀-Alkylstearat | 9,00 |
| Glycerin, Butylenglycol (9:1) | 10,00 |
| Parfüm | 0,30 |
| Konservierung | 0,58 |
| Wasser | ad 100,00 |

**Beispiel 14**

| Prophylaktisch wirrkender bzw Antifaltenstift mit hohem Wasser- und Moisturizeranteil | |
|---|---|
| | Gew.-% |
| PEG-45/ Dodecyl Glycol Copolymer | 1,60 |
| Propylenglycolisostearat | 1,60 |
| Cetearyl Isononanoat | 14,70 |
| Ubiquinon (Cyclodextrin verkapselt) | 0.20 |
| Ubiquinon | 0.20 |
| Tocopherol | 0,20 |
| Abdeckende Pigmente | 1,50 |
| C₂₀₋₄₀-Alkylstearat | 9,00 |
| Glycerin, NaCl | 10,00 |
| Parfüm | 0,30 |
| Konservierung | 0,58 |
| Wasser | ad 100,00 |

**Beispiel 15**

| Prophylaktisch wirkender bzw Antifaltenstift mit hohem Wasser- und Moisturizeranteil | |
|---|---|
| | Gew.-% |
| PEG-45/ Dodecyl Glycol Copolymer | 2,00 |
| Glycerinisostearat | 2,40 |
| PEG-8 Bienenwachs | 1,00 |
| Octyldodecanol | 10,75 |
| Squalen | 1,00 |
| Ubiquinon (Cyclodextrin verkapselt) | 0.20 |
| Ubiquinon | 0.20 |
| Biotin | 0,20 |
| Abdeckende Pigmente | 10,00 |
| C₂₀₋₄₀-Alkylstearat | 8,00 |
| Glycerin | 10,00 |
| Jojobaöl | 1,00 |
| Parfüm | 0,30 |
| Konservierung | 0,58 |
| Wasser | ad 100,00 |

**Beispiel 16**

| Prophylaktisch wirrkender bzw Antifaltenstift mit hohem Wasser- und Moisturizeranteil | |
|---|---|
| | Gew.-% |
| PEG-45/ Dodecyl Glycol Copolymer | 1,80 |
| Polyglyceryl-3-diisostearat | 2,70 |
| PEG-8 Bienenwachs | 1,10 |
| Octyldodecanol | 5,90 |
| Squalan | 1,00 |
| Ricinusöl | 3,00 |
| Jojobaöl | 1,00 |
| Avocadoöl | 1,50 |
| Macadamiaöl | 1,50 |
| Hydrogeniertes Polydecen | 4,24 |
| Tocopherolacetat | 1,10 |
| Cetylpalmitat | 1,10 |
| Ubiquinon (Cyclodextrin verkapselt) | 0.20 |
| Carnithin | 0.20 |
| Biotin | 0,20 |
| Abdeckende Pigmente | 11,50 |
| Butyl Methoxydibenzoylmethan | 1,00 |
| 4-Methylbenzylidene Kampfer | 1,00 |
| Glycerin, Natriumpyrolidoncarbonsäuresalz (9:1) | 7,50 |
| Parfüm | 0,30 |
| Konservierung | 0,58 |
| Wasser | ad 100,00 |

**Beispiel 17**

| Prophylaktisch wirrkender bzw Antifaltenstift mit hohem Wasser- und Moisturizeranteil | |
|---|---|
| | Gew.-% |
| PEG-45/ Dodecyl Glycol Copolymer | 1,80 |
| Polyglyceryl-3-diisostearat | 2,70 |
| PEG-8 Bienenwachs | 1,10 |
| Octyldodecanol | 5,90 |
| Squalan | 1,00 |
| Ricinusöl | 3,00 |
| Jojobaöl | 1,00 |
| Avocadoöl | 1,50 |
| Macadamiaöl | 1,50 |
| Hydrogeniertes Polydecen | 4,24 |
| Tocopherolacetat | 1,10 |
| Cetylpalmitat | 1,10 |
| Ubiquinon (Cyclodextrin verkapselt) | 0.20 |
| Biotin | 0.20 |
| Abdeckende Pigmente | 11,50 |
| Octocrylen | 1,00 |
| 4-Methylbenzylidene Kampfer | 1,00 |
| Glycerin, Hyaluronsäure-Salz (9:1) | 7,50 |
| Parfüm | 0,30 |
| Konservierung | 0,58 |
| Wasser | ad 100,00 |

**Beispiel 18**

| prophylaktisch wirkender bzw Antifaltenstift mit hohem Wasser- und Moisturizeranteil | |
|---|---|
| | Gew.-% |
| PEG-45/ Dodecyl Glycol Copolymer | 1,60 |
| Polyglyceryl-3-diisostearat | 1,60 |
| Caprylsäure/Caprinsäure Triglycerid | 14,70 |
| C₂₀₋₄₀-Alkylstearat | 9,00 |
| Tocopherol | 3,00 |
| Retinol | 0.20 |
| Phenylen-1,4-bis-(2-benzimidazyl)-3,3'-5,5'-tetrasulfonsäure-bis-natriumsalz | 3,00 |
| Dioctylbutamidotriazon | 3,00 |
| Glycerin, Polyethylenglycol (9:1). | 10,00 |
| Parfüm | 0,30 |
| Konservierung | 0,58 |
| Wasser | ad 100,00 |

**Beispiel 19**

| Prophylaktisch wirkender bzw Antifaltenstift mit hohem Wasser- und Moisturizeranteil | |
|---|---|
| | Gew.-% |
| PEG-45/ Dodecyl Glycol Copolymer | 1,60 |
| Polyglyceryl-3-diisostearat | 1,60 |
| Caprylsäure/Caprinsäure Triglycerid | 14,70 |
| C₁₈₋₃₆-Säuretriglycerid | 1,00 |
| C₂₀₋₄₀-Alkylstearat | 9,00 |
| α-Glucosylrutin, Creatin (1:1) | 0,50 |
| Ethylhexyltriazon | 2,00 |
| Phenylbenzimidazol Sulfionsäure Salz | 1.00 |
| Glycerin, Fucogel (9:1) | 10,00 |
| Konservierung | 0,58 |
| Wasser | ad 100,00 |

**Beispiel 20**

| Prophylaktisch wirkender bzw Antifaltenstift mit hohem Wasser- und Moisturizeranteil | |
|---|---|
| | Gew.-% |
| PEG-45/ Dodecyl Glycol Copolymer | 2,00 |
| Glycerinisostearat | 2,40 |
| PEG-8 Bienenwachs | 1,00 |
| Ethylhexyl Salicylat | 2.00 |
| Octyldodecanol | 10,75 |
| Squalen | 1,00 |
| Ubiquinon (Cyclodextrin verkapselt) | 0.20 |
| Ubiquinon | 0.20 |
| Biotin | 0,20 |
| Abdeckende Pigmente | 10,00 |
| C₂₀₋₄₀-Alkylstearat | 8,00 |
| Glycerin, Harnstoff (9:1) | 10,00 |
| Jojobaöl | 1,00 |
| Parfüm | 0,30 |
| Konservierung | 0,58 |
| Wasser | ad 100,00 |

**Beispiel 21**

| Anti-Akne-Stift | |
|---|---|
| | Gew.-% |
| Cetearyl Isononanoat | 15.00 |
| PEG-45/ Dodecyl Glycol Copolymer | 1,60 |
| Polyglyceryl-3-diisostearat | 1.60 |
| Milchsäure | 3.00 |
| Salicylsäure | 0.50 |
| Abdeckende Pigmentmischung | 10.00 |
| Natronlauge (10% ig) | 6.70 |
| C₂₀₋₄₀-Alkylstearat | 8.00 |
| Glycerin | 10.00 |
| Konservierung | 0.50 |
| Wasser | ad 100,000 |

**Beispiel 22**

| Anti-Akne-Stift | |
|---|---|
| | Gew.-% |
| Cetearyl Isononanoat | 15.00 |
| PEG-45/ Dodecyl Glycol Copolymer | 1,60 |
| Polyglyceryl-3-diisostearat | 1.60 |
| Milchsäure | 3.00 |
| Salicylsäure | 0.50 |
| Zinksulfat | 0.70 |
| Abdeckende Pigmentmischung | 10.00 |
| Natronlauge (10% ig) | 6.70 |
| C₂₀₋₄₀-Alkylstearat | 8.00 |
| Glycerin | 10.00 |
| Konservierung | 0.50 |
| Wasser | ad 100,00 |

**Beispiel 23**

| Anti-Akne-Stift | |
|---|---|
| | Gew.-% |
| Cetearyl Isononanoat | 15.00 |
| PEG-45/ Dodecyl Glycol Copolymer | 1,60 |
| Polyglyceryl-3-diisostearat | 1.60 |
| Milchsäure | 3.00 |
| Salicylsäure | 0.50 |
| Polyaminopropyl Biguanid | 1.00 |
| Abdeckende Pigmentmischung | 10.00 |
| Natronlauge (10% ig) | 6.70 |
| C₂₀₋₄₀-Alkylstearat | 8.00 |
| Glycerin | 10.00 |
| Konservierung | 0.50 |
| Wasser | ad 100,000 |

**Beispiel 24**

| Anti-Akne-Stift | |
|---|---|
| | Gew.-% |
| Cetearyl Isononanoat | 15.00 |
| PEG-45/ Dodecyl Glycol Copolymer | 1,60 |
| Polyglyceryl-3-diisostearat | 1.60 |
| Alumininumchlorhydrat | 4.00 |
| Salicylsäure | 0.50 |
| Abdeckende Pigmentmischung | 10.00 |
| Natronlauge (10% ig) | 6.70 |
| C₂₀₋₄₀-Alkylstearat | 8.00 |
| Glycerin | 10.00 |
| Konservierung | 0.50 |
| Wasser | ad 100,000 |

**Beispiel 25**

| Antiaknestift | |
|---|---|
| | Gew.-% |
| Polyglyceryl-3 Diisostearat | 1,60 |
| PEG-45/ Dodecyl Glycol Copolymer | 1,60 |
| Glycerylcaprate | 0,50 |
| Polyglyceryl-3 caprylat | 0.50 |
| Caprylic/Capric Triglyceride | 4,00 |
| Octyldodecanol | 4,00 |
| Dicaprylylether | 4,00 |
| Cetearylbehenat | 6,00 |
| Milchsäure | 2,00 |
| Octacosanylstearat | 8,00 |
| Glycerin | 10,000 |
| Wasser | ad 100,000 |

**Beispiel 26**

| Antiaknestift | |
|---|---|
| | Gew.-% |
| Polyglyceryl-3 Diisostearat | 1,60 |
| PEG-45/ Dodecyl Glycol Copolymer | 1,60 |
| Glycerylcaprat | 3.00 |
| Caprylic/Capric Triglyceride | 4,00 |
| Octyldodecanol | 4,00 |
| Dicaprylylcarbonat | 4,00 |
| Cetearylbehenat | 6,00 |
| Salicylsäure | 1,00 |
| Octacosanylstearat | 6,00 |
| Glycerin | 10,00 |
| Wasser | ad 100,00 |

**Beispiel 27**

| Anti-Akne-Stift | |
|---|---|
| | Gew.-% |
| Cetearyl Isononanoat | 15.00 |
| PEG-45/ Dodecyl Glycol Copolymer | 1,60 |
| Polyglyceryl-3-diisostearat. | 1.60 |
| Milchsäure | 2.00 |
| Salicylsäure | 0.50 |
| Citronensäure | 1.00 |
| Abdeckende Pigmentmischung | 10.00 |
| Natronlauge (10% ig) | 6.70 |
| C₂₀₋₄₀-Alkylstearat | 8.00 |
| Glycerin | 10.00 |
| Konservierung | 0.50 |
| Wasser | ad 100,000 |

**Beispiel 28**

| Anti-Akne-Stift | |
|---|---|
| | Gew.-% |
| Cetearyl Isononanoat | 15.00 |
| PEG-45/ Dodecyl Glycol Copolymer | 1,60 |
| Polyglyceryl-3-diisostearat | 1.60 |
| Milchsäure | 3.00 |
| Salicylsäure | 0.50 |
| 2-Ethylhexylglycerinether | 1.00 |
| Abdeckende Pigmentmischung | 10.00 |
| Natronlauge (10% ig) | 6.70 |
| C₂₀₋₄₀-Alkylstearat | 8.00 |
| Glycerin | 10.00 |
| Konservierung | 0.50 |
| Wasser | ad 100,000 |

**Beispiel 29**

| Anti-Akne-Stift | |
|---|---|
| | Gew.-% |
| Cetearyl Isononanoat | 15.00 |
| PEG-45/ Dodecyl Glycol Copolymer | 1,60 |
| Polyglyceryl-3-diisostearat | 1.60 |
| Milchsäure | 2.00 |
| Salicylsäure | 0:50 |
| Citronensäure | 1.00 |
| Abdeckende Pigmentmischung | 10.00 |
| Natronlauge (10% ig) | 6.70 |
| C₂₀₋₄₀-Alkylstearat | 8.00 |
| Glycerin | 10.00 |
| Konservierung | 0.50 |
| Wasser | ad 100,00 |

**Beispiel 30**

| Anti-Akne-Stift | |
|---|---|
| | Gew.-% |
| Cetearyl Isononanoat | 15.00 |
| PEG-45/ Dodecyl Glycol Copolymer | 1,60 |
| Sorbitanisostearat | 1.60 |
| Milchsäure | 2.00 |
| Salicylsäure | 0.50 |
| Citronensäure | 1.00 |
| Abdeckende Pigmentmischung | 10.00 |
| Natronlauge (10% ig) | 6.70 |
| C₂₀₋₄₀-Alkylstearat | 8.00 |
| Glycerin | 10.00 |
| Konservierung | 0.50 |
| Wasser | ad 100,00 |

**Beispiel 31**

| Anti-Akne-Stift | |
|---|---|
| | Gew.-% |
| Cetearyl Isononanoat | 15.00 |
| PEG-45/ Dodecyl Glycol Copolymer | 1,60 |
| PEG-30 Dipolyhydroxystearat | 1.60 |
| Milchsäure | 3.00 |
| Salicylsäure | 0.50 |
| Abdeckende Pigmentmischung | 10.00 |
| Natronlauge (10% ig) | 6.70 |
| C₂₀₋₄₀-Alkylstearat | 8.00 |
| Methylpalmitat | 1.00 |
| Glycerin | 10.00 |
| Konservierung | 0.50 |
| Wasser | ad 100,000 |

**Beispiel 32**

| Anti-Akne-Stift | |
|---|---|
| | Gew.-% |
| Cetearyl Isononanoat | 15.00 |
| PEG-45/ Dodecyl Glycol Copolymer | 1,60 |
| Polyglyceryl-2 Dipolyhydroxystearat | 1.60 |
| Milchsäure | 3.00 |
| Salicylsäure | 0.50 |
| Abdeckende Pigmentmischung | 10.00 |
| Natronlauge (10% ig) | 6.70 |
| C₂₀₋₄₀-Alkylstearat | 8.00 |
| Glycerin | 10.00 |
| Konservierung | 0.50 |
| Wasser | ad 100,000 |

**Beispiel 33**

| Anti-Akne-Stift | |
|---|---|
| | Gew.-% |
| Cetearyl Isononanoat | 15.00 |
| PEG-45/ Dodecyl Glycol Copolymer | 1,60 |
| PEG-40 Sorbitanperisostearat | 1.60 |
| Milchsäure | 3.00 |
| Salicylsäure | 0.50 |
| Abdeckende Pigmentmischung | 10.00 |
| Natronlauge (10% ig) | 6.70 |
| C₂₀₋₄₀-Alkylstearat | 8.00 |
| Glycerin, Natriumpyroldioncarbonsäuresalz 10:1 | 10.00 |
| Konservierung | 0.50 |
| Wasser | ad 100,000 |

**Beispiel 34**

| Anti-Akne-Stift | |
|---|---|
| | Gew.-% |
| Cetearyl Isononanoat | 15.00 |
| PEG-45/ Dodecyl Glycol Copolymer | 1,60 |
| Cetylalkohol | 1.60 |
| Milchsäure | 3.00 |
| Salicylsäure | 0.50 |
| Abdeckende Pigmentmischung | 10.00 |
| Natronlauge (10% ig) | 6.70 |
| C₂₀₋₄₀-Alkylstearat | 8.00 |
| Glycerin, Hyaluronsäure 10:1 | 10.00 |
| Konservierung | 0.50 |
| Wasser | ad 100,000 |

**Beispiel 35**

| Anti-Akne-Stift | |
|---|---|
| | Gew.-% |
| Cetearyl Isononanoat | 15.00 |
| PEG-45/ Dodecyl Glycol Copolymer | 1,60 |
| Propylenglycolisostearat | 1.60 |
| Milchsäure | 3.00 |
| Salicylsäure | 0.50 |
| Abdeckende Pigmentmischung | 10.00 |
| Natronlauge (10% ig) | 6.70 |
| C₂₀₋₄₀-Alkylstearat | 8.00 |
| Glycerin, NMF 10:1 | 10.00 |
| Konservierung | 0.50 |
| Wasser | ad 100,000 |

**Beispiel 36**

| Anti-Akne-Stift | |
|---|---|
| | Gew.-% |
| Cetearyl Isononanoat | 15.00 |
| PEG-45/ Dodecyl Glycol Copolymer | 1,60 |
| Steareth-2 | 1.60 |
| Milchsäure | 3.00 |
| Salicylsäure | 0.50 |
| Abdeckende Pigmentmischung | 10.00 |
| Natronlauge (10% ig) | 6.70 |
| C₂₀₋₄₀-Alkylstearat | 8.00 |
| PEG-8 Bienenwachs | 1.00 |
| Behenylbehenat | 0,50 |
| Glycerin, Harnstoff 10:1 | 10.00 |
| Konservierung | 0.50 |
| Wasser | ad 100,000 |

**Beispiel 37**

| Anti-Akne-Stift | |
|---|---|
| | Gew.-% |
| Cetearyl Isononanoat | 15.00 |
| PEG-45/ Dodecyl Glycol Copolymer | 1,60 |
| Polyglyceryl-3 methylglucose distearat | 1.60 |
| Milchsäure | 3.00 |
| Salicylsäure | 0.50 |
| Abdeckende Pigmentmischung | 10.00 |
| Natronlauge (10% ig) | 6.70 |
| C₂₀₋₄₀-Alkylstearat | 8.00 |
| C₁₈₋₃₆-Acid Triglycerid | 1,00 |
| Glycerin, Fucogel 10:1 | 10.00 |
| Konservierung | 0.50 |
| Wasser | ad 100,000 |

**Beispiel 38**

| Anti-Akne-Stift als Concealer | |
|---|---|
| | Gew.-% |
| Cetearyl Isononanoat | 15.00 |
| PEG-45/ Dodecyl Glycol Copolymer | 1,60 |
| Polyglyceryl-3-diisostearat | 1.60 |
| Milchsäure | 3.00 |
| 2-Butyloktansäure | 0.50 |
| Abdeckende Pigmentmischung | 10.00 |
| Natronlauge (10% ig) | 6.70 |
| C₂₀₋₄₀-Alkylstearat | 8.00 |
| Glycerin | 10.00 |
| Konservierung | 0.50 |
| Wasser | ad 100,000 |

**Beispiel 39**

| Deko-Lippenstift | |
|---|---|
| | Gew.-% |
| PEG-45/ Dodecyl Glycol Copolymer | 1,60 |
| Polyglyceryl-3-diisostearat | 2,40 |
| Simethicon | 0,50 |
| Octyldodecanol | 4,00 |
| Polydecen | 4,80 |
| Ricinusoel | 5,00 |
| Ethylhexyl Cocoat | 4,80 |
| Buxus Chinensis | 1,00 |
| Squalan | 1,00 |
| PEG-8 Bienenwachs | 1,00 |
| Butyl Methoxydibenzoylmethan | 0,80 |
| 4-Methylbenzyliden Kampfer | 1,60 |
| C₂₀₋₄₀-Alkylstearat | 8,00 |
| Glycerin | 10,00 |
| Farbabrieb | 11,60 |
| Parfüm | 0,30 |
| Wirkstoffe | 1,10 |
| Konservierung | 0,58 |
| Wasser | ad 100,00 |

**Beispiel 40**

| Foundationstift | |
|---|---|
| | Gew.-% |
| PEG-45/ Dodecyl Glycol Copolymer | 1,60 |
| Polyglyceryl-3-diisostearat | 1,60 |
| Simethicon | 0,50 |
| Octyldodecanol | 7,00 |
| Polydecen | 7,00 |
| Ricinusoel | 7,00 |
| Butyl Methoxydibenzoylmethan | 0,80 |
| 4-Methylbenzylidene Kampfer | 1,60 |
| Candelilla Cera | 4,50 |
| C₂₀₋₄₀-Alkylstearat | 4,50 |
| Glycerin | 10,00 |
| Farbabrieb | 11,60 |
| Parfüm | 0,30 |
| Wirkstoffe | 1,10 |
| Konservierung | 0,58 |
| Wasser | ad 100,00 |

**Beispiel 41**

| Deko-Lippenstift | |
|---|---|
| | Gew.-% |
| PEG-45/ Dodecyl Glycol Copolymer | 1,600 |
| Polyglyceryl-3-diisostearat | 2,40 |
| Simethicon | 0,50 |
| Octyldodecanol | 3,20 |
| Polydecen | 4,80 |
| Ricinusoel | 5,00 |
| Dicaprylyl Carbonat | 4,80 |
| Buxus Chinensis | 1,00 |
| Squalan | 1,00 |
| Hydroxyethylcellulose | 0,30 |
| Butyl Methoxydibenzoylmethan | 0,80 |
| 4-Methylbenzylidene Kampfer | 1,60 |
| C₂₀₋₄₀-Alkylstearat | 9,50 |
| Glycerin | 10,00 |
| Farbabrieb | 11,60 |
| Parfüm | 0,30 |
| Wirkstoffe | 1,10 |
| Konservierung | 0,58 |
| Wasser | ad 100,00 |

**Beispiel 42**

| Lippenstift | |
|---|---|
| | Gew.-% |
| PEG-45/ Dodecyl Glycol Copolymer | 1,60 |
| Polyglyceryl-3-diisostearat | 2,40 |
| Simethicon | 0,50 |
| Polydecen | 4,80 |
| Ricinusoel | 5,00 |
| Dicaprylyl Carbonat | 4,80 |
| Buxus Chinensis | 1,00 |
| Squalan | 1,00 |
| Triisostearin | 3,50 |
| Butyl Methoxydibenzoylmethan | 0,80 |
| 4-Methylbenzylidene Kampfer | 1,60 |
| C₂₀₋₄₀-Alkylstearat | 9,50 |
| Glycerin | 10,00 |
| Farbabrieb | 11,60 |
| Parfüm | 0,30 |
| Wirkstoffe | 1,10 |
| Konservierung | 0,58 |
| Wasser | ad 100,00 |

**Beispiel 43**

| Deko-Lippenstift | |
|---|---|
| | Gew.-% |
| PEG-45/ Dodecyl Glycol Copolymer | 1,60 |
| Polyglyceryl-3-diisostearat | 2,40 |
| Simethicon | 0,50 |
| Polydecen | 4,80 |
| Octyldodecanol | 5,00 |
| Ricinusoel | 3,00 |
| Persea Gratissima | 1,50 |
| Macadamia Ternifolia | 1,50 |
| Buxus Chinensis | 1,00 |
| Squalan | 1,00 |
| Ethylhexyl Methoxycinnamat | 2,00 |
| Bis-Ethylhexyloxyphenol Methoxyphenyl Triazin | 0,25 |
| C₂₀₋₄₀-Alkylstearat | 8,00 |
| Cetyl Palmitat | 1,00 |
| PEG-8 Bienenwachs | 1,00 |
| Glycerin | 7,50 |
| Pearlpigment | 2,00 |
| Farbabrieb | 14,00 |
| Parfüm | 0,30 |
| Wirkstoffe | 1,10 |
| Konservierung | 0,58 |
| Wasser | ad 100,00 |

**Beispiel 44**

| Deko-Lippenstift | |
|---|---|
| | Gew.-% |
| PEG-45/ Dodecyl Glycol Copolymer | 1,60 |
| Polyglyceryl-3-diisostearat | 2,40 |
| Simethicon | 0,50 |
| Octyldodecanol | 7,00 |
| Polydecen | 4,80 |
| Ricinusoel | 3,00 |
| Buxus Chinensis | 1,00 |
| Squalan | 1,00 |
| Macadamiaoel | 1,50 |
| Persea Gratissima | 1,50 |
| Ethylhexyl Methoxycinnamat | 2,00 |
| Bis-Ethylhexyloxyphenol Methoxlphenyl Triazin | 0,25 |
| Cetyl Palmitat | 1,00 |
| C₂₀₋₄₀-Alkylstearat | 8,00 |
| Glycerin | 7,50 |
| Farbabrieb | 11,60 |
| Parfüm | 0,30 |
| Wirkstoffe | 1,10 |
| Konservierung | 0,58 |
| Wasser | ad 100,00 |

**Beispiel 45**

| Deko-Lippenstift | |
|---|---|
| | Gew.-% |
| PEG-45/ Dodecyl Glycol Copolymer | 1,60 |
| Sorbitanisostearat | 2,40 |
| Simethicone | 0,50 |
| Octyldodecanol | 7,00 |
| Polydecen | 4,80 |
| Ricinusoel | 3,00 |
| Buxus Chinensis | 1,00 |
| Squalan | 1,00 |
| Macadamiaoel | 1,50 |
| Persea Gratissima | 1,50 |
| Ethylhexyl Methoxycinnamat | 2,00 |
| Bis-Ethylhexyloxyphenol Methoxlphenyl Triazin | 0,25 |
| Cetyl Palmitat | 1,00 |
| C₂₀₋₄₀-Alkylstearat | 8,00 |
| Glycerin | 7,50 |
| Farbabrieb | 11,60 |
| Parfüm | 0,30 |
| Wirkstoffe | 1,10 |
| Konservierung | 0,58 |
| Wasser | ad 100,00 |

**Beispiel 46**

| Abdeckstift | |
|---|---|
| | Gew.-% |
| PEG-45/ Dodecyl Glycol Copolymer | 1,60 |
| PEG-7 hydrogeniertes Ricinusöl | 2,40 |
| Simethicon | 0,50 |
| Octyldodecanol | 7,00 |
| Polydecen | 4,80 |
| Ricinusoel | 3,00 |
| Buxus Chinensis | 1,00 |
| Squalan | |
| Macadamiaoel | 1,50 |
| Persea Gratissima | 1,50 |
| Ethylhexyl Methoxycinnamat | 2,00 |
| Bis-Ethylhexyloxyphenol Methoxlphenyl Triazin | 0,25 |
| Cetyl Palmitat | 1,00 |
| C₂₀₋₄₀-Alkylstearat | 8,00 |
| Glycerin | 7,50 |
| Farbabrieb | 11,60 |
| Parfüm | 0,30 |
| Wirkstoffe | 1,10 |
| Konservierung | 0,58 |
| Wasser | ad 100,00 |

**Beispiel 47**

| Abdeckstiftstift | |
|---|---|
| | Gew.-% |
| PEG-45/ Dodecyl Glycol Copolymer | 1,60 |
| Polyglyceryl-2 Dipolyhydroxystearat | 1,60 |
| Simethicone | 0,50 |
| Octyldodecanol | 7,00 |
| Polydecen | 7,00 |
| Ricinusoel | 7,00 |
| Butyl Methoxydibenzoylmethan | 0,80 |
| 4-Methylbenzylidene Kampfer | 1,60 |
| Candelilla Cera | 4,50 |
| C₂₀₋₄₀-Alkylstearat | 4,50 |
| Glycerin | 10,00 |
| Farbabrieb | 11,60 |
| Parfüm | 0,30 |
| Wirkstoffe | 1,10 |
| Konservierung | 0,58 |
| Wasser | ad 100,00 |

**Beispiel 49**

| Kajalstift | |
|---|---|
| | Gew.-% |
| PEG-45/ Dodecyl Glycol Copolymer | 1,60 |
| PEG-40 Sorbitanperisostearat | 1,60 |
| Simethicon | 0,50 |
| Octyldodecanol | 7,00 |
| Polydecen | 7,00 |
| Ricinusoel | 7,00 |
| Butyl Methoxydibenzoylmethan | 0,80 |
| 4-Methylbenzylidene Kampfer | 1,60 |
| Candelilla Cera | 4,50 |
| C₂₀₋₄₀-Alkylstearat | 4,50 |
| Glycerin | 10,00 |
| Farbabrieb | 11,60 |
| Parfüm | 0,30 |
| Wirkstoffe | 1,10 |
| Konservierung | 0,58 |
| Wasser | ad 100,00 |

**Beispiel 50**

| Kajalstift | |
|---|---|
| | Gew.-% |
| PEG-45/ Dodecyl Glycol Copolymer | 1,60 |
| Glycerinisostearat | 1,60 |
| Simethicon | 0,50 |
| Octyldodecanol | 7,00 |
| Polydecen | 7,00 |
| Ricinusoel | 7,00 |
| Butyl Methoxydibenzoylmethan | 0,80 |
| 4-Methylbenzylidene Kampfer | 1,60 |
| Candelilla Cera | 4,50 |
| C₂₀₋₄₀-Alkylstearat | 4,50 |
| Glycerin | 10,00 |
| Farbabrieb | 11,60 |
| Parfüm | 0,30 |
| Wirkstoffe | 1,10 |
| Konservierung | 0,58 |
| Wasser | ad 100,00 |

**Beispiel 51**

| Augenbrauenstift | |
|---|---|
| | Gew.-% |
| PEG-45/ Dodecyl Glycol Copolymer | 1,60 |
| Cetylalkohol | 1,60 |
| Simethicon | 0,50 |
| Octyldodecanol | 7,00 |
| Polydecen | 7,00 |
| Ricinusoel | 7,00 |
| Butyl Methoxydibenzoylmethan | 0,80 |
| 4-Methylbenzylidene Kampfer | 1,60 |
| Candelilla Cera | 4,50 |
| C₂₀₋₄₀-Alkylstearat | 4,50 |
| Glycerin | 10,00 |
| Farbabrieb | 11,60 |
| Parfüm | 0,30 |
| Wirkstoffe | 1,10 |
| Konservierung | 0,58 |
| Wasser | ad 100,00 |

**Beispiel 52**

| Lidschattenstift | |
|---|---|
| | Gew.-% |
| PEG-45/ Dodecyl Glycol Copolymer | 1,60 |
| Propylenglycolisostearat | 1,60 |
| Simethicon | 0,50 |
| Octyldodecanol | 7,00 |
| Polydecen | 7,00 |
| Ricinusoel | 7,00 |
| Butyl Methoxydibenzoylmethan | 0,80 |
| 4-Methylbenzylidene Kampfer | 1,60 |
| Candelilla Cera | 4,50 |
| C₂₀₋₄₀-Alkylstearat, Methylpalmitat (9:1) | 4,50 |
| Glycerin | 10,00 |
| Farbabrieb | 11,60 |
| Parfüm | 0,30 |
| Wirkstoffe | 1,10 |
| Konservierung | 0,58 |
| Wasser | ad 100,00 |

**Beispiel 53**

| Lidschattenstift | |
|---|---|
| | Gew.-% |
| PEG-45/ Dodecyl Glycol Copolymer | 1,60 |
| Isostearylglycerinether | 1,60 |
| Simethicon | 0,50 |
| Octyldodecanol | 7,00 |
| Polydecen | 7,00 |
| Ricinusoel | 7,00 |
| Butyl Methoxydibenzoylmethan | 0,80 |
| 4-Methylbenzylidene Kampfer | 1,60 |
| Candelilla Cera | 4,50 |
| C₂₀₋₄₀-Alkylstearat | 4,50 |
| Glycerin | 10,00 |
| Farbabrieb | 11,60 |
| Parfüm | 0,30 |
| Wirkstoffe | 1,10 |
| Konservierung | 0,58 |
| Wasser | ad 100,00 |

**Beispiel 54**

| Abdeckstift | |
|---|---|
| | Gew.-% |
| PEG-45/ Dodecyl Glycol Copolymer | 1,60 |
| Glycerin Sorbitan Isostearat | 1,60 |
| Simethicon | 0,50 |
| Octyldodecanol | 7,00 |
| Polydecen | 7,00 |
| Ricinusoel | 7,00 |
| Butyl Methoxydibenzoylmethan | 0,80 |
| 4-Methylbenzylidene Kampfer | 1,60 |
| Candelilla Cera | 4,50 |
| C₂₀₋₄₀-Alkylstearat | 4,50 |
| Glycerin | 10,00 |
| Farbabrieb | 11,60 |
| Parfüm | 0,30 |
| Liponsäure | 1,00 |
| Konservierung | 0,58 |
| Wasser | ad 100,00 |

**Beispiel 55**

| Abdeckstift | |
|---|---|
| | Gew.-% |
| PEG-45/ Dodecyl Glycol Copolymer | 1,60 |
| Steareth-2 | 1,60 |
| Simethicon | 0,50 |
| Octyldodecanol | 7,00 |
| Polydecen | 7,00 |
| Ricinusoel | 7,00 |
| Butyl Methoxydibenzoylmethan | 0,80 |
| 4-Methylbenzylidene Kampfer | 1,60 |
| Candelilla Cera | 4,50 |
| C₂₀₋₄₀-Alkylstearat | 4,50 |
| Glycerin, Chitosan | 10,00 |
| Farbabrieb | 11,60 |
| Parfüm | 0,30 |
| Kreatin | 1,10 |
| Konservierung | 0,58 |
| Wasser | ad 100,00 |

**Beispiel 56**

| Deko-Lippenstift | |
|---|---|
| | Gew.-% |
| PEG-45/ Dodecyl Glycol Copolymer | 1,60 |
| Propylenglycolisostearat | 1,60 |
| Simethicon | 0,50 |
| Octyldodecanol | 7,00 |
| Polydecen | 7,00 |
| Ricinusoel | 7,00 |
| Butyl Methoxydibenzoylmethan | 0,80 |
| 4-Methylbenzylidene Kampfer | 1,60 |
| Candelilla Cera | 4,50 |
| C₂₀₋₄₀-Alkylstearat | 4,50 |
| Glycerin, Fucogel (9:1) | 10,00 |
| Farbabrieb | 11,60 |
| Parfüm | 0,30 |
| Coenzym Q10 (verkapslet in Cycldextrin) | 1,10 |
| Konservierung | 0,58 |
| Wasser | ad 100,00 |

**Beispiel 57**

| Deko-Lippenstift | |
|---|---|
| | Gew.-% |
| PEG-45/ Dodecyl Glycol Copolymer | 1,60 |
| PEG-30 Dipolyhydroxystearat | 1,60 |
| Simethicon | 0,50 |
| Octyldodecanol | 7,00 |
| Polydecen | 7,00 |
| Ricinusoel | 7,00 |
| Butyl Methoxydibenzoylmethan | 0,80 |
| 4-Methylbenzylidene Kampfer | 1,60 |
| Candelilla Cera | 4,50 |
| C₂₀₋₄₀-Alkylstearat, Cetearylbehenat (9:1) | 4,50 |
| Glycerin, Milchsäure (9:1) | 10,00 |
| Farbabrieb | 11,60 |
| Parfüm | 0,30 |
| Biotin, Coenzym Q-10 (1:1) | 0.50 |
| Konservierung | 0,58 |
| Wasser | ad 100,00 |

**Beispiel 58**

| Lippenpflegestift | |
|---|---|
| | Gew.-% |
| PEG-45/ Dodecyl Glycol Copolymer | 1,60 |
| Polyglyceryl-3-diisostearat | 1,60 |
| Caprylic/Capric Triglycerid | 8,60 |
| Octyldodecanol | 8,60 |
| Dicaprylylether | 8,60 |
| Ricinusoel | 5,40 |
| C₁₈₋₃₆-Säuretriglycerid | 15,00 |
| Tocopherol, Carnithin (1:1) | 0.20 |
| Glycerin, Propylenglycol (9:1) | 10,00 |
| Konservierung | 0,58 |
| Wasser | ad 100,00 |

**Beispiel 59**

| Gesichtspflegestift mit hohem Wasser und Moisturizeranteil | |
|---|---|
| | Gew.-% |
| PEG-45/ Dodecyl Glycol Copolymer | 1,60 |
| Polyglyceryl-3-diisostearat | 2,40 |
| Octyldodecanol | 9,00 |
| Polydecen | 7,00 |
| Buxus Chinensis | 1,00 |
| Squalan | 1,00 |
| Ethylhexyl Methoxycinnamat | 5,00 |
| Bis-Ethylhexyloxyphenol Methoxyphenyl Triazin | 0,25 |
| C₂₀₋₄₀-Alkylstearat | 8,00 |
| Tocopherolacetat | 0,30 |
| PEG-8 Bienenwachs | 1,00 |
| Glycerin, Natriumpyrolidoncarbonsäuresalz (9:1) | 10,00 |
| Parfüm | 0,30 |
| Wirkstoffe | 1,00 |
| Konservierung | 0,58 |
| Wasser | ad 100,00 |

**Beispiel 60**

| Lippenpflegestift | |
|---|---|
| | Gew.-% |
| PEG-45/ Dodecyl Glycol Copolymer | 1,60 |
| Polyglyceryl-3-diisostearat | 2,40 |
| Octyldodecanol | 9,00 |
| Polydecen | 7,00 |
| Buxus Chinensis | 1,00 |
| Squalan | 1,00 |
| Ethylhexyl Methoxycinnamat | 5,00 |
| Bis-Ethylhexyloxyphenol Methoxyphenyl Triazin | 0,25 |
| C₂₀₋₄₀-Alkylstearat | 8,00 |
| PEG-8 Bienenwachs | 1,00 |
| Glycerin, Hyaluronsäure-Salz (9:1) | 10,00 |
| Parfüm | 0,30 |
| Dioic Acid | 0.50 |
| Konservierung | 0,58 |
| Wasser | ad 100,00 |

**Beispiel 61**

| Lippenpflegestift | |
|---|---|
| | Gew.-% |
| PEG-45/ Dodecyl Glycol Copolymer | 1,60 |
| Polyglyceryl-3-diisostearat | 1,60 |
| Octyldodecanol | 11,30 |
| Polydecen | 11,30 |
| Ricinusöl | 11,30 |
| Buxus Chinensis | 1,00 |
| Sheabutter | 1,00 |
| C₂₀₋₄₀-Alkylstearat | 9,00 |
| Glycerin, Polyethylenglycol (9:1) | 10,00 |
| Parfüm | 0,10 |
| Vitamin C, Tocpherol (1:1) | 1,10 |
| Konservierung | 0,58 |
| Wasser | ad 100,00 |

**Beispiel 62**

| Lippenpflegestift | |
|---|---|
| | Gew.-% |
| PEG-45/ Dodecyl Glycol Copolymer | 1,60 |
| Polyglyceryl-3-diisostearat | 1,60 |
| Octyldodecanol | 10,00 |
| Ricinusöl | 11,30 |
| Buxus Chinensis | 5,00 |
| Sheabutter | 1,00 |
| C₂₀₋₄₀-Alkylstearat | 8,00 |
| Glycerin, Harnstoff (9:1) | 10,00 |
| Parfüm | 0,10 |
| Wirkstoffe | 1,10 |
| Konservierung | 0,58 |
| Wasser | ad 100,00 |

**Beispiel 63**

| Lippenpflegestift | |
|---|---|
| | Gew.-% |
| PEG-45/ Dodecyl Glycol Copolymer | 1,60 |
| Polyglyceryl-3-diisostearat | 1,60 |
| Octyldodecanol | 5,00 |
| Polydecen | 5,00 |
| Ricinusoel | 5,00 |
| Buxus Chinensis | 1,00 |
| Butyrosperumum Parkii | 1,00 |
| Cera Microcristallina | 2,00 |
| C₂₀₋₄₀-Alkylstearat | 6,00 |
| Glycerin, Glycin (9:1) | 10,00 |
| Parfüm | 0,10 |
| Wirkstoffe | 1,10 |
| Konservierung | 0,58 |
| Wasser | ad 100,00 |

**Beispiel 64**

| Lippenpflegestift | |
|---|---|
| | Gew.-% |
| PEG-45/ Dodecyl Glycol Copolymer | 1,600 |
| Polyglyceryl-3-diisostearat | 1,60 |
| Octyldodecanol | 5,00 |
| Polydecen | 5,00 |
| Ricinusoel | 5,00 |
| Buxus Chinensis | 1,00 |
| Butyrosperumum Parkii | 1,00 |
| Hydrogenierte Coco-Glyceride | 2,00 |
| C₂₀₋₄₀-Alkylstearat | 6,00 |
| Glycerin, Harnstoff (9:1) | 10,00 |
| Parfüm | 0,10 |
| Wirkstoffe | 1,10 |
| Konservierung | 0,58 |
| Wasser | ad 100,00 |

**Beispiel 65**

| Lippenpflegestift | |
|---|---|
| | Gew.-% |
| PEG-45/ Dodecyl Glycol Copolymer | 1,60 |
| Polyglyceryl-3-diisostearat | 1,60 |
| Octyldodecanol | 5,00 |
| Polydecene | 5,00 |
| Ricinusoel | 5,00 |
| Buxus Chinensis | 1,00 |
| Butyrosperumum Parkii | 1,00 |
| Cetyl Palmitat | 2,00 |
| C₂₀₋₄₀-Alkylstearat | 6,00 |
| Glycerin, NaCl (7:3) | 10,00 |
| Parfüm | 0,10 |
| Wirkstoffe | 1,10 |
| Konservierung | 0,58 |
| Wasser | ad 100,00 |

**Beispiel 66**

| Lippenpflegestift | |
|---|---|
| | Gew.-% |
| PEG-45/ Dodecyl Glycol Copolymer | 1,60 |
| Polyglyceryl-3-diisostearat | 1,60 |
| Octyldodecanol | 5,00 |
| Polydecen | 5,00 |
| Ricinusoel | 5,00 |
| Buxus Chinensis | 1,00 |
| Butyrosperumum Parkii | 1,00 |
| Myristyl Myristat | 2,00 |
| C₂₀₋₄₀-Alkylstearat | 6,00 |
| Glycerin, Butylenglycol (9:1) | 10,00 |
| Parfüm | 0,10 |
| Wirkstoffe | 1,10 |
| Konservierung | 0,58 |
| Wasser | ad 100,00 |

**Beispiel 67**

| Lippenpflegestift | |
|---|---|
| | Gew.-% |
| PEG-45/ Dodecyl Glycol Copolymer | 1,60 |
| Polyglyceryl-3-diisostearat | 1,60 |
| Octyldodecanol | 11.45 |
| Hydrogeniertes Polydecen | 11.45 |
| Biosaccharid Gum | 5,00 |
| Buxus Chinensis | 1.00 |
| Persea Gratissima | 1,00 |
| Tocopherolacetat | 1.00 |
| Cetyl Palmitat | 0.55 |
| C₂₀₋₄₀-Alkylstearat | 6,55 |
| Hydrogenierte Cocoglyceride | 2.20 |
| PVP Hecadecen Copolymer | 2.00 |
| Squalan | 1.00 |
| Cera alba | 2,20 |
| Panthenol | 0.13 |
| Butyrospermum parkii | 1.00 |
| Octocrylen | 3.00 |
| Glycerin, Butylenglycol (9:1) | 10,00 |
| Parfüm | 0,10 |
| Konservierung | 0,58 |
| Wasser | ad 100,00 |

**Beispiel 68**

| Lippenpflegestift | |
|---|---|
| | Gew.-% |
| PEG-45/ Dodecyl Glycol Copolymer | 1,60 |
| Polyglyceryl-3-diisostearat | 1,60 |
| Octyldodecanol | 11.00 |
| Hydrogeniertes Polydecen | 11.00 |
| Biosaccharid Gum | 5,00 |
| Buxus Chinensis | 1.00 |
| Persea Gratissima | 1,00 |
| Macadamia Ternifolia | 1.00 |
| Tocopherolacetat | 1.00 |
| Cetyl Palmitat | 0.50 |
| C₂₀₋₂₀-Alkylstearat | 6,00 |
| Hydrogenierte Cocoglyceride | 2.00 |
| PVP Hecadecen Copolymer | 2.00 |
| Squalan | 1.00 |
| Cera alba | 2,00 |
| Panthenol | 0.13 |
| Butyrospermum parkii | 1.00 |
| Octocrylen | 3.00 |
| Lanolinöl, | 2.00 |
| Glycerin | 10,00 |
| Parfüm | 0,10 |
| Konservierung | 0,58 |
| Wasser | ad 100,00 |

**Beispiel 69**

| Sonnenschutzstift | |
|---|---|
| | Gew.-% |
| PEG-45/ Dodecyl Glycol Copolymer | 1,60 |
| Polyglyceryl-3-diisostearat | 1,60 |
| Capryl/Caprinsäuretriglycerid | 5,24 |
| Cetearyl Isononanoate | 5,24 |
| Butylenglycol Dicaprylat/dicaprat | 5,24 |
| Ethylhexyl Methoxycinnamat | 5,00 |
| Bis- Ethylhexyloxyphenol Methoxyphenyl Triazin | 3,00 |
| Ethylhexyltriazon | 3,00 |
| Titandioxid | 4,00 |
| PVP/Hexadecen Copolymer | 0,50 |
| C₂₀₋₄₀-Alkylstearat | 9,00 |
| Glycerin | 10,00 |
| Tocopherolacetat | 1,00 |
| Konservierung | 0,58 |
| Wasser | ad 100,00 |

**Beispiel 70**

| Sonnenschutzstift | |
|---|---|
| | Gew.-% |
| PEG-45/ Dodecyl Glycol Copolymer | 1,60 |
| Polyglyceryl-3-diisostearat | 1,60 |
| Capryl/Caprinsäuretriglycerid | 5,24 |
| Cetearyl Isononanoate | 5,24 |
| Butylenglycol Dicaprylat/dicaprat | 5,24 |
| Ethylhexyl Methoxycinnamate | 7,00 |
| Bis- Ethylhexyloxyphenol Methoxyphenyl Triazin | 2,50 |
| Ethylhexyltriazon | 3,00 |
| Titandioxid | 2,00 |
| PVP/Hexadecen Copolymer | 0,50 |
| C₂₀₋₄₀-Alkylstearat | 9,00 |
| Glycerin | 10,00 |
| Tocopherolacetat | 1,00 |
| Konservierung | 0,58 |
| Wasser | ad 100,00 |

**Beispiel 71**

| Sonnenschutzstift | |
|---|---|
| | Gew.-% |
| PEG-45/ Dodecyl Glycol Copolymer | 1,60 |
| Polyglyceryl-3-diisostearat | 1,60 |
| Capryl/Caprinsäuretriglycerid | 5,24 |
| Cetearyl Isononanoate | 5,24 |
| Butylenglycol Dicaprylat/dicaprat | 5,24 |
| Ethylhexyl Methoxycinnamat | 5,00 |
| Bis- Ethylhexyloxyphenol Methoxyphenyl Triazin | 2,50 |
| Ethylhexyltriazon | 3,00 |
| Titandioxid | 4,00 |
| Phenylbenzimidazol Sulfonsäure | 2,00 |
| Natronlauge | 0,55 |
| PVP/Hexadecen Copolymer | 0,50 |
| C₂₀₋₄₀-Alkylstearat | 9,00 |
| Glycerin | 10,00 |
| Tocopherolacetat | 1,00 |
| Konservierung | 0,58 |
| Wasser | ad 100,00 |

**Beispiel 72**

| Sonnenschutzstift | Gew.-% |
|---|---|
| PEG-45/ Dodecyl Glycol Copolymer | 1,60 |
| Polyglyceryl-3-diisostearat | 1,60 |
| Capryl/Caprinsäuretriglycerid | 5,24 |
| Cetearyl Isononanoat | 4,10 |
| Butylenglycol Dicaprylat/dicaprat | 4,07 |
| Ethylhexyl Methoxycinnamat | 5,00 |
| Bis- Ethylhexyloxyphenol Methoxyphenyl Triazin | 2,00 |
| Octocrylen | 5,00 |
| Diethylhexyl Butamido Triazon | 2,00 |
| Titandioxid | 4,00 |
| PVP/Hexadecen Copolymer | 0,50 |
| C₂₀₋₄₀-Alkylstearat | 9,00 |
| Glycerin | 10,00 |
| Tocopherolacetat | 1,00 |
| Konservierung | 0,58 |
| Wasser | ad 100,00 |

**Beispiel 73**

| Sonnenschutzstift | Gew.-% |
|---|---|
| PEG-45/ Dodecyl Glycol Copolymer | 1,60 |
| Polyglyceryl-3-diisostearat | 1,60 |
| Cetearyl Isononanoate | 15,00 |
| Butylene Glycol Dicaprylate/Dicaprat | 5,00 |
| Ethylhexyl Methoxycinnamat | 3,60 |
| Butyl Methoxycinnamat | 1,00 |
| Methylbenzylidencampher | 3,60 |
| Titandioxid | 3,00 |
| C₂₀₋₄₀-Alkylstearat | 8,50 |
| Glycerin | 10,00 |
| Konservierung | 0,58 |
| Wasser | ad 100,00 |

**Beispiel 74**

| Sonnenschutzstift | |
|---|---|
| | Gew.-% |
| PEG-45/ Dodecyl Glycol Copolymer | 1,60 |
| Polyglyceryl-3-diisostearat | 1,60 |
| Cetearyl Isononanoat | 10,00 |
| Butylene Glycol Dicaprylate/Dicaprat | 5,00 |
| Ethylhexyl Methoxycinnamat | 5,00 |
| Ethylhexyl Triazon | 3,00 |
| Bis-Ethylhexyloxyphenol Methoxyphenyl Triazin | 2,50 |
| Titandioxid | 2,00 |
| C₂₀₋₄₀-Alkylstearat | 8,50 |
| Glycerin | 10,00 |
| Konservierung | 0,58 |
| Wasser | ad 100,00 |

**Beispiel 75**

| Sonnenschutzstift | |
|---|---|
| | Gew.-% |
| PEG-45/ Dodecyl Glycol Copolymer | 1,60 |
| Polyglyceryl-3-diisostearat | 1,60 |
| Cetearyl Isononanoate | 10,00 |
| Butylene Glycol Dicaprylate/Dicaprat | 5,00 |
| Ethylhexyl Methoxycinnamat | 5,00 |
| Ethylhexyl Triazon | 3,00 |
| Bis-Ethylhexyloxyphenol Methoxyphenyl Triazin | 2,50 |
| Disodium Phenyl Dibenzimidazol Tetrasulfonat | 0,50 |
| Natronlauge | 0,50 |
| Titandioxid | 2,00 |
| C₂₀₋₄₀-Alkylstearat | 8,50 |
| Glycerin | 10,00 |
| Konservierung | 0,50 |
| Wasser | ad 100,00 |

**Beispiel 75a**

| Sonnenschutzstift | Gew.-% |
|---|---|
| PEG-45/ Dodecyl Glycol Copolymer | 1,60 |
| Polyglyceryl-3-diisostearat | 1,60 |
| Cetearyl Isononanoat | 5,00 |
| Caprylic/Capric Triglycerid | 5,00 |
| Butylene Glycol Dicaprylate/Dicaprat | 5,00 |
| Ethylhexyl Methoxycinnamat | 5,00 |
| Ethylhexyl Triazon | 3,00 |
| Bis-Ethylhexyloxyphenol Methoxyphenyl Triazin | 2,50 |
| Titandioxid | 2,00 |
| C₂₀₋₄₀-Alkylstearat | 8,50 |
| Glycerin, Natriumpyroldioncarbonsäuresalz | 10,00 |
| (9:1) | |
| Konservierung | 0,58 |
| Wasser | ad 100,00 |

**Beispiel 76**

| Sonnenschutzstift | Gew.-% |
|---|---|
| PEG-45/ Dodecyl Glycol Copolymer | 1,60 |
| Polyglyceryl-3-diisostearat | 1,60 |
| Cetearyl Isononanoat | 10,00 |
| Butylene Glycol Dicaprylate/Dicaprat | 5,00 |
| Ethylhexyl Methoxycinnamat | 5,00 |
| Ethylhexyl Triazon | 3,00 |
| Bis-Ethylhexyloxyphenol Methoxyphenyl Triazin | 2,50 |
| Titandioxid | 2,00 |
| C₂₀₋₄₀-Alkylstearat | 6,00 |
| C₁₈₋₃₆-Acid Triglycerid | 4,00 |
| Glycerin, Hyaluronsäuresalz (9:1) | 10,00 |
| Wirkstoffe (Glyosylrutin) | 0.50 |
| Konservierung | 0,58 |
| Wasser | ad 100,00 |

**Beispiel 77**

| Sonnenschutzstift | |
|---|---|
| | Gew.-% |
| PEG-45/ Dodecyl Glycol Copolymer | 1,60 |
| Polyglyceryl-3 Diisostearat | 1,60 |
| Caprylic/ Capric Triglycerid | 5,24 |
| Simethicon | 0,50 |
| Glycerin, Glycin (9:1) | 10,00 |
| Cetearyl Isononanoat | 5,24 |
| PVP/ Hexadecene Copolymer | 0,50 |
| PEG-8 Bienenwachs | 0.50 |
| C20-40 Alkyl Stearat | 8,50 |
| Butylene Glycol Dicaprylate/ Dicaprat | 5,24 |
| Wirkstoff (Q-10) | 0,50 |
| Konservierung | 0,58 |
| Ethylhexyl Methoxycinnamate | 5,00 |
| Ethylhexyl Triazon | 3,00 |
| Titanium Dioxide + Trimethoxycaprylylsilian | 4,00 |
| Bis-Ethylhexylphenol Methoxyphenyl Triazin | 2,50 |
| Wasser | ad 100,00 |

**Beispiel 78**

| Sonnenschutzstift | |
|---|---|
| | Gew.-% |
| PEG-45/ Dodecyl Glycol Copolymer | 1,60 |
| Polyglyceryl-3 Diisostearat | 1,60 |
| Caprylic/ Capric Triglycerid | 5,24 |
| Simethicon | 0,50 |
| Glycerin, Harnstoff (9:1) | 10,00 |
| Cetearyl Isononanoate | 5,24 |
| PVP/ Hexadecene Copolymer | 0,50 |
| C20-40 Alkyl Stearat | 8,50 |
| Carnaubawachs | 0.50 |
| Butylene Glycol Dicaprylate/ Dicaprat | 5,24 |
| Wirkstoff (Liponsäure, Carnithin 1:1) | 1,00 |
| Konservierung | 0,58 |
| Ethylhexyl Methoxycinnamat | 7,00 |
| Ethylhexyl Triazon | 3,00 |
| Titanium Dioxid | 2,00 |
| Bis-Ethylhexylphenol Methoxyphenyl Triazin | 2,50 |
| Wasser | ad 100,00 |

**Beispiel 79**

| Sonnenschutzstift | |
|---|---|
| | Gew.-% |
| PEG-45/ Dodecyl Glycol Copolymer | 1,60 |
| Polyglyceryl-3 Diisostearat | 1,60 |
| Caprylic/ Capric Triglycerid | 5,24 |
| Simethicon | 0,50 |
| Glycerin, Polyethylenglycol (9:1) | 10,00 |
| Cetearyl Isononanoat | 5,24 |
| PVP/ Hexadecene Copolymer | 0,50 |
| C20-40 Alkyl Stearat | 9,00 |
| Methylpalmitat | 1,00 |
| Butylene Glycol Dicaprylate/ Dicaprat | 5,24 |
| Wirkstoff (Creatin) | 1,00 |
| Konservierung | 0,58 |
| Ethylhexyl Methoxycinnamat | 5,00 |
| Phenylbenzimidazole Sulfonsäure | 2,00 |
| Natronlauge (105) | 0,55 |
| Ethylhexyl Triazon | 3,00 |
| Titanium Dioxide | 2,00 |
| Bis-Ethylhexylphenol Methoxyphenyl Triazin | 2,50 |
| Wasser | ad 100,00 |

**Beispiel 80**

| Sonnenschutzstift | |
|---|---|
| | Gew.-% |
| PEG-45/ Dodecyl Glycol Copolymer | 1,60 |
| Polyglyceryl-3 Diisostearat | 1,60 |
| Caprylic/ Capric Triglycerid | 4,41 |
| Simethicon | 0,50 |
| Glycerin, Fucogel | 10,00 |
| Cetearyl Isononanoat | 4,41 |
| PVP/ Hexadecene Copolymer | 0,50 |
| C20-40 Alkyl Stearat | 9,00 |
| Butylene Glycol Dicaprylate/ Dicaprat | 4,41 |
| Wirkstoff (Adenosin) | 1,00 |
| Konservierung | 0,58 |
| Ethylhexyl Methoxycinnamate | 5,00 |
| Titanium Dioxid | 2,00 |
| Octocrylen | 5,00 |
| Bis-Ethylhexylphenol Methoxyphenyl Triazin | 2,00 |
| Diethylhexyl Butamido Triazon | 3,00 |
| Wasser | ad 100,00 |

**Beispiel 81**

| Sonnenschutzstift bzw Aftersun-Stift | |
|---|---|
| | Gew.-% |
| PEG-45/ Dodecyl Glycol Copolymer | 1,60 |
| Polyglyceryl-3 Diisostearat | 1,60 |
| Caprylic/ Capric Triglycerid | 4,07 |
| Simethicon | 0,50 |
| Glycerin, Natriumchlorid (5:5) | 10,00 |
| Cetearyl Isononanoat | 4,07 |
| PVP/ Hexadecene Copolymer | 0,50 |
| C20-40 Alkyl Stearat | 9,00 |
| Butylene Glycol Dicaprylate/ Dicaprat | 4,07 |
| Wirkstoff (Tocopherol, Vitamin C 1:3)) | 4,00 |
| Konservierung | 0,58 |
| Ethylhexyl Methoxycinnamat | 5,00 |
| Octocrylen | 5,00 |
| Titanium Dioxide | 4,00 |
| Bis-Ethylhexylphenol Methoxyphenyl Triazin | 2,00 |
| Diethylhexyl Butamido Triazon | 2,00 |
| Wasser | ad 100,00 |

**Beispiel 82**

| Sonnenschutzstift bzw. Aftersun-Stift | |
|---|---|
| | Gew.-% |
| PEG-45/ Dodecyl Glycol Copolymer | 1,60 |
| Sorbitanisostearat | 1,60 |
| Caprylic/ Capric Triglycerid | 4,40 |
| Simethicon | 0,50 |
| Glycerin | 10,00 |
| Cetearyl Isononanoat | 4,40 |
| PVP/ Hexadecene Copolymer | 0,50 |
| C20-40 Alkyl Stearat | 9,00 |
| Butylene Glycol Dicaprylate/ Dicaprat | 4,40 |
| Wirkstoff (Biotin, Tocopherol, 1:1) | 1,00 |
| Konservierung | 0,58 |
| Ethylhexyl Methoxycinnamat | 7,00 |
| Ethylhexyl Triazon | 3,00 |
| Titanium Dioxide | 4,00 |
| Bis-Ethylhexylphenol Methoxyphenyl Triazin | 2,50 |
| Parfüm | 0,50 |
| Wasser | ad 100,00 |

**Beispiel 83**

| Sonnenschutzstift bzw. Aftersun-Stift | |
|---|---|
| | Gew.-% |
| PEG-45/ Dodecyl Glycol Copolymer | 1,60 |
| PEG-30 Dipolyhydroxystearat | 1,60 |
| Caprylic/ Capric Triglycerid | 4,57 |
| Simethicon | 0,50 |
| Glycerin | 10,00 |
| Dicaprylylcarbonat | 4,57 |
| PVP/ Hexadecene Copolymer | 0,50 |
| C20-40 Alkyl Stearat | 9,00 |
| Butylene Glycol Dicaprylate/ Dicaprat | 4,57 |
| Wirkstoff (Panthenol, Tocopherol 1:1) | 1,00 |
| Konservierung | 0,58 |
| Ethylhexyl Methoxycinnamate + BHT | 5,00 |
| Phenylbenzimidazole Sulfonsäure | 2,00 |
| Natronlauge (10%) | 0,55 |
| Ethylhexyl Triazon | 3,00 |
| Bis-Ethylhexylphenol Methoxyphenyl Triazin | 2,00 |
| Titanium Dioxid + Alumina + Simethicon + Aqua | 4,00 |
| Parfüm | 0,50 |
| Wasser | ad 100,00 |

**Beispiel 84**

| Sonnenschutzstift | |
|---|---|
| | Gew.-% |
| PEG-45/ Dodecyl Glycol Copolymer | 1,60 |
| Polyglyceryl-3 Diisostearat | 1,60 |
| Caprylic/ Capric Triglycerid | 4,40 |
| Simethicon | 0,50 |
| Glycerin | 10,00 |
| Cetearyl Isononanoat, C12-15 Alkylbenzoat (1.1) | 4,40 |
| PVP/ Hexadecene Copolymer | 0,50 |
| C20-40 Alkyl Stearat | 9,00 |
| Butylene Glycol Dicaprylate/ Dicaprat | 4,40 |
| Wirkstoff (Zinksalz, Bisabolol 1:1) | 1,00 |
| Konservierung | 0,58 |
| Ethylhexyl Methoxycinnamat | 7,00 |
| Ethylhexyl Triazon | 3,000 |
| Bis-Ethylhexylphenol Methoxyphenyl Triazin | 2,50 |
| Titanium Dioxid + Alumina + Simethicon + Aqua | 4,00 |
| Parfüm | 0,50 |
| Wasser | ad 100,00 |

**Beispiel 85**

| Sonnenschutzstift | |
|---|---|
| | Gew.-% |
| PEG-45/ Dodecyl Glycol Copolymer | 1,60 |
| Polyglyceryl-3 methylglucose distearat | 1,60 |
| Caprylic/ Capric Triglycerid | 5,07 |
| Simethicon | 0,50 |
| Glycerin | 10,00 |
| Cetearyl Isononanoat, Lanolinöl | 5,07 |
| PVP/ Hexadecene Copolymer | 0,50 |
| C20-40 Alkyl Stearat | 9,00 |
| Butylene Glycol Dicaprylate/ Dicaprat | 5,07 |
| Wirkstoff | 1,00 |
| Konservierung | 0,58 |
| Ethylhexyl Methoxycinnamat | 5,00 |
| Ethylhexyl Triazone | 3,00 |
| Bis-Ethylhexylphenol Methoxyphenyl Triazin | 2,50 |
| Titanium Dioxid + Alumina + Simehticon + Aqua | 4,00 |
| Parfüm | 0,50 |
| Wasser | ad 100,00 |

**Beispiel 86**

| Sonnenschutzstift | |
|---|---|
| | Gew.-% |
| PEG-45/ Dodecyl Glycol Copolymer | 1,60 |
| Polyglyceryl-3 Diisostearat | 1,60 |
| Caprylic/ Capric Triglycerid, Cocoglycerid | 4,74 |
| Simethicon | 0,50 |
| Glycerin | 10,00 |
| Cetearyl Isononanoat | 4,74 |
| PVP/ Hexadecene Copolymer | 0,50 |
| C20-40 Alkyl Stearat | 9,00 |
| -Butylene Glycol Dicaprylate/ Dicaprat | 4,74 |
| Wirkstoff (Tocopherolacetat) | 1,00 |
| Konservierung | 0,58 |
| Ethylhexyl Methoxycinnamat | 5,00 |
| Ethylhexyl Triazon | 3,00 |
| Bis-Ethylhexylphenol Methoxyphenyl Triazin | 2,50 |
| Silica Dimethyl Silyat | 1,00 |
| Titanium Dioxide + Alumina + Simehticon+ Aqua | 4,00 |
| Parfüm | 0,50 |
| Wasser | ad 100,00 |

**Beispiel 87**

| Sonnenschutzstift | |
|---|---|
| | Gew.-% |
| PEG-45/ Dodecyl Glycol Copolymer | 1,60 |
| Polyglyceryl-3-diisostearat | 1,60 |
| Capryl/Caprinsäuretriglycerid | 5,24 |
| Cetearyl Isononanoate | 5,24 |
| Butylenglycol Dicaprylat/dicaprat | 5,24 |
| Ethylhexyl Methoxycinnamat | 5,00 |
| Bis- Ethylhexyloxyphenol Methoxyphenyl Triazin | 3,00 |
| Ethylhexyltriazon | 3,00 |
| Titandioxid* und Proylenglycol | 2.00 |
| PVP/Hexadecen Copolymer | 0,50 |
| C₂₀₋₄₀-Alkylstearat | 9,00 |
| Glycerin | 10,00 |
| Tocopherolacetat | 1,00 |
| Konservierung | 0,58 |
| Wasser | ad 100,00 |

Wasserdispergierbares Titandioxid: Tioveil AQ+10%Propylenglycol von Tioxid Specalities

**Beispiel 88**

| Hautaufhellender Stift | |
|---|---|
| | Gew.-% |
| Caprylic/Capric Triglycerid | 14,6666 |
| Aqua | 61,5534 |
| Glycerin | 10 |
| Phenoxyethanol | 0,5 |
| PEG-45/Dodecyl Glycol Copolymer | 1,6 |
| Polyglyceryl-3 Diisostearat | 1,6 |
| C20-40 Alkyl Stearat | 9 |
| Hexamidin Diisethionat | 0,08 |
| 8-Hexadecen-1,16-Dicarbonsäure | 1 |

**Beispiele 89-91: Seidenschimmerstifte; Beispiel 92: Plegestift**

| | 89 | 90 | 91 | 92 |
|---|---|---|---|---|
| | Gew.-% | Gew.-% | Gew.-% | Gew.-% |
| Butylen Glycol Dicaprylat/Dicaprat | 8,1120 | 4,7735 | 4,7735 | 4,7735 |
| C18-36 Fettsäure Triglyceride | 0,6000 | 1,1000 | 1,1000 | 1,1000 |
| C20-40 Alkyl Stearat | 11,4000 | 20,9000 | | |
| Caprylic/Capric Triglyceride | 8,3110 | 4,7730 | 4,7730 | 4,7730 |
| Cetearyl Isononanoat | 8,1120 | 4,7735 | 4,7735 | 4,7735 |
| Parfum | 0,4000 | 0,4000 | 0,4000 | 0,4000 |
| Glycerin | 10,0000 | 10,0000 | 10,0000 | 10,0000 |
| Hexamidin Diisethionat | 0,0800 | 0,0800 | 0,0800 | 0,0800 |
| PEG-45/Dodecyl Glycol Copolymer | 1,6000 | 1,6000 | 1,6000 | 1,6000 |
| Phenoxyethanol | 0,5000 | 0,5000 | 0,5000 | 0,5000 |
| Polyethylene (Performalene 400) | | | 20,9000 | 20,9000 |
| Polyglyceryl-3 Diisostearat | 1,6000 | 1,6000 | 1,6000 | 1,6000 |
| Simethicon | 0,5000 | 0,5000 | 0,5000 | 0,5000 |
| Titandioxid + Eisenoxide + Mica | 0,5000 | 0,5000 | 0,5000 | |
| Titandioxid + Mica + Zinn Oxide | 3,0000 | 3,0000 | 3 | |
| Wasser | Ad 100 | Ad 100 | Ad 100 | Ad 100 |

**Beispiele 93-97**

| | 93 | 94 | 95 | 96 | 97 |
|---|---|---|---|---|---|
| | Pflegestift | Abdeckstift | Foundation Stift | Sonnenschutzstift | Blushstift |
| Caprylic/Capric Triglyceride | 8 | 5 | 5 | 8 | 3 |
| Octyldodecanol | 7 | 5 | 5 | 8 | 6 |
| Carpylyl Carbonate | | | 3 | | |
| Dicaprylylether | | | 2 | | |
| Paraffinöl | 2 | | | | 1 |
| Pentaerythrityl Tetraisostearate | 2 | 4 | | 8 | |
| C12-15 Alkyl Benzoate | 2 | | | | |
| Isopopyl Palmitate | | | | | 2 |
| Jojobaöl | 2 | | | 1 | 1 |
| Lanolinöl | | | | | 1 |
| Simethicone | | 0,5 | 0,5 | | 0,5 |
| PEG-45/ Dodecyl Glycol Copolymer | 3 | 3,5 | 2 | | 2 |
| Polyglyceryl-3 Diisostearate | 2,5 | | 1,5 | 2 | 2,4 |
| PEG-30 Di-Polyhydroxystearate | | | | 2,5 | |
| Sucrose Distearate | 0,5 | | | | |
| Bis-Diglyceryl Polyacyladipate-2 | 9 | 2 | | | |
| Cetyl Palmitate | 2,5 | | | | 1 |
| C16-36 Alkyl Stearate | 14 | 1 | 2 | 1 | |
| C20-40 Alkyl Stearate | | 8 | 8 | 9 | 8 |
| Carnaubawachs | 1,5 | 1,5 | | | 2 |
| Bienenwachs | 0,5 | | | | |
| Candelillawachs | | | | | 1 |
| PVP / Eicosene Copolymer | | 1 | | 1 | 0,2 |
| Butyl Methoxydibenzoylmethane | | | | 1 | |
| mikronisiertes Titan Dioxid | | 2 | | 4 | |
| 4-Methylbenzylidene Camphor | | | | 3,6 | 5 |
| Octyl Methoxycinnamate | | 2 | | 3,6 | 2,5 |
| Nylon-12 | | 3 | | | |
| Bismuthoxichlorid (BiOCl) | 2 | | 3 | 3 2 | 2 |
| Bornitrid | | | | 3 | 1 |
| Lauroyl Lysine | | 0,5 | | | |
| Polymethylsilsesquioxane (Tospearl) | | | 0,5 | 1 | |
| Silica LDP | | | | 1 | |
| PTFE | 2,5 | | | | |
| PMMA | | 6 | 3 | | |
| Titandioxid Al₂O₃ gecoated | | 7 | 6 | | 2 |
| Eisenoxide | | 4 | 4 | | 6 |
| Ultramarin | | 0,5 | 0,6 | | |
| Perlglanzpigmente | 3 | | | | 2 |
| Rokonsal S1 | 0,4 | | | | |
| Germall II | | 0,25 | | | 0,25 |
| Glydant Plus | | | | 0,3 | |
| JM Acti Care | | | 0,05 | | |
| Glycerin | 5 | 2 | 10 | 5 | 10 |
| Parfum, BHT, Neutralisationsmittel, Sequestriemittel | q.s | q.s | q.s | q.s | q.s |
| Wasser | ad 100 | ad 100 | ad 100 | ad 100 | ad 100 |

## Patentansprüche

1. Bei Raumtemperatur feste W/O-Emulsionen, enthaltend bezogen auf die Gesamtzubereitung
(a) eine Fettphase, welche
(a1) mindestens eine Ölkomponente, wobei die Ölkomponenten in einem Gehalt von 1 bis 20 Gew.% vorliegen,
(a2) mindestens eine Wachskomponente umfasst, wobei die Wachskomponenten in einem Gehalt von 5 bis 20 Gew.% vorliegen und das Verhältnis von Öl- zu Wachskomponenten zwischen 3:1 und 1:3 liegt,
(b) eine Wasserphase, welche
(b1) 35 bis 65 Gew.% Wasser sowie
(b2) 4 bis 40 Gew.% eines Hautbefeuchtungsmittels gewählt aus der Gruppe Glycerin, Chitosan, Fucogel, Propylenglycol, Polyethylenglycol, Dipropylenglycol, Butylenglycol, Mannitol, Milchsäure, Polyethylenglycol, Glycin, Natriumpyrolidoncarbonsäure, Hyaluronsäure, Salze der angegebenen Säuren sowie, Harnstoff und Salze von Metallen der ersten und zweiten Hauptgruppe umfasst,
(c)
(c) einen W/O-Emulgator oder ein Gemisch aus mehreren W/O-Emulgatoren, gewählt aus der Gruppe der grenzflächenaktiven Substanzen der allgemeinen Struktur A-B-A', wobei A und A' gleiche oder verschiedene hydrophobe organische Reste darstellen und B eine hydrophile Gruppe bedeutet, wobei der oder die Emulgator(en) in Konzentrationen bis 5 Gew.% vorliegen und
(d)
(d) Stabilisatoren gewählt aus der Gruppe der Substanzen der allgemeinen Formel 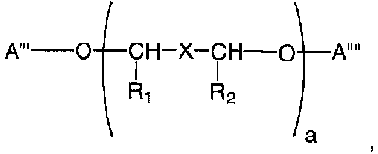 wobei
- A'" und A'''' gleiche oder verschiedene hydrophobe organische Reste darstellen,
- a eine Zahl von 1 bis 100 darstellt,
- X eine Einfachbindung oder die Gruppe 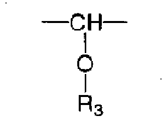
- darstellt,
- R₁ und R₂ unabhängig voneinander aus der Gruppe H, Methyl gewählt werden, dass aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1 - 20 Kohlenstoffatomen,
- wobei die Reste A''' und A'''' können gleich oder verschieden sein und gewählt werden aus der Gruppe 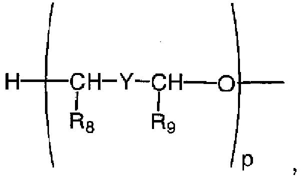
- wobei R₈ und R₉ gleich oder verschieden sein können und gewählt werden aus der Gruppe der gesättigten und ungesättigten Alkyl- und Acylreste mit 1 - 30 Kohlenstoffatomen, p eine Zahl von 1 - 20 darstellt und Y eine Einfachbindung oder die Gruppe 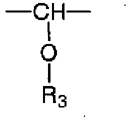 darstellt,
- wobei R₃ gewählt wird aus der Gruppe H, sowie der verzweigten und unverzweigten, gesättigten und ungesättigten Alkyl- und Acylreste mit 1 - 30 Kohlenstoffatomen, ferner können die Gruppe A''' und A"" unabhängig voneinander auch Alkylreste oder Acylreste darstellen,
in Konzentrationen von 0,01 bis 5 Gew.%.

2. W/O-Emulsionen nach Anspruch 1, **dadurch gekennzeichnet, dass** der W/O-Emulgator oder die W/O-Emulgatoren gewählt werden aus der Gruppe der Substanzen der allgemeinen Formel 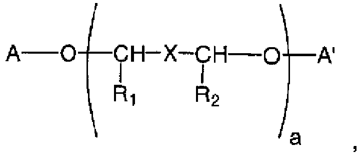 wobei
- A und A' gleiche oder verschiedene hydrophobe organische Reste darstellen,
- a eine Zahl von 1 bis 100 darstellt,
- X eine Einfachbindung oder die Gruppe 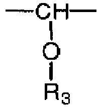
- darstellt,
- R₁ und R₂ unabhängig voneinander so gewählt werden H, Methyl, dass aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1 - 20 Kohlenstoffatomen,oder dass der oder die W/O-Emulgatoren gewählt werden aus der Gruppe der Fettalkohole mit 8 - 30 Kohlenstoffatomen, Monoglycerinester gesättigter und/oder ungesättigter, verzweigter und/oder unverzweigter Alkancarbonsäuren oder Hydroxyalkansäuren einer Kettenlänge von 8 - 24 C-Atomen, Diglycerinester gesättigter und/oder ungesättigter, verzweigter und/oder unverzweigter Alkancarbonsäuren oder Hydroxyalkansäuren einer Kettenlänge von 8 - 24 C-Atomen, Triglycerinester gesättigter und/oder ungesättigter, verzweigter und/oder unverzweigter Alkancarbonsäuren oder Hydroxyalkansäuren einer Kettenlänge von 8-24 C-Atomen, Polyglycerinester gesättigter und/oder ungesättigter, verzweigter und/oder unverzweigter Alkancarbonsäuren oder Hydroxyalkansäuren einer Kettenlänge von 8 - 24 C-Atomen mit bis zu 10 Glycerineinheiten, Monoglycerinether gesättigter und/oder ungesättigter, verzweigter und/oder unverzweigter Alkohole einer Kettenlänge von 8 - 24 C-Atomen, Diglycerinether gesättigter und/oder ungesättigter, verzweigter und/oder unverzweigter Alkohole einer Kettenlänge von 8 - 24 C-Atomen, Triglycerinether gesättigter und/oder ungesättigter, verzweigter und/oder unverzweigter Alkohole einer Kettenlänge von 8 - 24 C-Atomen, Polyglycerinether gesättigter und/oder ungesättigter, verzweigter und/oder unverzweigter Alkohole einer Kettenlänge von 8 - 24, C-Atomen mit bis zu 10 Glycerineinheiten, Propylenglycolester gesättigter und/oder ungesättigter, verzweigter und/oder unverzweigter Alkancarbonsäuren oder Hydroxyalkansäuren einer Kettenlänge von 8 - 24 C-Atomen, Sorbitanester gesättigter und/oder ungesättigter, verzweigter und/oder unverzweigter Alkancarbonsäuren oder Hydroxyalkansäuren einer Kettenlänge von 8-24 C-Atomen, Sorbitanester von Polyolen, insbesondere des Glycerins, Pentaerythritylester gesättigter und/oder ungesättigter, verzweigter und/oder unverzweigter Alkancarbonsäuren oder Hydroxyalkansäuren einer Kettenlänge von 8 - 24 C-Atomen, Methylglucose Ester gesättigter und/oder ungesättigter, verzweigter und/oder unverzweigter Alkancarbonsäuren oder Hydroxyalkansäuren einer Kettenlänge von 8 - 24 C-Atomen, Polyglycerin Methylglucose Ester gesättigter und/oder ungesättigter, verzweigter und/oder unverzweigter Alkancarbonsäuren oder Hydroxyalkansäuren einer Kettenlänge von 8 - 24 C-Atomen, der Glyceryfettsäure Citrate, Cetyl Dimethicon Copolyole, der Alkyl Methicon Copolyole, der Alkyl Dimthicon Ethoxy Glucoside, oder dass die vorstehend genannten Typen von W/O-Emulgatoren zusätzlich in der Weise polyethoxyliert und/oder polypropoxyliert sind, daß sie ethoxylierte und/oder propopoxylierte W/O-Emulgatoren darstellen.

3. W/O-Emulsionen nach mindestens einem der vorangehenden Ansprüche, **dadurch gekennzeichnet, dass** der W/O-Emulgator oder die W/O-Emulgatoren so gewählt werden, dass die Reste A und A' werden vorteilhaft gewählt aus der Gruppe der verzweigten und unverzweigten, gesättigten und ungesättigten Alkyl- und Acylreste und Hydroxyacylreste mit 10-30 Kohlenstoffatomen sowie ferner aus der Gruppe der über Esterfunktionen miteinander verbundenen Hydroxyacylgruppen, nach dem Schema. 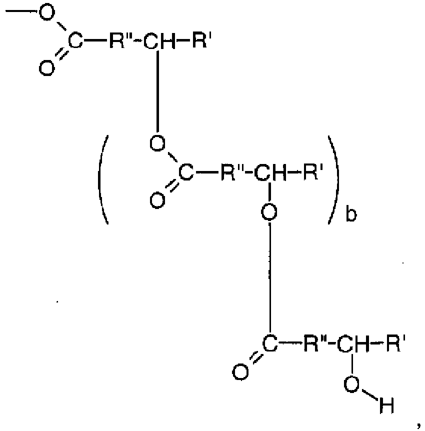 wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann.

4. W/O-Emulsionen nach mindestens einem der vorangehenden Ansprüche, **dadurch gekennzeichnet, dass** der oder die W/O-Emulgatoren gewählt werden aus der Gruppe PEG-30 Dipolyhydroxystearat, Decaglycerylheptaoleat, Polyglyceryl-3-Diisostearat, PEG-8 Distearat, Diglycerin Dipolyhydroxystearat, Glycerinisostearat, Sorbitanisostearat, Polyglyceryl-3 methylglucose distearat, Steareth-2.

5. W/O-Emulsionen nach mindestens einem der vorangehenden Ansprüche, **dadurch gekennzeichnet, dass** als Stabilisator das PEG-45 /Dodecylglycolcopolymer und/oder das PEG-22 / Dodecylglycolcopolymer und/oder das Methoxy PEG-22/Dodecyl Glycol Copolymer verwendet werden.

6. Kosmetischer und/oder dermatologischer Stift, enthaltend Emulsionen nach mindestens einem der vorangehenden Ansprüche, **dadurch gekennzeichnet, dass** die Stifthülse beidseitig von oben und unten befüllbar ist.

7. Emulsion oder Stift nach mindestens einem der vorangehenden Ansprüche, **dadurch gekennzeichnet, dass** zusätzlich mindestens ein Pigment und/oder mindestens ein Farbstoff und/oder mindestens ein Puderstoff enthalten sind.

8. Emulsion oder Stift nach mindestens einem der Ansprüche 1 bis 7, **dadurch gekennzeichnet, dass** sie mindestens einen gegen Falten wirksamen Stoff enthält.

9. Emulsion oder Stift nach mindestens einem der Ansprüche 1 bis 7, **dadurch gekennzeichnet, dass** sie mindestens eine UVA-Filtersubstanz und/oder mindestens eine UVB-Filtersubstanz und/oder mindestens ein anorganisches Pigment enthält.

10. Emulsion oder Stift nach mindestens einem der Ansprüche 1 bis 7, **dadurch gekennzeichnet, dass** sie mindestens einen gegen Akne wirksamen Stoff enthalten.

## Claims

1. W/O emulsions, solid at room temperature, comprising, based on the total preparation.
(a) a fatty phase which includes
(a1) at least one oil component, wherein the oil components are present in a content of from 1 to 20% by weight,
(a2) at least one wax component, wherein the wax components are present in a content of from 5 to 20% by weight and the ratio of oil components to wax components is between 3:1 and 1:3,
(b) a water phase which includes
(b1) 35 to 65% by weight of water and
(b2) 4 to 40% by weight of a skin-moisturizing agent chosen from the group consisting of glycerol, chitosan, Fucogel, propylene glycol, polyethylene glycol, dipropylene glycol, butylene glycol, mannitol, lactic acid, polyethylene glycol, glycine, sodium pyrrolidonecarboxylic acid, hyaluronic acid, salts of the given acids, and urea and salts of metals of the first and second main group,
(c)
(c) a W/O emulsifier or a mixture of two or more W/O emulsifiers chosen from the group of interface-active substances of the general structure A-B-A', where A and A' are identical or different hydrophobic organic radicals, and B is a hydrophilic group, wherein the emulsifier(s) are present in concentrations up to 5% by weight and
d) stabilizers chosen from the group of substances of the general formula 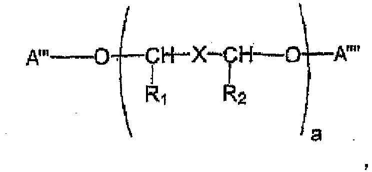 where
- A'" and A"" are identical or different hydrophobic organic radicals,
- a is a number from 1 to 100,
- X is a single bond or the group 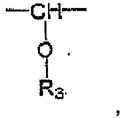
- R₁ and R₂, independently of one another, are chosen from the group consisting of H, methyl, but such that both radicals are not methyl at the same time,
- R₃ is chosen from the group consisting of H, and branched and unbranched, saturated and unsaturated alkyl and acyl radicals having 1-20 carbon atoms,
- where the radicals A''' and A'''' may be identical or different and are chosen from the group 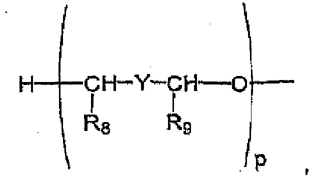
- where R₈ and R₉ may be identical or different and are chosen from the group of saturated and unsaturated alkyl and acyl radicals having 1-30 carbon atoms, p is a number from 1-20, and Y is a single bond or the group 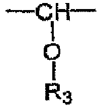
- where R₃ is chosen from the group consisting of H, and branched and unbranched, saturated and unsaturated alkyl and acyl radicals having 1-30 carbon atoms, in addition the group A''' and A'''' may, independently of one another, also be alkyl radicals or acyl radicals,
in concentrations of from 0.01 to 5% by weight.

2. W/O emulsions according to Claim 1, **characterized in that** the W/O emulsifier or the W/O emulsifiers are chosen from the group of substances of the general formula 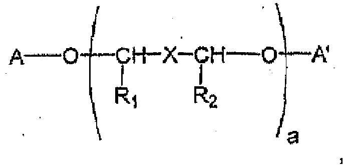 where
- A and A' are identical or different hydrophobic organic radicals,
- a is a number from 1 to 100,
- X is a single bond or the group 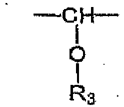
- R₁ and R₂, independently of one another, are H, methyl, but chosen such that both radicals are not methyl at the same time,
- R₃ is chosen from the group consisting of H, and branched and unbranched, saturated and unsaturated alkyl and acyl radicals having 1-20 carbon atoms, or that the W/O emulsifier or emulsifiers are chosen from the group of fatty alcohols having 8-30 carbon atoms, monoglycerol esters of saturated and/or unsaturated, branched and/or unbranched alkanecarboxylic acids or hydroxyalkanoic acids with a chain length of 8-24 carbon atoms, diglycerol esters of saturated and/or unsaturated, branched and/or unbranched alkanecarboxylic acids or hydroxyalkanoic acids with a chain length of 8-24 carbon atoms, triglycerol esters of saturated and/or unsaturated, branched and/or unbranched alkanecarboxylic acids or hydroxyalkanoic acids with a chain length of 8-24 carbon atoms, polyglycerol esters of saturated and/or unsaturated, branched and/or unbranched alkanecarboxylic acids or hydroxyalkanoic acids with a chain length of 8-24 carbon atoms with up to 10 glycerol units, monoglycerol ethers of saturated and/or unsaturated, branched and/or unbranched alcohols with a chain length of 8-24 carbon atoms, diglycerol ethers of saturated and/or unsaturated, branched and/or unbranched alcohols with a chain length of 8-24 carbon atoms, triglycerol ethers of saturated and/or unsaturated, branched and/or unbranched alcohols with a chain length of 8-24 carbon atoms, polyglycerol ethers of saturated and/or unsaturated, branched and/or unbranched alcohols with a chain length of 8-24 carbon atoms, with up to 10 glycerol units, propylene glycol esters of saturated and/or unsaturated, branched and/or unbranched alkanecarboxylic acids or hydroxyalkanoic acids with a chain length of 8-24 carbon atoms, sorbitan esters of saturated and/or unsaturated, branched and/or unbranched alkanecarboxylic acids or hydroxyalkanoic acids with a chain length of 8-24 carbon atoms, sorbitan esters of polyols, in particular of glycerol, pentaerythrityl esters of saturated and/or unsaturated, branched and/or unbranched alkanecarboxylic acids or hydroxyalkanoic acids with a chain length of 8-24 carbon atoms, methylglucose esters of saturated and/or unsaturated, branched and/or unbranched alkanecarboxylic acids or hydroxyalkanoic acids with a chain length of 8-24 carbon atoms, polyglycerol methylglucose esters of saturated and/or unsaturated, branched and/or unbranched alkanecarboxylic acids or hydroxyalkanoic acids with a chain length of 8-24 carbon atoms, of glyceryl fatty acid citrates, cetyl dimethicone copolyols, of alkyl methicone copolyols, of alkyl dimethicone ethoxyglucasides, or that the abovementioned types of W/O emulsifiers have been additionally polyethoxylated and/or polypropoxylated in such a way that they represent ethoxylated and/or propoxylated W/O emulsifiers.

3. W/O emulsions according to at least one of the preceding claims, **characterized in that** the W/O emulsifier or the W/O emulsifiers are chosen such that the radicals A and A' are advantageously chosen from the group of branched and unbranched, saturated and unsaturated alkyl and acyl radicals and hydroxyacyl radicals having 10-30 carbon atoms, and also from the group of hydroxyacyl groups joined together via ester functions, according to the scheme 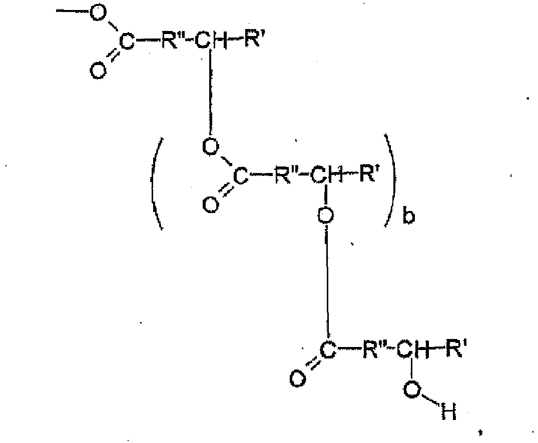 where R' is chosen from the group of branched and unbranched alkyl groups having 1 to 20 carbon atoms and R" is chosen from the group of branched and unbranched alkylene groups having 1 to 20 carbon atoms, and b can assume numbers from 0 to 200.

4. W/O emulsions according to at least one of the preceding claims, **characterized in that** the W/O emulsifier or emulsifiers are chosen from the group consisting of PEG-30 dipolyhydroxystearate, decaglyceryl heptaoleate, polyglyceryl-3 diisostearate, PEG-8 distearate, diglycerol dipolyhydroxystearate, glycerol isostearate, sorbitan isostearate, polyglyceryl-3 methylglucose distearate, steareth-2.

5. W/O emulsions according to at least one of the preceding claims, **characterized in that** the stabilizer used is the PEG-45/dodecyl glycol copolymer and/or the PEG-22/dodecyl glycol copolymer and/or the methoxy PEG-22/dodecyl glycol copolymer.

6. Cosmetic and/or dermatological stick comprising emulsions according to at least one of the preceding claims, **characterized in that** the stick sleeve can be filled on both sides from top and bottom.

7. Emulsion or stick according to at least one of the preceding claims, **characterized in that** at least one pigment and/or at least one dye and/or at least one powder substance are additionally present.

8. Emulsion or stick according to at least one of Claims 1 to 7, **characterized in that** it comprises at least one antiwrinkle substance.

9. Emulsion or stick according to at least one of Claims 1 to 7, **characterized in that** it comprises at least one UVA filter substance and/or at least one UVB filter substance and/or at least one inorganic pigment.

10. Emulsion or stick according to at least one of claims 1 to 7, **characterized in that** it comprises at least one antiacne substance.

## Revendications

1. Émulsions E/H solides à température ambiante, contenant par rapport à la préparation totale
(a) une phase grasse, qui comprend
(a1) au moins un composant huileux, les composants huileux étant présents en une teneur de 1 à 20 % en poids,
(a2) au moins un composant cireux, les composants cireux étant présents en une teneur de 5 à 20 % en poids et le rapport entre les composants huileux et cireux étant compris entre 3:1 et 1:3 ;
(b) une phase aqueuse, qui comprend
(b1) 35 à 65 % en poids d'eau et
(b2) 4 à 40 % en poids d'un agent d'hydratation de la peau, choisi dans le groupe constitué par la glycérine, le chitosan, le fucogel, le propylène glycol, le polyéthylène glycol, le dipropylène glycol, le butylène glycol, le mannitol, l'acide lactique, le polyéthylène glycol, la glycine, l'acide pyrrolidone-carboxylique sodique, l'acide hyaluronique, les sels des acides indiqués, ainsi que l'urée et les sels de métaux du premier et deuxième groupe principal,
(c) un émulsifiant E/H ou un mélange de plusieurs émulsifiants E/H, choisis dans le groupe des substances tensioactives de structure générale A-B-A', A et A' représentant des radicaux organiques hydrophobes identiques ou différents, et B signifiant un groupe hydrophile, le ou les émulsifiants étant présents en concentrations de jusqu'à 5 % en poids, et
(d) des stabilisateurs choisis dans le groupe des substances de formule générale 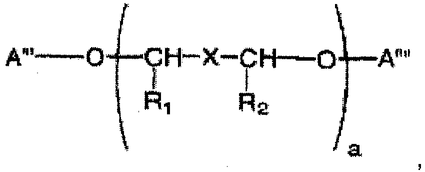 dans laquelle
- A''' et A"" représentent des radicaux organiques hydrophobes identiques ou différents,
- a représente un nombre de 1 à 100,
- X représente une liaison simple ou le groupe 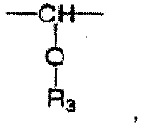
- R₁ et R₂ sont choisis indépendamment l'un de l'autre dans le groupe constitué par H, méthyle, les deux radicaux ne représentant toutefois pas simultanément méthyle,
- R₃ est choisi dans le groupe constitué par H, ainsi que les radicaux alkyle et acyle ramifiés et non ramifiés, saturés et insaturés, de 1 à 20 atomes de carbone,
- les radicaux A'" et A"" pouvant être identiques ou différents, et étant choisis dans le groupe constitué par 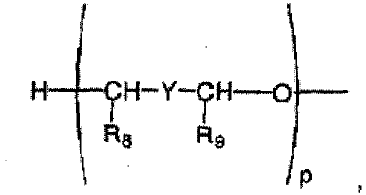
- R₈ et R₉ pouvant être identiques ou différents, et étant choisis dans le groupe des radicaux alkyle et acyle saturés et insaturés de 1 à 30 atomes de carbone, p représentant un nombre de 1 à 20 et Y représentant une liaison simple ou le groupe 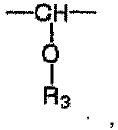
- R₃ étant choisi dans le groupe constitué par H, ainsi que les radicaux alkyle et acyle ramifiés et non ramifiés, saturés et insaturés, de 1 à 30 atomes de carbone, les groupes A''' et A"" pouvant par ailleurs également représenter indépendamment l'un de l'autre des radicaux alkyle ou des radicaux acyle,
en concentrations de 0,01 à 5 % en poids.

2. Émulsions E/H selon la revendication 1, **caractérisées en ce que** l'émulsifiant E/H ou les émulsifiants E/H sont choisis dans le groupe des substances de formule générale 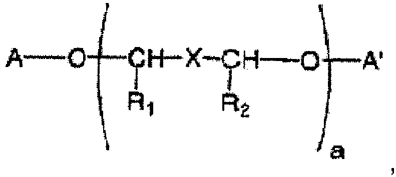 dans laquelle A et A' représentent des radicaux organiques hydrophobes identiques ou différents, a représente un nombre de 1 à 100,
- X représente une liaison simple ou le groupe 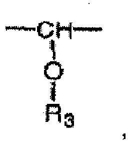
- R₁ et R₂ sont choisis indépendamment l'un de l'autre parmi H, méthyle, les deux radicaux ne représentant toutefois pas simultanément méthyle,
- R₃ est choisi dans le groupe constitué par H, ainsi que les radicaux alkyle et acyle ramifiés et non ramifiés, saturés et insaturés, de 1 à 20 atomes de carbone,
ou **en ce que** le ou les émulsifiants E/H soient choisis dans le groupe constitué par les alcools gras de 8 à 30 atomes de carbone, les esters de monoglycérine d'acides alcanecarboxyliques ou d'acides hydroxyalcanoïques saturés et/ou insaturés, ramifiés et/ou non ramifiés, d'une longueur de chaîne de 8 à 24 atomes C, les esters de diglycérine d'acides alcanecarboxyliques ou d'acides hydroxyalcanoïques saturés et/ou insaturés, ramifiés et/ou non ramifiés, d'une longueur de chaîne de 8 à 24 atomes C, les esters de triglycérine d'acides alcanecarboxyliques ou d'acides hydroxyalcanoïques saturés et/ou insaturés, ramifiés et/ou non ramifiés, d'une longueur de chaîne de 8 à 24 atomes C, les esters de polyglycérine d'acides alcanecarboxyliques ou d'acides hydroxyalcanoïques saturés et/ou insaturés, ramifiés et/ou non ramifiés, d'une longueur de chaîne de 8 à 24 atomes C, contenant jusqu'à 10 unités glycérine, les éthers de monoglycérine d'alcools saturés et/ou insaturés, ramifiés et/ou non ramifiés, d'une longueur de chaîne de 8 à 24 atomes C, les éthers de diglycérine d'alcools saturés et/ou insaturés, ramifiés et/ou non ramifiés, d'une longueur de chaîne de 8 à 24 atomes C, les éthers de triglycérine d'alcools saturés et/ou insaturés, ramifiés et/ou non ramifiés, d'une longueur de chaîne de 8 à 24 atomes C, les éthers de polyglycérine d'alcools saturés et/ou insaturés, ramifiés et/ou non ramifiés, d'une longueur de chaîne de 8 à 24 atomes C, contenant jusqu'à 10 unités glycérine, les esters de propylène glycol d'acides alcanecarboxyliques ou d'acides hydroxyalcanoïques saturés et/ou insaturés, ramifiés et/ou non ramifiés, d'une longueur de chaîne de 8 à 24 atomes C, les esters de sorbitane d'acides alcanecarboxyliques ou d'acides hydroxyalcanoïques saturés et/ou insaturés, ramifiés et/ou non ramifiés, d'une longueur de chaîne de 8 à 24 atomes C, les esters de sorbitane de polyols, notamment de glycérine, les esters de pentaérythrityle d'acides alcanecarboxyliques ou d'acides hydroxyalcanoïques saturés et/ou insaturés, ramifiés et/ou non ramifiés, d'une longueur de chaîne de 8 à 24 atomes C, les esters de méthylglucose d'acides alcanecarboxyliques ou d'acides hydroxyalcanoïques saturés et/ou insaturés, ramifiés et/ou non ramifiés, d'une longueur de chaîne de 8 à 24 atomes C, les esters de polyglycérine méthylglucose d'acides alcanecarboxyliques ou d'acides hydroxyalcanoïques saturés et/ou insaturés, ramifiés et/ou non ramifiés, d'une longueur de chaîne de 8 à 24 atomes C, les citrates d'acides gras de glycéryle, les cétyldiméthicone-copolyols, les alkylméthicone-copolyols, les alkyldiméthicone-éthoxyglucosides, ou **en ce que** les types mentionnés précédemment d'émulsifiants E/H soient en outre polyéthoxylés et/ou polypropoxylés de sorte qu'ils consistent en des émulsifiants E/H éthoxylés et/ou propoxylés.

3. Émulsions E/H selon au moins l'une quelconque des revendications précédentes, **caractérisées en ce que** l'émulsifiant E/H ou les émulsifiants E/H sont choisis de sorte que les radicaux A et A' soient de préférence choisis dans le groupe constitué par les radicaux alkyle et acyle et les radicaux hydroxyacyle ramifiés et non ramifiés, saturés et insaturés, de 10 à 30 atomes de carbone, ainsi qu'également dans le groupe constitué par les groupes hydroxyacyle reliés les uns avec les autres par des fonctions ester, selon le schéma 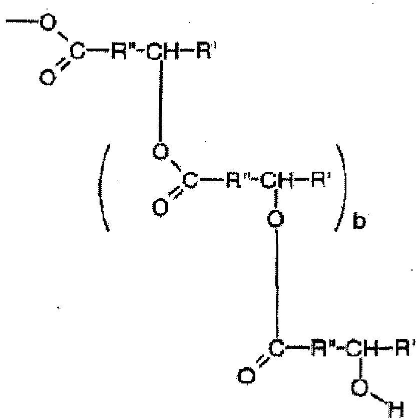 R' étant choisi dans le groupe constitué par les groupes alkyle ramifiés et non ramifiés de 1 à 20 atomes de carbone et R" étant choisi dans le groupe constitué par les groupes alkylène ramifiés et non ramifiés de 1 à 20 atomes de carbone, et b pouvant représenter des nombres de 0 à 200.

4. Émulsions E/H selon au moins l'une quelconque des revendications précédentes, **caractérisées en ce que** le ou les émulsifiants E/H sont choisis dans le groupe constitué par le dipolyhydroxystéarale de PEG-30, l'heptaoléate de décaglycéryle, le 3-diisostéarate de polyglycéryle, le distéarate de PEG-8, le dipolyhydroxystéarate de diglycérine, l'isostéarate de glycérine, l'isostéarate de sorbitane, le distéarate de polyglycéryl-3-méthyglucose, le stéareth-2.

5. Émulsions E/H selon au moins l'une quelconque des revendications précédentes, **caractérisées en ce que** le copolymère PEG-45/dodécylglycol et/ou le copolymère PEG-22/dodécylglycol et/ou le copolymère méthoxy-PEG-22/dodécylglycol sont utilisés en tant que stabilisateur.

6. Bâton cosmétique et/ou dermatologique, contenant des émulsions selon au moins l'une quelconque des revendications précédentes, **caractérisé en ce que** l'enveloppe du bâton peut être remplie des deux côtés par le haut et par le bas.

7. Émulsion ou bâton selon au moins l'une quelconque des revendications précédentes, caractérisé(e) en ce qu'au moins un pigment et/ou au moins un colorant et/ou au moins une poudre sont en outre contenus.

8. Émulsion ou bâton selon au moins l'une quelconque des revendications 1 à 7, caractérisé(e) en ce qu'il ou elle contient au moins une substance antirides.

9. Émulsion ou bâton selon au moins l'une quelconque des revendications 1 à 7, caractérisé(e) en ce qu'il ou elle contient au moins une substance de filtration des UVA et/ou au moins une substance de filtration des UVB et/ou au moins un pigment inorganique.

10. Émulsion ou bâton selon au moins l'une quelconque des revendications 1 à 7, caractérisé(e) en ce qu'il ou elle contient au moins au moins une substance active contre l'acné.
